# FASCICULE DE BREVET EUROPEEN

(11) **EP 2 078 009 B1**
(45) Date de publication et mention de la délivrance du brevet: **18.03.2015**
(21) Numéro de dépôt: 07858463.8
(22) Date de dépôt: 17.10.2007
(51) Int. Cl.: C07D 401/12, C07D 403/12, C07D 471/04, C07D 487/04, C07D 519/00, A61K 31/437, A61K 31/506, A61P 25/16, A61P 25/28, A61P 35/00

(54) **NOUVEAUX DERIVES DU FLUORENE, COMPOSITIONS LES CONTENANT ET UTILISATION COMME INHIBITEURS DE LA PROTEINE CHPAERONE HSP90**
NEUE FLUORENDERIVATE, ZUSAMMENSETZUNGEN DAMIT UND IHRE VERWENDUNG ALS CHAPERON HSP 90-HEMMER
NEW FLUORENE DERIVATIVES, COMPOSITIONS CONTAINING THE SAME AND USE THEREOF AS INHIBITORS OF THE PROTEIN CHAPERONE HSP 90

(30) Priorité: 24.10.2006 FR 0609331
(43) Date de publication de la demande: 15.07.2009
(73) Titulaire: SANOFI, 75008 Paris (FR)
(72) Inventeur: THOMPSON, Fabienne, 75013 Paris (FR); MAILLIET, Patrick, 75013 Paris (FR); RUXER, Jean-Marie, 75013 Paris (FR); GOULAOUIC, Hélène, 75013 Paris (FR); VALLEE, François, 75013 Paris (FR); MINOUX, Hervé, 75013 Paris (FR); PILORGE, Fabienne, 75013 Paris (FR); BERTIN, Luc, 75013 Paris (FR); HOURCADE, Stéphane, 75013 Paris (FR); MENDEZ-PEREZ, Maria, 65926 Frankfurt (DE); HAMLEY, Peter, 65926 Frankfurt (DE)
(74) Mandataire: Le Coupanec, Pascale A.M.P.
(86) Numéro de dépôt international: PCT/FR2007/001703
(87) Numéro de publication internationale: WO 2008/049994

(56) Documents cités:
- WO-A-2005/028434
- WO-A-2006/123061
- JANIN Y L: "Heat Shock Protein 90 Inhibitors. A Text Book Example of Medicinal Chemistry?" JOURNAL OF MEDICINAL CHEMISTRY, AMERICAN CHEMICAL SOCIETY. WASHINGTON, US, vol. 48, no. 24, 8 novembre 2005 (2005-11-08), pages 7503-7512, XP002373723 ISSN: 0022-2623

## Description

La présente invention concerne de nouveaux composés chimiques, dérivés tricycliques et plus particulièrement de nouveaux dérivés hétérocycliques de carbazole, d'azacarbazole, de phénanthridine, de phénothazine, de phénoxazine et de dibenzazépine, les compositions qui les contiennent, et leur utilisation comme médicaments.

Plus particulièrement, l'invention concerne, selon un premier aspect, de nouveaux dérivés hétérocycliques de carbazole, de phénanthridine, de phénothazine de phénoxazine et de dibenzazépine présentant une activité anticancéreuse, et en particulier une activité inhibitrice de la protéine chaperone Hsp90, et plus particulièrement via l'inhibition de l'activité catalytique de type ATPasique de la protéine chaperone Hsp90.

### Protéines Chaperones :

Les chaperones moléculaires de la famille « Heat Shock Proteins » (HSPs), classées en fonction de leur masse moléculaire (Hsp27, Hsp70, Hsp90...), sont des éléments clés de l'équilibre entre la synthèse et la dégradation des protéines cellulaires, responsables du repliement correct des protéines. Elles jouent un rôle vital en réponse au stress cellulaire. Les HSPs, et en particulier Hsp90, sont également impliquées dans la régulation de diverses fonctions majeures de la cellule, via leur association avec diverses protéines clients impliquées dans la prolifération cellulaire ou l'apoptose (Jolly C. et Morimoto R.I., J. N. Cancer Inst..(2000), 92, 1564-72; Smith D.F. et al., Pharmacological Rev. (1998), 50, 493-513; Smith D.F. , Molecular Chaperones in the Cell, 165-178, Oxford University Press 2001).

### Chaperone Hsp90 et inhibiteurs d'Hsp90 dans le traitement des cancers :

La chaperone Hsp90, qui représente 1 à 2% du contenu protéique de la cellule a été récemment mise en évidence comme une cible particulièrement prometteuse en thérapie anticancéreuse (cf pour revue : Moloney A. et Workman P., Expert Opin. Biol. Ther. (2002), 2(1), 3-24 ; Chiosis et al, Drug Discovery Today (2004), 9, 881-888). Cet intérêt tient en particulier aux interactions cytoplasmiques d'Hsp90 avec les principales protéines clients d'Hsp90, protéines qui sont impliquées dans les six mécanismes de progression des tumeurs, tels que définis par Hanahan D. et Weinberg R.A. (Cell (2002), 100, 57-70), à savoir :
- une capacité à proliférer en l'absence de facteurs de croissance : EGFR-R/HER2, Src, Akt, Raf, MEK, Bcr-Abl, Flt-3 ...
- une capacité à échapper à l'apoptose : forme mutée de p53, Akt, survivine ...
- une insensibilité aux signaux d'arrêt de prolifération : Cdk4, Plk , Wee1 ...
- une capacité à activer l'angiogénèse : VEGF-R, FAK, HIF-1, Akt, ...
- une capacité à proliférer sans limite réplicative : hTert ...
- une capacité à envahir de nouveaux tissus et à métastaser : c-Met

Parmi les autres protéines clients de Hsp90, des récepteurs aux hormones stéroïdiennes, tels que le récepteur à l'estrogène ou le récepteur à l'androgène, présentent également un intérêt important dans le cadre des thérapies anticancéreuses.

Il a été montré récemment que la forme alpha d'Hsp90 avait également un rôle extracellulaire via son interaction avec la métalloprotéase MMP-2, elle-même impliquée dans l'invasion tumorale (Eustace B.K. et al, Nature Cell Biology (2004), 6, 507-514).

Hsp90 est constituée de deux domaines N- et C-terminaux séparés par une région fortement chargée. L'interaction dynamique entre ces deux domaines, coordonnée par la fixation de nucléotides et de co-chaperones, détermine la conformation de la chaperone et son état d'activation. L'association des protéines clients dépend principalement de la nature des co-chaperones Hsp70/Hsp40, Hop60 etc ... et de la nature du nucléotide ADP ou ATP liée au domaine N-terminal de Hsp90. Ainsi l'hydrolyse de l'ATP en ADP et le facteur d'échange ADP/ATP contrôlent l'ensemble de la « machinerie » chaperone, et il a été montré qu'il suffit de prévenir l'hydrolyse de l'ATP en ADP - activité ATPasique d'Hsp90 - pour libérer dans le cytoplasme des protéines-clients qui seront alors dégradées au protéasome (Neckers L et Neckers K, Expert Opin. Emerging Drugs (2002), 7, 277-288 ; Neckers L, Current Médicinal Chemistry, (2003), 10, 733-739 ; Piper P.W., Current Opin. Invest. New Drugs (2001), 2, 1606-1610).

### Rôle d'Hsp90 et de ses inhibiteurs dans des pathologies autres que le cancer:

Diverses pathologies humaines sont la conséquence d'un repliement incorrect de protéines clés, conduisant notamment à des maladies neurodégénératives suite à l'agrégation de certaines protéines comme dans les maladies d'Alzheimer et de Huntington ou les maladies liées aux prions (Tytell M. et Hooper P.L., Emerging Ther. Targets (2001), 5, 3788-3796). Dans ces pathologies, des approches visant à inhiber Hsp90 dans le but d'activer les voies de stress (Hsp70 par exemple) pourraient être bénéfiques (Nature Reviews Neuroscience 6 : 11, 2005). Quelques exemples sont cités ci-dessous :
i) La maladie de Huntington : Cette maladie neurodégénérative est due à une extension de triplets CAG dans l'exon 1 du gène codant pour la protéine huntingtine. Il a été montré que la geldanamycine inhibe l'agrégation de cette protéine du fait de la surexpression des chaperones Hsp70 et Hsp40 (Human Molecular Genetics 10: 1307, 2001).
ii) La maladie de Parkinson: Cette maladie est due à la perte progressive des neurones dopaminergiques et caractérisée par l'aggrégation de la protéine alpha-synuclein. Il a été montré que la geldanamycine est capable de protéger la drosophile contre la toxicité de l'alpha-synuclein sur les neurones dopaminergiques.
iii) L'ischémie cérébrale focale : Il a été montré dans un modèle animal de rat que la geldanamycine protège le cerveau contre l'ischémie cérébrale, et ce du fait de l'effet de stimulation de la transcription des gènes codant pour les « heat-shock proteins » par un inhibiteur d'Hsp90.
iv) Les maladies d'Alzheimer et la sclérose en plaques : Ces maladies sont dues en partie à l'expression de cytokines pro-inflammatoires et de la forme inductible de la NOS (Nitric oxide synthase) dans le cerveau, et cette expression délétaire est supprimée par la réponse au stress. En particulier, les inhibiteurs d'Hsp90 sont capables d'engranger cette réponse au stress, et il a été montré in vitro que la geldanamycine et le 17-AAG présentent une activité anti-inflammatoire dans des cellules gliales de cerveau (J. Neuroscience Res. 6 : 461, 2002).
v) La sclérose latérale amyotrophique : Cette maladie neurodégénérative est due à la perte progressive des neurones moteurs. Il a été montré que l'arimoclomol, un inducteur tu heat-shock proteins », retarde l'évolution de la maladie dans un modèle animal (Nature Medicine 10: 402, 2004). Etant donné qu'un inhibiteur d'Hsp90 est également un inducteur des protéines heat-shock (Mol. Cell Biol. 19: 8033, 1999 ; Mol. Cell Biol. 18: 4949, 1998), il est probable qu'un effet bénéfique pourrait être obtenu également dans cette pathologie pour ce type d'inhibiteurs.

D'autre part, un inhibiteur de la protéine Hsp90 pourrait être potentiellement utile dans diverses maladies, autres que le cancer cité précedemment, telles que des infections parasitaires, virales, fongiques, ou des maladies neurodégénératives et ce par une action directe sur Hsp90 et des protéines clients particulières. Quelques exemples sont présentés ci-dessous :
vi) la malaria : la protéine Hsp90 de Plasmodium falciparum présente 59% d'identité et 69% de similarité avec la protéine Hsp90 humaine, et il a été montré que la geldanamycine inhibe la croissance du parasite in vitro (Malaria Journal 2 : 30, 2003 ; J. Biol. Chem. 278 : 18336, 2003 ; J. Biol. Chem. 279: 46692, 2004).
vii) Les filarioses de Brugia et de Bancroft : ces parasites filaires lymphatiques possèdent une protéine Hsp90 pouvant potentiellement être inhibée par des inhibiteurs de la protéine humaine. En effet, il a été montré pour un autre parasite proche, le Brugia pahangi, que ce dernier est sensible à l'inhibition par la geldanamycine. Les séquences de B. pahangi et humaines sont identiques à 80% et similaires à 87%. (Int. J. for Parasitology 35: 627,2005)
viii) La toxoplasmose : Toxoplasma gondii, le parasite responsable de la toxoplasmose, possède une protéine chaperone Hsp90, pour laquelle il a été montré l'induction au cours de la conversion tachyzoite-bradyzoite, correspondant au passage de l'infection chronique vers la toxoplasmose active. De plus, la geldanamycine bloque in vitro cette conversion tachyzoite-bradyzoite (J. Mol. Biol. 350 : 723, 2005)
ix) Les mycoses résistantes aux traitements : Il est possible que la protéine Hsp90 potentialise l'évolution de la résistance aux drogues, en permettant à de nouvelles mutations de se développer. Par conséquent, un inhibiteur de Hsp90, seul ou en combinaison avec un autre traitement antifongique, pourrait se révéler utile dans le traitement de certaines souches résistantes (Science 309 : 2185, 2005). De plus, l'anticorps anti-Hsp90 développé par Neu Tec Pharma démontre une activité contre C. albicans, sensible et résistant au fluconazole, C. krusei, C. tropicalis, C. glabrata, C. lusitaniae et C. parapsilosis in vivo (Current Molecular Medicine 5 : 403, 2005).
x) L'hépatite B : Hsp90 est l'une des protéines de l'hôte qui interagit avec la transcriptase inverse du virus de l'hépatite B au cours du cycle de réplication du virus. Il a été montré que la geldanamycine inhibe la réplication de l'ADN viral et l'encapsulation de l'ARN viral (Proc. Natl. Acad. Sci. USA 93: 1060, 1996)
xi) L'hépatite C : La protéine Hsp90 humaine participe à l'étape de clivage entre les protéines NS2 et NS3 par la protéase virale. La geldanamycine et le radicicol sont capables d'inhiber ce clivage NS2/3 in vitro (Proc. Natl. Acad. Sci. USA 98 : 13931, 2001)
xii) Le virus de l'Herpes: La geldanamycine a démontré des activités d'inhibition de la réplication du virus HSV-1 in vitro, et ce avec un bon index thérapeutique (Antimicrobial Agents and Chemotherapy 48 : 867, 2004). Les auteurs ont également trouvé une activité de la geldanamycine sur les autres virus HSV-2, VSV, Cox B3, HIV-1 et le coronavirus SARS (données non montrées).
xiii) La dengue (ou grippe tropicale) : Il a été montré que la protéine humaine Hsp90 participe à l'étape d'entrée du virus, en formant un complexe contenant également Hsp70 qui sert de récepteur au virus ; Un anticorps anti-Hsp90 diminue le pouvoir infectieux du virus in vitro (J. of Virology 79 : 4557, 2005)
xiv) L'atrophie musculaire spinale et bulbaire : (SBMA: Spinal and bulbar muscular atrophy), une maladie neurodégénérative héréditaire caractérisée par une extension de triplets CAG dans le gène du récepteur aux androgènes. Il a été montré que le 17-AAG, un dérivé de la Geldanamycine, présente une activité in vivo sur des animaux transgéniques servant de modèles expérimentaux de cette maladie (Nature Medicine 11 : 1088, 2005).

### Inhibiteurs d'Hsp90 :

Les premiers inhibiteurs connus d'Hsp90 sont des composés de la famille des amsamycines, en particulier la Geldanamycine (1) et l'Herbimycine A. Des études de rayon X ont montré que la Geldanamycine se lie au site ATP du domaine N-terminal d'Hsp90 où elle inhibe l'activité ATPasique de la chaperone (Prodromou C. et al, Cell (1997), 90, 65-75)

Actuellement le NIH et Kosan BioSciences assurent le développement clinique du 17-AAG (2), qui est un inhibiteur d'Hsp90 dérivé de la geldanamycine (1), qui bloque l'activité ATPasique d'Hsp90, en se liant au site de reconnaissance N-terminal de l'ATP. Les résultats des essais cliniques de phase I du 17-AAG (1) conduisent aujourd'hui à initier des essais de phase II, mais orientent aussi les recherches vers des dérivés plus solubles tel que l'analogue 3 (17-DMAG de Kosan BioSciences), porteur d'une chaîne diméthylaminée à la place du résidu méthoxy, et vers des formulations optimisées du 17AAG (CNF1010 de Conforma Therapeutics) :

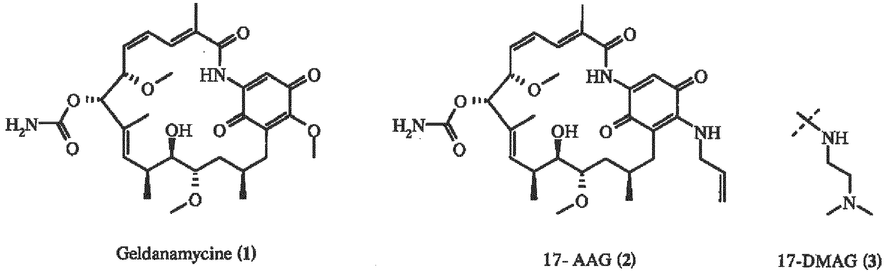

L'analogue réduit du 17-AAG (WO 2005063714 / US 2006019941) est également en études cliniques de phase **I** depuis peu par la société Infinity Pharmaceuticals. De nouveaux dérivés de geldanamycine ont été décrits récemment (WO2006016773 / US6855705 / US 2005026894/ WO2006 /050477 / US 2006205705).

Le Radicicol (4) est également un inhibiteur d'Hsp90 d'origine naturelle (Roe S.M. et al, J. Med Chem. (1999), 42, 260-66). Toutefois, si celui-ci est de loin le meilleur inhibiteur in vitro d'Hsp90, son instabilité métabolique vis à vis des nucléophiles soufrés le rend difficilement utilisable in vivo. Des dérivés oximes bien plus stables tel que le KF 55823 (5) ou le KF 25706 ont été développés par la société Kyowa Hakko Kogyo (Soga et al, Cancer Research (1999), 59, 2931-2938)

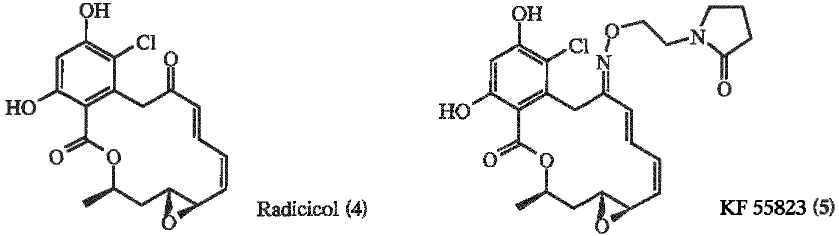

Des structures d'origine naturelle apparentées au radicicol ont également été récemment décrites, comme la zéaralénone (6) par la société Conforma Therapeutics (WO 03041643) ou les composés (7-9).

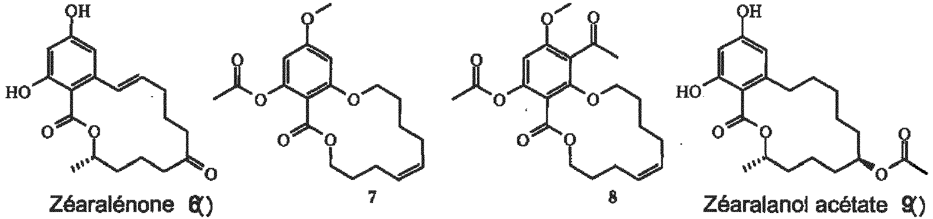

La demande de brevet US2006089495 décrit des composés mixtes comprenant un noyau quinone, comme les dérivés d'amsamycine, et un noyau résorcinol comme les analogues de radicicol, comme inhibiteurs d'Hsp90.

Un inhibiteur d'Hsp90 d'origine naturelle, la novobiocine (10) se lie à un site ATP différent situé dans le domaine C-terminal de la protéine (Itoh H. et al, Biochem J. (1999), 343, 697-703. Récemment des analogues simplifiés de la Novobiocine ont été identifiés comme plus puissants inhibiteurs d'Hsp90 que la Novobiocine elle-même (J. Amer. Chem. Soc. (2005), 127(37), 12778-12779).

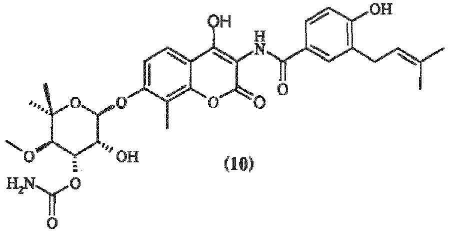

La demande de brevet WO 2006/050501 revendique des analogues de Novobiocine comme inhibiteurs d'Hsp90.

Un depsipeptide, nommé Pipalamycin ou ICI101 a également été décrit comme inhibiteur non compétitif du site ATP d'Hsp90 (J. Pharmacol. Exp. Ther. (2004), 310, 1288-1295).

La Sherperdine, nonapeptide KHSSGCAFL, mime une partie de la séquence K79-K90 (KHSSGCAFLSVK) de la Survivine et bloque l'interaction des protéines de la famille IAP avec Hsp90 in vitro (WO 2006014744).

Des petits peptides, comprenant une séquence de type Otoferline (YSLPGYMVKKLLGA), ont été récemment décrits comme inhibiteurs de Hsp90 (WO 2005072766).

Des purines, comme les composés PU3 (11) (Chiosis et al, Chem. Biol. (2001), 8, 289-299) et PU24FCI (12) (Chiosis et al, Curr. Canc. Drug 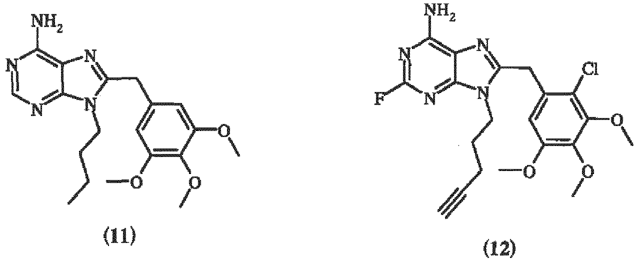 Targets (2003), 3, 371-376; WO 2002/036075) ont également été décrites comme inhibiteurs d'Hsp90 :

Un dérivé de purine CNF2024 (13) a été récemment introduit en clinique par la société Conforma therapeutics, en collaboration avec le Sloan Kettering Memorial Institute for Cancer Research (WO 2006/084030).

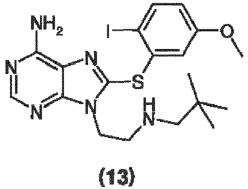

La demande de brevet FR2880540 (Aventis) revendique une autre famille de purines inhibitrices d'Hsp90.

La demande de brevet WO2004/072080 (Cellular Genomics) revendique une famille de 8-hétéroaryl-6-phényl-imidazo[1,2-a]pyrazines comme modulateurs de l'activité d'hsp90.

La demande de brevet WO2005/028434 (Conforma Therapeutics) revendique des aminopurines, des aminopyrrolopyrimidines, des aminopyrazolopyrimidines et des aminotriazolopyrimidines comme inhibiteurs d'Hsp90.

La demande de brevet WO2004/050087 (Ribotarget/Vernalis) revendique une famille de pyrazoles utiles pour traiter des pathologies liées à l'inhibition des « Heat Shock Proteins » tels que la chaperone Hsp90.

La demande de brevet WO2004/056782 (Vernalis) revendique une nouvelle famille de pyrazoles utiles pour traiter des pathologies liées à l'inhibition des « Heat Shock Proteins » tels que la chaperone Hsp90.

La demande de brevet WO2004/07051 (Vernalis) revendique des dérivés d'arylisoxazoles utiles pour traiter des pathologies liées à l'inhibition des « Heat Shock Proteins » tels que la chaperone Hsp90.

La demande de brevet WO2004/096212 (Vernalis) revendique une troisième famille de pyrazoles utiles pour traiter des pathologies liées à l'inhibition des « Heat Shock Proteins » tels que la chaperone Hsp90.

La demande de brevet WO2005/00300 (Vernalis) revendique de manière plus générale des hétérocycles à 5 chaînons, substitués par des radicaux aryles, utiles pour traiter des pathologies liées à l'inhibition des « Heat Shock Proteins » tels que la chaperone Hsp90.

La demande de brevet JP2005/225787 (Nippon Kayaku) revendique une autre famille de pyrazoles comme inhibiteurs d'Hsp90.

La demande WO2006/018082 (Merck) revendique une autre famille de pyrazoles comme inhibiteurs d'Hsp90.

La demande de brevet WO2005/00778 (Kyowa Hakko Kogyo) revendique une famille de dérivés de benzophénone comme inhibiteurs d'Hsp90, utiles pour le traitement des tumeurs.

La demande de brevet WO2005/06322 (Kyowa Hakko Kogyo) revendique une famille de dérivés de résorcinol comme inhibiteurs d'Hsp90.

La demande de brevet WO2005/051808 (Kyowa Hakko Kogyo) revendique une famille de dérivés d'acides résorcinyl-benzoïques comme inhibiteurs d'Hsp90.

Les demandes de brevet WO2005/021552, WO2005/0034950, WO2006/08503 WO2006/079789 et WO2006/090094 (Vernalis) revendiquent des familles de pyrimidothiophènes ou de pyridothiophènes , utiles pour traiter des pathologies liées à l'inhibition des « Heat Shock Proteins » tels que la chaperone Hsp90.

La demande WO2006/010595 (Novartis) revendique une famille d'indazoles comme inhibiteurs d'Hsp90.

La demande WO2006/010594 (Novartis) revendique une famille de dihydrobenzimidazolones comme inhibiteurs d'Hsp90.

La demande de brevet WO2006/055760 (Synta Pharma) revendique une famille de diaryl-triazole comme inhibiteurs d'Hsp90.

La demande de brevet WO2006/087077 (Merck) revendique une famille de (s-triazol-3-yl)phenols comme inhibiteurs d'Hsp90.

La demande de brevet FR2882361 (Aventis) revendique une famille de 3-aryl-1,2-benzisoxazoles comme inhibiteurs d'Hsp90.

La demande de brevet WO2006/091963 (Serenex) revendique des familles de tetrahydroindolones et de tetrahydroindazolone comme inhibiteurs d'Hsp90.

La demande de brevet DE10200509440 (Merck) revendique une famille de thiénopyridines comme inhibiteurs d'Hsp90.

La demande de brevet WO2006/095783 (Nippon Kayaku) revendique une famille de triazoles comme inhibiteurs d'Hsp90.

La demande de brevet WO2006101052 (Nippon Kayaku) revendique une famille de dérivés acétyléniques comme inhibiteurs d'Hsp90.

La demande de brevet WO2006105372 (Conforma Therapeutics) revendique une famille d'alkynyl pyrrolo[2,3-d]pyrimidines comme inhibiteurs d'Hsp90.

La demande de brevet WO2006101052 (Nippon Kayaku) revendique une famille de dérivés acétylèniques comme inhibiteurs d'Hsp90.

La demande de brevet WO2006105372 (Conforma Therapeutics) revendique une famille d'alkynyl pyrrolo[2,3-d]pyrimidines comme inhibiteurs d'Hsp90.

La présente invention concerne des produits de formule (I) : 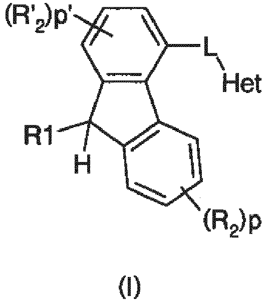 dans lesquels :
Het est choisi parmi les radicaux imidazolyle benzofuranyle, quinoléinyle, pyridinyle, indolyle, benzoxazolyle, pyrimidinyle, triazolopyridinyle, benzoxazinyle, quinoxalinyle, indazolyle, pyrrolopyridinyle, tetrahydro-1,8-naphtyridinyle: ces radicaux étant éventuellement substitués par un ou plusieurs radicaux R identiques ou différents choisis parmi les atomes d'halogène et les radicaux cyano et morpholino:
R1 représente le radical -NH-C(O)-hétéroaryle, avec hétéroaryle choisi parmi les radicaux quinolyle, pyridyle, purines, quinoxaline, pyrazole, pyrimidinyle, pyrrolo[2,3-b]pyridine, pyrrolo[2,3-c]pyrimidine imidazo[4,5-b]pyridine, ces radicaux hétéroaryle étant éventuellement substitués par un ou plusieurs radicaux identiques ou différents choisis parmi les atomes d'halogène, les radicaux méthyle, éthyle, NH2, Nhalk et NH-Me,
R2 et R'2, représentent H,
L représente simple liaison ou C(O),
lesdits produits de formule (I) étant sous toutes les formes tautomères et isomères possibles racémiques, énantiomères et diastéréoisomères, ainsi que les sels d'addition avec les acides minéraux et organiques ou avec les bases minérales et organiques desdits produits de formule (Ib).

Dans les produits de formule (I) et dans ce qui suit, les termes indiqués ont les significations qui suivent :
- le terme halogène désigne les atomes de fluor, de chlore, de brome ou d'iode et de préférence de fluor, chlore ou brome.
- le terme radical alkyle désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés. On cite plus particulièrement les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaires ou ramifiés.
- le terme radical alcoxy désigne un radical linéaire ou ramifié renfermant au plus 12 atomes de carbone et préférentiellement 6 atomes de carbone choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés,
- Le terme alkylthio ou alkyl-S- désigne un radical linéaire ou ramifié renfermant au plus 12 atomes de carbone et représente notamment les radicaux méthylthio, éthylthio, isopropylthio et heptylthio. Dans les radicaux comportant un atome de soufre, l'atome de soufre peut être oxydé en radical SO ou S(O)2.
- le terme radical acyle ou r-CO- désigne un radical linéaire ou ramifié renfermant au plus 12 atomes de carbone dans lequel le radical r représente un atome d'hydrogène, un radical alkyle, cycloalkyle, cycloalkényle, cycloalkyle, hétérocycloalkyle ou aryle, ces radicaux ayant les valeurs indiquées ci-dessus et étant éventuellement substitués comme indiqué : on cite par exemple les radicaux formyle, acétyle, propionyle, butyryle ou benzoyle, ou encore valéryle, hexanoyle, acryloyle, crotonoyle ou carbamoyle
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical cycloalkylalkyle désigne un radical dans lequel cycloalkyle et alkyle sont choisis parmi les valeurs indiquées ci-dessus : ce radical désigne ainsi par exemple les radicaux cyclopropylméthyle, cyclopentylméthyle, cyclohexylméthyle et cycloheptylméthyle.
- par radical acyloxy, on entend les radicaux acyl-O- dans lesquels acyl a la signification indiquée ci-dessus : on cite par exemple les radicaux acétoxy ou propionyloxy.
- par radical acylamino, on entend les radicaux acyl-N- dans lesquels acyl a la signification indiquée ci-dessus.
- le terme radical aryle désigne les radicaux insaturés, monocycliques ou constitués de cycles condensés, carbocycliques. Comme exemples de tel radical aryle, on peut citer les radicaux phényle ou naphtyle.
- par arylalkyle on entend les radicaux résultant de la combinaison des radicaux alkyle cités précédemment éventuellement substitués et les radicaux aryles également cités ci-dessus, éventuellement substitués : on cite par exemple les radicaux benzyle, phényléthyle, 2-phénéthyle, triphénylméthyle ou naphthlèneméthyle.
- le terme radical hétérocyclique désigne un radical carbocylique saturé (hétérocycloalkyle) ou partiellement ou totalement insaturé (hétéroaryle) constitué de 4 à 10 chaînons interrompus par un ou plusieurs hétéroatomes, identiques ou différents, choisis parmi les atomes d'oxygène, d'azote ou de soufre.

Comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridyle, pyrrolidinyle, imidazolidinyle, imidazolidine-2,4-dione, pyrazolidinyle, morpholinyle ou encore tétrahydrofuryle, hexahydropyranne, tétrahydrothiényle, chromanyle, dihydrobenzofuranyle, indolinyle, perhydropyranyle, pyrindolinyle, tétrahydroquinoléinyle, tétrahydroisoquinoléinyle ou thioazolidinyle, tous ces radicaux étant éventuellement substitués.

Parmi les radicaux hétérocycloalkyles, on peut citer notamment les radicaux pipérazinyle éventuellement substitué, N-méthylpipérazinyle, pipéridyle, éventuellement substitués, pyrrolidinyle éventuellement substitué, imidazolidinyle, pyrazolidinyle, morpholinyle, hexahydropyranne ou thioazolidinyle.

Par radical hétérocycloalkylalkyle, on entend les radicaux dans lesquels les restes hétérocycloalkyle et alkyle ont les significations précédentes
parmi les radicaux hétéroaryles à 5 chaînons on peut citer les radicaux furyle, pyrrolyle, tétrazolyle, thiazolyle, isothiazolyle, diazolyle, thiadiazolyle, thiatriazolyle, oxazolyle, oxadiazolyle, isoxazolyle, imidazolyle, pyrazolyle, thiényle, triazolyle.

Parmi les radicaux hétéroaryles à 6 chaînons on peut citer notamment les radicaux pyridyle tel que 2-pyridyle, 3-pyridyle et 4-pyridyle, pyrimidyle, pyridazinyle, pyrazinyle.

Comme radicaux hétéroaryles condensés contenant au moins un hétéroatome choisi parmi le soufre, l'azote et l'oxygène, on peut citer par exemple benzothiényle, benzofuryle, benzopyrrolyle, benzothiazolyle, benzimidazolyle, purinyle, pyrrolopyrimidinyle, pyrolopyridinyle, benzoxazolyle, benzisoxazolyle, benzisothiazolyle, thionaphtyle, chroményle, indolizinyle, quinazolinyle, quinoxalinyle, indolyle, indazolyle, purinyle, quinolyle, isoquinolyle et naphtyridinyle.

Par radical alkylamino, on entend les radicaux dans lesquels le radical alkyl est choisi parmi les radicaux alkyle cités ci-dessus. On préfère les radicaux alkyle ayant au plus 4 atomes de carbone et on peut citer par exemple les radicaux méthylamino, éthylamino, propylamino ou butylamino, linéaire ou ramifié.

Par radical dialkylamino, on entend les radicaux dans lesquels les radicaux alkyl identiques ou différents sont choisis parmi les radicaux alkyle cités ci-dessus. Comme précédemment on préfère les radicaux alkyle ayant au plus 4 atomes de carbone et on peut citer par exemple les radicaux diméthylamino, diéthylamino, méthyléthylamino linéaire ou ramifié.

Le terme patient désigne les êtres humains mais aussi les autres mammifères.

On peut citer à titre d'exemples des esters de produits de formule (I) contenant un groupe hydroxyle tels que les acétates, citrates, lactates, tartrates, malonates, oxalates, salicylates, propionates, succinates, fumarates, maléates, méthylene-bis-b-hydroxynaphthoates, gentisates, iséthionates, di-p-toluoyltartrates, méthanesulfonates, éthanesulfonates, camphorsulfonates, benzenesulfonates, p-toluènesulfonates, cyclohexyl-sulfamates et quinates.

Des esters de produits de formule (I) particulièrement utiles contenant un groupe hydroxyle peuvent être préparés à partir de restes acides tels que ceux décrits par Bundgaard et. al., J. Med. Chem., 1989, 32, page 2503-2507: ces esters incluent notamment des (aminométhyl)-benzoates substitués, dialkylamino-méthylbenzoates dans lesquels les deux groupements alkyle peuvent être liés ensemble ou peuvent être interrompus par un atome d'oxygène ou par un atome d'azote éventuellement substitué soit un atome d'azote alkylé ou encore des morpholino-méthyl)benzoates, e.g. 3- ou 4-(morpholinométhyl)-benzoates, et (4-alkylpiperazin-1-yl)benzoates, e.g. 3- ou 4-(4-alkylpiperazin-1-yl)benzoates.

Le ou les radicaux carboxy des produits de formule (I) peuvent être salifiés ou estérifiés par les groupements divers connus de l'homme du métier parmi lesquels on peut citer, à titre d'exemples non limitatifs, les composés suivants.
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxy-méthyle, propionyloxyméthyle, méthylthiométhyle, diméthyl-aminoéthyle, benzyle ou phénéthyle.

Par carboxy estérifié on entend par exemple les radicaux tels que les radicaux alkyloxycarbonyle par exemple méthoxycarbonyle, éthoxycarbonyle, propoxycarbonyle, butyl ou tert-butyloxycarbonyle, cyclobutyloxycarbonyle, cyclopentyloxycarbonyle ou cyclohexyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié, on entend les radicaux du type -CONH2 dont les atomes d'hydrogène sont éventuellement substitués par un ou deux radicaux alkyle pour former des radicaux alkylamino ou dialkylamino eux-mêmes éventuellement substitués comme indiqué ci-dessus ou ci-dessous, ces radicaux pouvant également former avec l'atome d'azote auquel ils sont liés une amine cyclique tel que définie ci-dessus.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine. On préfère le sel de sodium.

Lorsque les produits de formule (I) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.
On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

La présente invention concerne ainsi plus particulièrement les produits de formule (I) tels que définis ci-dessus dans lesquels Het, R2, R'2, p, p' et L ont l'une quelconque des significations définies ci-dessus et R1 est choisi parmi les radicaux suivants : 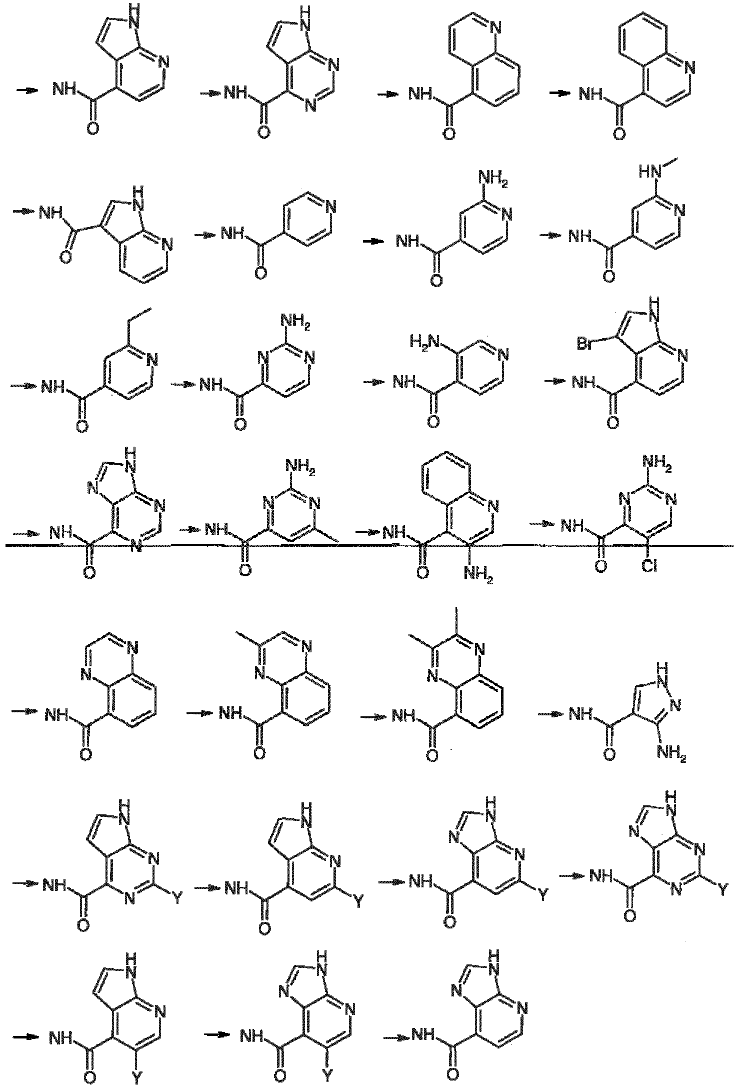 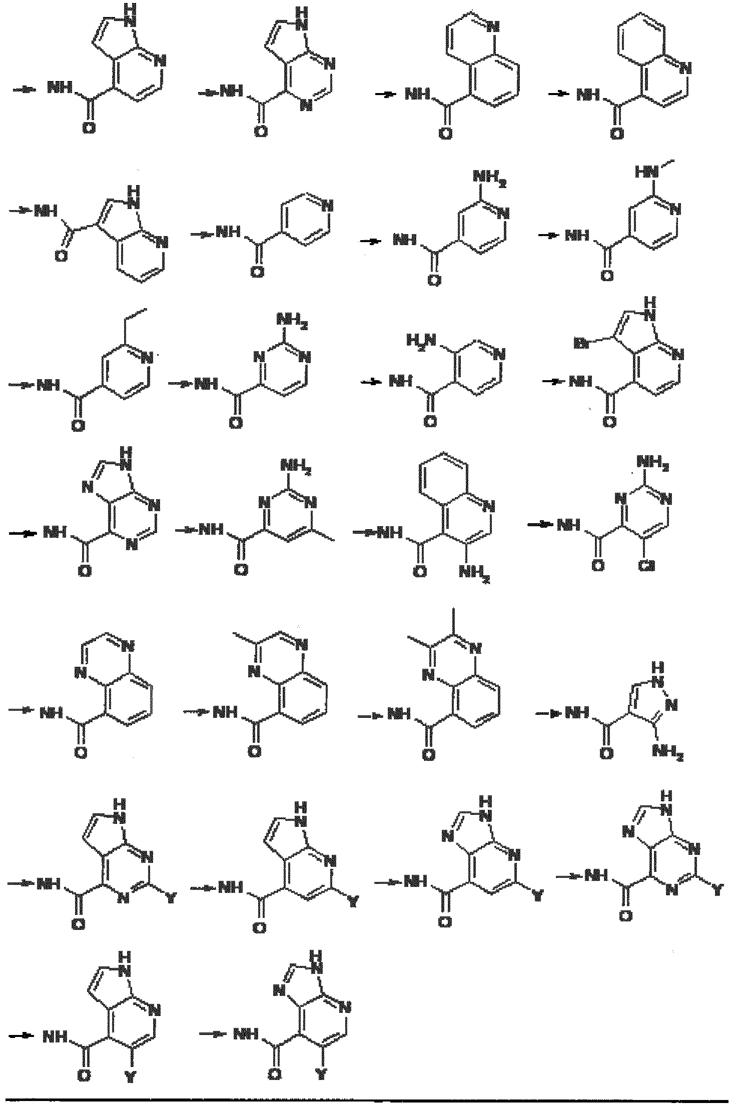 avec Y représente un atome d'halogène ou un radical méthyle ou éthyle, lesdits produits de formule (I) étant sous toutes les formes tautomères et isomères possibles racémiques, énantiomères et diastéréoisomères, ainsi que les sels d'addition avec les acides minéraux et organiques ou avec les bases minérales et organiques desdits produits de formule (I).

La présente invention concerne tout particulièrement les produits de formule (I) tels que définis ci-dessus dont les noms suivent :
- [4-(1H-imidazol-2-yl)-9H-fluorène-9(R,S)-yl]-amide de l'acide 2-amino-5-chloro-pyrimidine-4-carboxylique
- [4-(benzofuran-2-yl)-9H-fluorèn-9(R,S)-yl)]-amide de l'acide 1H-pyrrolo[2,3-b]pyridine-4-carboxylique.
- [4-(quinoléin-3-yl)-9H-fluorèn-9(R,S)-yl)]-amide de l'acide 1H-pyrrolo[2,3-b]pyridine-4-carboxylique
- [4-(6-fluoro-pyridin-3-yl)-9H-fluorèn-9-(R,S)-yl]-amide de l'acide 1H-pyrrolo[2,3-b]pyridine-4-carboxylique.
- [4-(1 H-indol-2-yl)-9H-fluorèn-9-(R,S)-yl]-amide de l'acide 1 H-pyrrolo[2,3-b]pyridine-4-carboxylique
- [4-(benzoxazol-2-yl)-9H-fluorèn-9-(R,S)-yl]-amide de l'acide 1 H-pyrrolo[2,3-b]pyridine-4-carboxylique
- [4-(pyrimidin-5-yl)-9H-fluorèn-9-(R,S)yl]-amide de l'acide 1 H-pyrrolo[2,3-b]pyridine-4-carboxylique
- [4-(quinoléin-2-yl)-9H-fluorèn-9-(R,S)-yl]-amide de l'acide 1H-pyrrolo[2,3-b]pyridine-4-carboxylique
- (4-[1,2,4]triazolo[1,5-a]pyridin-2-yl-9H-fluoren-9(R,S)-yl)-amide de l'acide 1H-pyrrolo[2,3-b]pyridine-4-carboxylique
- [4-(1,4-benzoxazin-2H-3-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 7H-pyrrolo[2,3-c]pyrimidine-4-carboxylique
- [4-(quinoxalin-2-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 6-bromo-3H-imidazo[4,5-b]pyridine-7-carboxylique
- [4-(2-morpholino-pyridin-5-yl)-9H-fluorèn-9(R,S)-yl]-amide de l'acide 1 H-pyrrolo[2,3-b]pyridine-4-carboxylique
- [4-(quinoléin-3-yl)-9H-fluorèn-9(R,S)-yl]-amide de l'acide 2-amino-5-chloro-pyrimidine-4-carboxylique
- [4-(indazole-1-carbonyl)-9H-fluorèn-9(R,S)-yl1-amide de l'acide 1H-pyrrolo[2,3-b]pyridine-4-carboxylique
- [4-(1H-pyrrolo[2,3-b]pyridin-5-yl)-9H-fluorèn-9(R,S)-yl]-amide de l'acide 1H-pyrrolo[2,3-b]pyridine-4-carboxylique
- 4-(5,6,7,8-tetrahydro-1,8-naphtyridin-3-yl)-9H-fluorèn-9(R,S)-yl]-amide de l'acide 1H-pyrrolo[2,3-b]pyridine-4-carboxylique
- 4-{imidazo[1,2-a]pyridin-2-yl}-9H-fluorèn-9-(R,S)-yl-amide de l'acide 1H-pyrrolo[2,3-b]pyridine-4-carboxylique
lesdits produits de formule (I) étant sous toutes les formes isomères possibles racémiques, énantiomères et diastéréo-isomères, ainsi que les sels d'addition avec les acides minéraux et organiques ou avec les bases minérales et organiques.

La présente invention divulgue les produits de fomule (I) répondant à la fomule (la) dans laquelle L représente une simple liaison et les produits de fomule (I) répondant à la fomule (Ib) dans laquelle L représente CH2, C(O), O, S ou NH représentés comme suit : 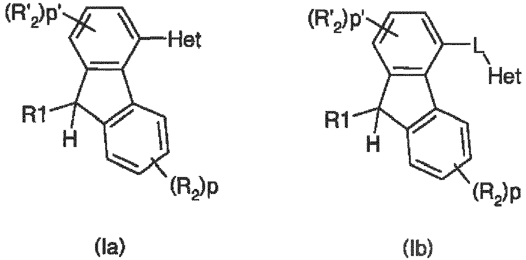 dans lesquels Het, R1, R2, R2', p et p' ont les valeurs définies ci-dessus pour les produits de fomule (I)
lesdits produits de formule (Ia) ou (Ib) étant sous toutes les formes tautomères et isomères possibles racémiques, énantiomères et diastéréoisomères, ainsi que les sels d'addition avec les acides minéraux et organiques ou avec les bases minérales et organiques desdits produits de formule (la) ou (Ib).

Les produits de formule (I) répondant à la formule (la) ou (Ib) peuvent être préparés selon les méthodes connues de l'homme du métier et particulièrement selon les méthodes décrites ci-après: la présente invention décrit ainsi également les méthodes de synthèse des produits de formule (la) ou (Ib) notamment les méthodes générales de synthèses décrites dans les schémas ci-après.

### Méthodes générales de synthèses des produits de formule générale (Ia) :

### Réaction de couplage à partir d'une 4-halo-fluorène-9-one :

Une première méthode générale de synthèse consiste à coupler une 9H-4-halo-fluorèn-9-one - telle que la 9H-4-bromo-fluorèn-9-one, qui peut être obtenue selon J. Amer. Chem. Soc. 1935, , 2443-6 ou la 9H-4-iodo-fluorèn-9-one, qui peut être obtenue selon Helv. Chim. Acta 1973, 3044-9 - ou le triflate de la 9-H-4-hydroxy-fluorè-9-one avec un dérivé organométallique d'hétérocycle, selon le schéma 1 :

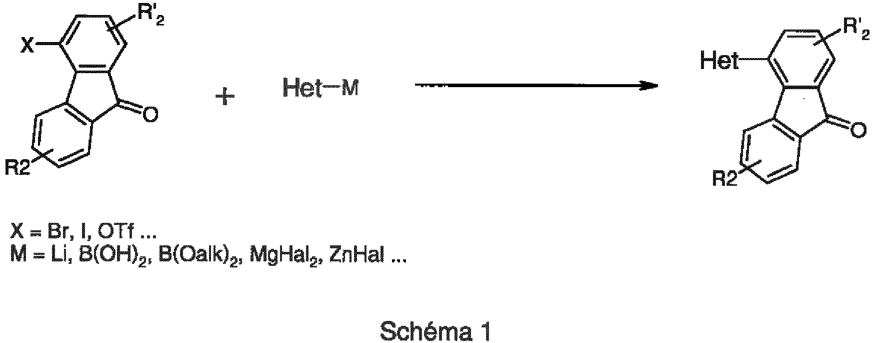

Dans le cadre de l'invention, il est particulièrement avantageux d'utiliser un acide boronique comme dérivé organométallique d'hétérocycle.

Dans le cadre de l'invention, il est particulièrement avantageux d'effectuer le couplage en présence d'un catalyseur dérivé de palladium (0), dans les conditions d'une réaction de type Suzuki.

Réaction de couplage à partir d'un dérivé organométallique de fluorèn-9-one-4-yle: Dans une deuxième méthode générale de synthèse, le couplage inverse peut être envisagé, en particulier en utilisant un hétérocycle bromé ou iodé et un dérivé organométallique de fluorèn-9-one, tel que l'acide 9-oxofluorène-4-boronique ou l'un de ses esters, selon le schéma 2 :

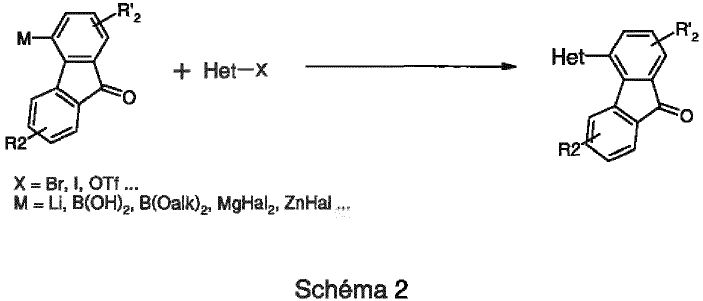

### Formation de l'hétérocycle à partir d'un dérivé d'acide ou d'aldéhyde de fluorèn-9-one-4-yle :

Dans une troisième méthode générale de synthèse, lorsque ledit hétérocycle est de type benzimidazole, benzoxazole, ou benzothiazole , attaché au dérivé de fluorène par sa position 2, il est particulièrement avantageux de former ledit hétérocycle par couplage d'un dérivé d'orthophénylènediamine ou d'orthoaminophénol ou d'orthoaminothiophénol avec un acide, un chlorure d'acide ou un aldéhyde en position 4 d'une fluorèn-9-one, suivi de cyclisation, selon le schéma 3 :

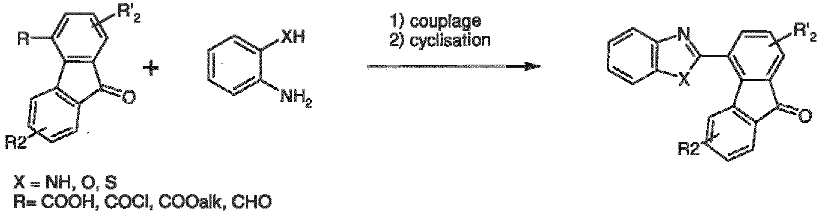

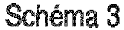

Lorsqu'on utilise un acide fluorèn-9-one-4-carboxylique, il est particulièrement avantageux d'activer cet acide à l'aide d'un agent de couplage connu de l'homme de l'art, tel que chlorhydrate de 1-(3-diméthylaminopropyl)-3-éthylcarbodiimide (EDCI), en présence de 1-hydroxybenzotriazole (HOBT). Diverses conditions de cyclisation du mélange d'amides intermédiaires peuvent être utilisées dans le cadre de l'invention, tels que l'acide acétique ou un mélange d'acide et d'anhydride trifluoroacétique. Il est également particulièrement avantageux dans le cadre de l'invention d'effectuer ce type de cyclisation thermique en milieu acide par chauffage dans un réacteur micro-onde.

Lorsqu'on utilise un dérivé de fluorèn-9-one-4-carboxaldéhyde, il est avantageux dans le cadre de l'invention, d'opérer :
- soit par chauffage micro-onde en présence de silice, selon Tetrahedron Lett. 1998 , 39, 4481-84 ;
- soit en présence de dichloro-dicyano-benzoquinone (DDQ), selon Tetrahedron 1995, 51, 5813-18 ;
- soit en présence d'un mélange de chlorure de thionyle et de pyridine, selon E.P.511187;
- soit en présence de chlorure ferrique, selon Eur. J. Med. Chem. 2006, 31, 635-42.

Lorsque ledit hétérocycle est de type imidazole, oxazole, ou thiazole, attaché au dérivé de fluorène par sa position 2, il est particulièrement avantageux de former ledit hétérocycle à partir d'un acide, d'un chlorure d'acide d'un ester ou d'un aldéhyde en position 4 d'une fluorèn-9-one, en opérant selon le schéma 4:

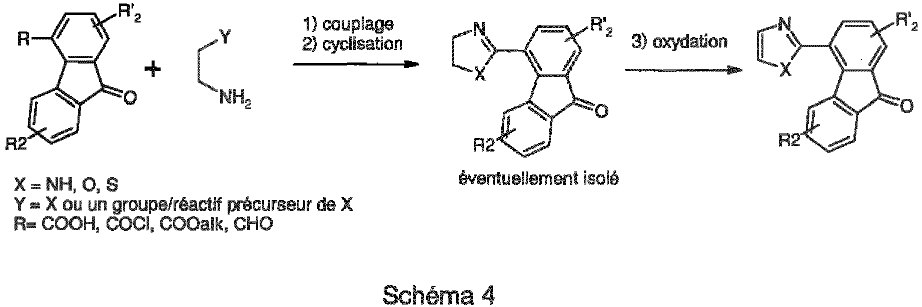

Dans le cadre de l'invention il est particulièrement avantageux d'opérer :
1. dans le cas où ledit hétérocycle est un imidazole ou une imidazoline :
   - à partir d'une 2-azido-éthylamine, selon Tetrahedron, 47(38), 1991, 8177-94,
   - d'une éthylènediamine, selon Biorg. Med. Chem Lett. 12(3), 2002, 471-75,
   - de glyoxal et d'ammoniaque, selon J. Med. Chem., 46(25), 2003, 5416-27 ;
2. dans le cas où ledit hétérocycle est un oxazole ou une oxazoline :
   - à partir d'un 2-azido-éthanol, selon J. Org. Chem., 61(7), 1996, 2487-96,
   - d'un 2-aminoéthanol, selon J. Med. Chem. 47(8), 2004,1969-86 ou Khim. Geterosikl. Soed. 1984(7), 881-4,
   - de diéthylacétal de 2-aminoacétaldéhyde, selon Heterocycles, 39(2), 1994, 767-78 ;
3. dans le cas où ledit hétérocycle est thiazole ou une thiazoline :
   - à partir d'une 2-chloro-éthylamine et de réactif de Lawesson, selon Helv. Chim. Acta, 88(2), 2005,187-95,
   - d'un 2-aminoéthanethiol, selon J. Org. Chem. 69(3), 2004, 811-4, ou Tetrahedron Lett., 41 (18), 2000, 3381-4,

D'une manière plus générale, il est avantageux dans le cadre de l'invention, de former un hétérocycle quelconque à partir d'un acide, d'un chlorure d'acide d'un ester ou d'un aldéhyde en position 4 d'une fluorèn-9-one, par l'une quelconque des méthodes de synthèse connues de l'homme de l'art, telles que celles décrites dans Comprehensive Organic Chemistry, par D. Barton et al. (Pergamon Press) ou Advances in Heterocyclic Chemistry (Academic Press) ou Heterocyclic Compounds (Wiley Intersciences).

La transformation du radical C=O en radicaux CHR1 tels que définis dans la formule générale (Ia) peut être effectuée selon les méthodes générales connues de l'homme de l'art, en particulier celles décrites dans Comprehensive Organic Chemistry, par D. Barton et al. (Pergamon Press), Advanced Organic Chemistry, par J. Marsh (Wiley Interscience), ou Compendium of Organic Synthetic methods (Wiley Interscience).

Réaction de couplage à partir d'un dérivé bromé ou organométallique de fluorèn-4-yle substitué en position 9 par un radical CHR1: Une quatrième méthode générale de synthèse consiste à effectuer d'abord la transformation du radical C=O d'un dérivé fluorèn-9-one, substitué en position 4, par un halogène, un triflate, un boronate ou un acide boronique 9-oxo-fluorène-4-carboxylique, en radical CHR1, tel que défini dans la formule générale (la), puis à coupler le dérivé obtenu avec un dérivé d'hétérocycle convenablement substitué par un halogène, un boronate ou acide boronique.

Formation de l'hétérocycle à partir d'un dérivé d'acide ou d'aldéhyde de fluorèn-4-yle substitué en position 9 par un radical CHR1 : Une cinquième méthode générale de synthèse consiste à former l'hétérocycle après avoir introduit préalablement le radical CHR1 sur un acide, un ester, un chlorure d'acide ou un aldéhyde en position 4 de noyau fluorène.

Lorsque ledit hétérocycle est de type benzimidazole, benzoxazole, ou benzothiazole, attaché au dérivé de fluorène par sa position 2, il est avantageux d'effectuer d'abord la transformation du radical C=O d'un dérivé fluorèn-9-one, substitué en position 4, par un dérivé d'acide carboxylique - acide, chlorure d'acide ou ester - ou par un aldéhyde, en radical CHR1, tel que défini dans la formule générale (I), puis à coupler le dérivé obtenu avec un dérivé d'o.phénylènediaminediamine ou d'o.aminophénol ou d'o.aminothiophénol, selon les conditions décrites précédemment.

Lorsque ledit hétérocycle est de type imidazole, oxazole, ou thiazole, attaché au dérivé de fluorène par sa position 2, il est avantageux d'effectuer d'abord la transformation du radical C=O d'un dérivé fluorèn-9-one, substitué en position 4, par un dérivé d'acide carboxylique - acide, chlorure d'acide ou ester - ou par un aldéhyde, en radical CHR1, tel que défini dans la formule générale (I), puis à former l'hétérocycle selon les méthodes décrites précédemment.

D'une manière plus générale, il est avantageux dans le cadre de l'invention, de former un hétérocycle quelconque à partir à partir d'un acide, d'un chlorure d'acide d'un ester ou d'un aldéhyde en position 4 d'un noyau fluorène substitué en position 9 par un radical CHR1, par l'une quelconque des méthodes de synthèse connues de l'homme de l'art, telles que celles décrites dans Comprehensive Organic Chemistry, par D. Barton et al. (Pergamon Press) ou Advances in Heterocyclic Chemistry (Academic Press) ou Heterocyclic Compounds (Wiley Intersciences).

### Préparation des composés de formule générale (Ib) dans lesquels L = CO .

Les composés de formule générale (Ib), dans lesquels L = CO, et pour lesquels l'hétérocycle Het est fixé via un atome de carbone, peuvent être avantageusement préparés par une réaction de type Friedel-Crafts selon le schéma 6 :
- soit par action d'un acide ou d'un chlorure d'acide fluorèn-9-one-4-carboxylique sur un hétérocycle suffisamment riche en électrons, notamment selon la méthode la méthode décrite dans Eur. J. Med. Chem 1988, 23(2), 165-72 ;
- soit par action d'un chlorure d'acide hétérocyclique sur un dérive de fluorèn-9-one.

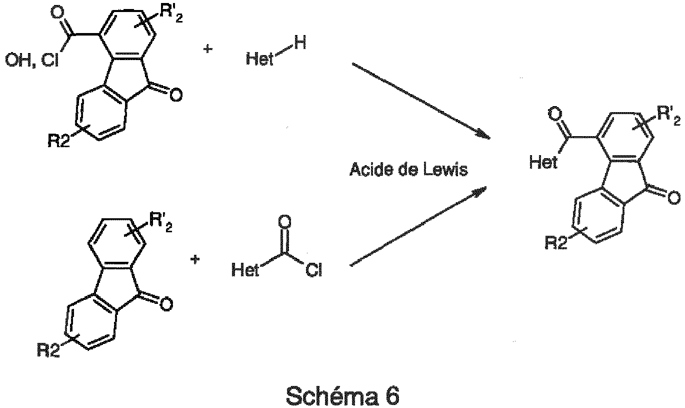

Les composés de formule générale (Ib), dans lesquels L = CO, et pour lesquels l'hétérocycle Het est fixé via un atome d'azote, tels que par exemple les hétérocycles de type pyrrole, pyrazole, imidazole, indole, isoindole, indazole, benzimidazole ou imidazo[4,3-c]pyridine, peuvent être avantageusement préparés par couplage d'un anion dérivé dudit hétérocycle avec un chlorure d'acide fluorèn-9-one-4-carboxylique selon le schéma 7 :

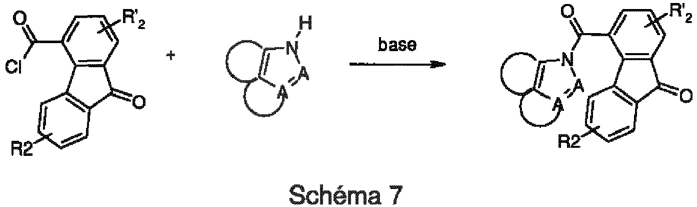

Les composés de formule générale (Ib), dans lesquels L = CH2 peuvent être avantageusement préparés selon l'une des méthodes générales décrites dans le schéma 8,
- soit par couplage entre un dérivé de 4-halogénométhyl-fluorèn-9-one et un composé organique hétérocyclique ;
- soit par cyclisation entre un dérivé d'acide (flurèn-9-one-4-yl)acétique et un noyau aromatique ou hétéroaromatique ortho-disubstitué :

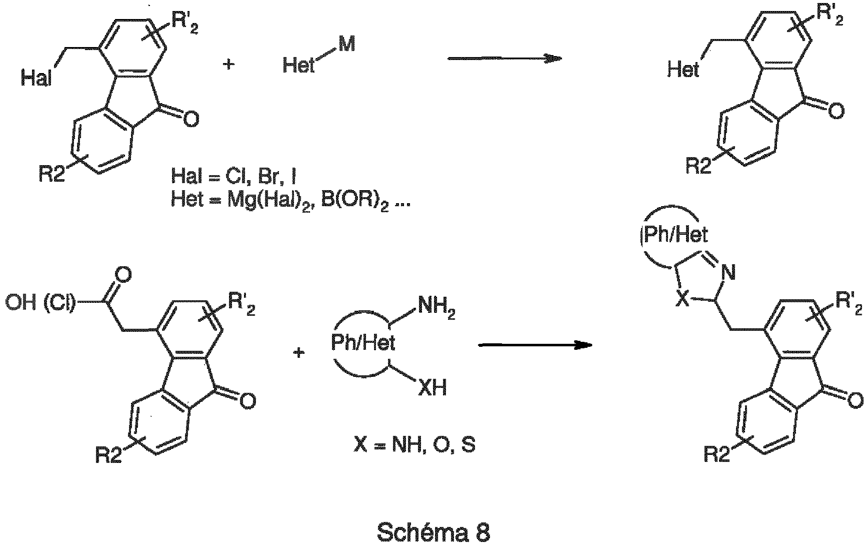

Les composés de formule générale (Ib), dans lesquels L = O peuvent être préparés selon l'une quelconque des méthodes générales de synthèse d' aryl(hétéroaryl)éthers connues de l'homme de l'art, notamment celles utilisant une catalyse avec de l'iodure de cuivre, en partant :
- soit d'une 4-halogéno-fluorèn-9-one et d'un sel alcallin d'hydroxyhétérocycle ;
- soit d'un sel alcallin de 4-hydroxy-fluorèn-9-one et d'un halogénure d'hétéroaryle.

Les composés de formule générale (Ib), dans lesquels L = S peuvent être préparés selon l'une quelconque des méthodes générales de synthèse d' aryl(hétéroaryl)thioéthers connues de l'homme de l'art, notamment celles utilisant une catalyse avec de l'acétate de palladium, en partant :
- soit d'une 4-halogéno-fluorèn-9-one et d'un sel alcallin de mercaptohétérocycle ;
- soit d'un sel alcalin de 4-mercapto-fluorèn-9-one et d'un halogénure d'hétéroaryle.

La transformation du radical C=O en radicaux CHR1 tels que définis dans la formule générale (Ib) dans lesquels L = CO, CH2, O et S peut être effectuée selon les méthodes générales connues de l'homme de l'art, en particulier celles décrites dans Comprehensive Organic Chemistry, par D. Barton et al. (Pergamon Press), Advanced Organic Chemistry, par J. Marsh (Wiley Interscience), ou Compendium of Organic Synthetic methods (Wiley Interscience), selon le schéma 9 :

Pour préparer les composés de formule générale (Ib) dans lesquels L = NH, il est particulièrement avantageux dans le cadre de l'invention, d'inverser la séquence réactionnelle, en transformant d'abord le radical C=O en radical CHR1, à partir d'un dérivé de 4-nitro-fluorèn-9-one, puis en réduisant le groupe nitro en fonction amine primaire, et enfin en effectuant une hétéroarylation de ladite amine primaire avec un bromure ou un iodure hétérocyclique en présence de palladium(0), dans les conditions de la réaction d'Hartwig-Buchwald, selon le schéma général 10 :

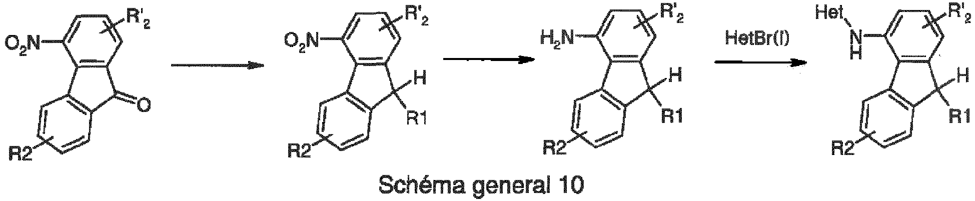

Les réactions décrites ci-dessus peuvent être réalisées selon les conditions décrites dans la préparation des exemples ci-après et également selon les méthodes générales connues de l'homme de l'art, en particulier celles décrites dans : Comprehensive Organic Chemistry, par D. Barton et al. (Pergamon Press) ; Advanced Organic Chemistry, par J. Marsh (Wiley Interscience).

Les produits objet de la présente invention sont doués de propriétés pharmacologiques intéressantes: on a constaté qu'ils possédaient notamment des propriétés inhibitrices des activités des protéines chaperones et notamment de leurs activités ATPasiques.

Parmi ces protéines chaperones, on cite notamment la chaperone humaine HSP90.

Les produits répondant à la formule générale (I) tels que définis ci-dessus présentent ainsi une activité inhibitrice de la chaperone Hsp90 importante.

Des tests donnés dans la partie expérimentale ci-après illustrent l'activité inhibitrice de produits de la présente invention vis-à-vis de telles protéines chaperones.

Ces propriétés rendent donc les produits de formule générale (I) de la présente invention utilisables comme médicaments pour le traitement de tumeurs malignes.

Les produits de formule (I) peuvent également être utilisés dans le domaine vétérinaire.

L'invention a donc pour objet l'application, à titre de médicaments, des produits de formule générale (I) pharmaceutiquement acceptables.

L'invention a particulièrement pour objet l'application à titre de médicaments, des produits de formule (I) tels que définis ci-dessus dont les noms suivent :
- [4-(1H-imidazol-2-yl)-9H-fluorène-9(R,S)-yl]-amide de l'acide 2-amino-5-chloro-pyrimidine-4-carboxylique
- [4-(benzofuran-2-yl)-9H-fluorèn-9(R,S)-yl)]-amide de l'acide 1H-pyrrolo[2,3-b]pyridine-4-carboxylique.
- [4-(quinoléin-3-yl)-9H-fluorèn-9(R,S)-yl)]-amide de l'acide 1H-pyrrolo[2,3-b]pyridine-4-carboxylique
- [4-(6-fluoro-pyridin-3-yl)-9H-fluorèn-9-(R,S)-yl]-amide de l'acide 1H-pyrrolo[2,3-b]pyridine-4-carboxylique.
- [4-(1 H-indol-2-yl)-9H-fluorèn-9-(R,S)-yl]-amide de l'acide 1H-pyrrolo[2,3-b]pyridine-4-carboxylique
- [4-(benzoxazol-2-yl)-9H-fluorèn-9-(R,S)-yl]-amide de l'acide 1 H-pyrrolo[2,3-b]pyridine-4-carboxylique
- [4-(pyrimidin-5-yl)-9H-fluorèn-9-(R,S)yl]-amide de l'acide 1H-pyrrolo[2,3-b]pyridme-4-carboxylique
- [4-(quinoléin-2-yl)-9H-fluorèn-9-(R,S)-yl]-amide de l'acide 1 H-pyrrolo[2,3-b]pyridine-4-carboxylique
- (4-[1,2,4]triazolo[1,5-a]pyridin-2-yl-9H-fluoren-9(R,S)-yl)-amide de l'acide 1H-pyrrolo[2,3-b]pyridine-4-carboxylique
- [4-(1,4-benzoxazin-2H-3-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 7H-pyrrolo[2,3-c]pyrimidine-4-carboxylique
- [4-(quinoxalin-2-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 6-bromo-3H-imidazo[4,5-b]pyridine-1-carboxylique
- [4-(2-morpholino-pyridin-5-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 2-amino-5-chloropyrimidine-4-carboxylique
- [4-(indazole-1-carbonyl)-9H-fluorèn-9(R,S)-yl]-amide de l'acide 1H-pyrrolo[2,3-blpyridine-4-carboxylique
- [4-(1H-pyrrolo[2,3-b]pyridin-5-yl)-9H-fluorèn-9(R,S)-yl]-amide de l'acide 1H-pyrrolo[2,3-b]pyridine-4-carboxylique
- 4-(5,6,7,8-tetrahydro-1,8-naphtyridin-3-yl)-9H-fluorèn-9(R,S)-yl]-amide de l'acide 1H-pyrrolo[2,3-b]pyridine-4-carboxylique
- 4-{imidazo[1,2-a]pyridin-2-yl}-9H-fluorèn-9-(R,S)-yl-amide de l'acide 1H-pyrrolo[2,3-b]pyridine-4-carboxylique
étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I).

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivées paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

La présente invention concerne ainsi l'utilisation de produits de formule (I) tels que définis ci -dessus ou de sels pharmaceutiquement acceptables desdits produits de formule (I) pour la préparation de médicaments destinés à inhiber l'activité de protéines chaperones et notamment d'Hsp90.

La présente invention concerne ainsi particulièrement l'utilisation de produits de formule (I) telle que définie ci-dessus ou de sels pharmaceutiquement acceptables desdits produits de formule (I) dans laquelle la protéine chaperone est HSP90.

La présente invention concerne ainsi l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité d'une protéine chaperone de type Hsp90 et notamment une telle maladie chez un mammifère.

La présente invention concerne l'utilisation de produits de formule (I) telle que définie ci-dessus ou de sels pharmaceutiquement acceptables desdits produits de formule (I) pour la préparation d'un médicament destiné à prévenir ou traiter une maladie appartenant au groupe suivant: les maladies neurodégénératives telles que la maladie de Huntington, la maladie de Parkinson, l'ischémie cérébrale focale, la maladie d'Alzheimer, la sclérose en plaques et la sclérose latérale amyotrophique, la malaria, les filarioses de Brugia et de Bancroft, la toxoplasmose, les mycoses résistantes aux traitements, l'hépatite B, l'hépatite C, le virus de l'Herpes, la dengue (ou grippe tropicale), l'atrophie musculaire spinale et bulbaire, désordres de la prolifération de cellules mésangiales, thromboses, rétinopathies, psoriasis, dégénération musculaire, maladies en oncologie, cancers.

La présente invention concerne ainsi l'utilisation de produits de formule (I) telle que définie ci-dessus ou de sels pharmaceutiquement acceptables desdits produits de formule (I) pour la préparation d'un médicament destiné à traiter des maladies en oncologie.

La présente invention concerne particulièrement l'utilisation de produits de formule (I) telle que définie ci-dessus ou de sels pharmaceutiquement acceptables desdits produits de formule (I) pour la préparation d'un médicament destiné à traiter des cancers.

Parmi ces cancers, la présente invention s'intéresse tout particulièrement au traitement des tumeurs solides et au traitement de cancers résistants aux agents cytotoxiques

La présente invention concerne ainsi notamment l'utilisation de produits de formule (I) telle que définie à l'une quelconque des revendications précédentes ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloîdes chroniques, les leucémies lymphoblastiques aigûes, les cancers de la prostate, du pancréas et du colon, les mélanomes métastatiques, les tumeurs de la thyroîde et les carcinomes rénaux.

Aussi parmi les principales indications potentielles d'inhibiteurs d'Hsp90, peut-on citer, à titre non limitatif:
- les cancers du poumon « à non petites cellules », les cancers du sein, les cancers de l'ovaire été les glioblastomes qui surexpriment EGF-R ou HER2 ;
- les leucémies myéloîdes chroniques qui surexpriment Bcr-Abl ;
- les leucémies lymphoblastiques aigûes qui surexpriment Flt-3 ;
- les cancers du sein, de la prostate, du poumon, du pancrés, du colon ou de l'ovaire qui surexpriment Akt ;
- les mélanomes metastatiques et les tumeurs de la thyroïde qui surexpriment la forme mutée de la protéine B-Raf ;
- les cancers de la prostate androgène-dépendants et androgène-indépendants ;
- les cancers du sein estrogène-dépendants et estrogène-indépendants ;
- les carcinomes rénaux qui surexpriment HIF-1a ou la protéine c-met mutée.

La présente invention s'intéresse encore plus particulièrement au traitement du cancer du sein, du colon et des poumons.

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou eh association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR , les antimétabolites antinéoplastique, les composés du platine, les inhibiteurs de protéasome, comme le Bortezomib, les inhibiteurs d'Histone Déactylase (HDACs), comme le SAHA, et notamment les inhibiteurs d'HDAC6, les composés faisant décroître l'activité des protéines kinases et également les composés anti-angiogéniques, les agonistes de la gonadoréline, les anti-androgènes, les bengamides, les biphophonates et le trastuzumab.

On peut citer ainsi à titre d'exemples, des agents anti-microtubules, comme les taxoides, les epothilones, les vinka-alkaloides, des agents d'alkylation tels que cyclophosphamide, des agents DNA-intercalant comme le cis-platinum, et l'oxaliplatine, des agents interactifs sur topoisomérase comme la camptothécine et dérivés, les anthracyclines comme l'adriamycine, des antimétabolites comme le 5-fluorouracile et dérivés et analogues.

Les produits de formule (I) selon la présente invention peuvent être préparés par l'application ou l'adaptation de méthodes connues et notamment des méthodes décrites dans la littérature comme par exemple celles décrites par R.C.Larock dans: Comprehensive Organic Transformations, VCH publishers, 1989.

Dans les réactions décrites ci-après, il peut être nécessaire de protéger des groupes fonctionnels réactifs tels que par exemple des groupements hydroxy, amino, imino, thio ou carboxy, quand ceux-ci sont désirés dans le produit final mais quand leur participation n'est pas souhaitée dans les réactions de synthèse des produits de formule (I).On peut utiliser des groupes protecteurs conventionnels en accord avec les pratiques usuelles standard comme ceux décrits par exemple par T.W. Greene and P.G.M.Wuts dans "Protective Groups in Organic Chemistry" John Wiley and Sons, 1991.

La partie expérimentale ci-après donne des exemples non limitatifs de produits de départ: d'autres produits de départ peuvent être trouvés dans le commerce ou préparés selon les méthodes usuelles connues de l'homme du métier.

Exemples illustrant l'invention : Les exemples dont la préparation suit illustrent la présente invention sans toutefois la limiter.

Tous les exemples décrits ont été caractérisés par spectroscopie RMN du proton et par spectroscopie de masse, la majorité de ces exemples ont également été caractérisés en spectroscopie Infra Rouge.

### Exemple 1 : Synthèse de [4-(1H-imidazol-2-yl)-9H-fluorène-9(R,S)-yl]-amide de l'acide 2-amino-5-chloro-pyrimidine-4-carboxylique

Etape 1 : Dans un tricol de 250 mL, on dissout 1,5 g de 2-azido-éthylamine, qui peut être préparée selon Tetrahedron, 47(38), 1991, 8177-8194, et 3,5 g de chlorure de l'acide fluorène-4-one-9-carboxylique dans 100 mL de dichlorométhane et 4 mL de triéthylamine. Après 20 heures d'agitation à température ambiante, le milieu réactionnel est versé sur de l'eau et extrait au dichlorométhane. Les phases organiques sont lavées avec une solution aqueuse saturée d'hydrogénocarbonate de sodium puis de l'eau, séchées sur sulfate de magnésium et concentrées sous pression réduite. Le résidu obtenu est empâté à l'oxyde de diisopropyle, filtré et séché au dessicateur à 35°C. On obtient ainsi 3,65 g du (2-azido-éthyl)-amide de l'acide 9-oxo-9H-fluorène-4-carboxylique sous forme d'une poudre jaune, utilisé tel quel à l'étape suivante, dont la caractéristique est la suivante :
Spectre de masse (E/I) : m/z = 292 (M+)

Etape 2 : Dans un ballon de 250 mL sous atmosphère d'argon, on agite, à température ambiante pendant une nuit, une solution de 3,65 g de (2-azido-éthyl)-amide de l'acide 9-oxo-9H-fluorène-4-carboxylique, obtenu à l'étape précédente, 4 g de difertbutyldicarbonate et 1,5 g de diméthylaminopyridine dans 55 mL de tétrahydrofurane. Le milieu réactionnel est versé sur de l'eau et extrait à l'acétate d'éthyle. Les phases organiques sont lavées avec une solution aqueuse de dihydrogénophosphate de sodium puis avec une solution aqueuse saturée de chlorure de sodium, séchées sur sulfate de magnésium et concentrées sous pression réduite. On obtient ainsi 5,25 g de l'ester *tert*-butyle de l'acide (2-azido-éthyl)-(9-oxo-9H-fluorène-4-carbonyl)carbamique sous forme d'une huile orange, utilisée tel quel à l'étape suivante, dont la caractéristique est la suivante :
Spectre de masse (LCMS) : m/z = 392 (M+)

Etape 3 : Dans un ballon de 250 mL sous atmosphère d'argon, on agite à température ambiante pendant 20 heures, une solution de 4,4 g de l'ester *tert*-butyle de l'acide (2-azido-éthyl)-(9-oxo-9H-fluorène-4-carbonyl) carbamique, obtenu à l'étape précédente, et 2,9 g de triphénylphosphine dans un mélange de 52 mL de toluène et 13 mL de dichlorométhane. On amène à sec sous pression réduite. On purifie par flash-chromatographie sur gel de silice (40-63µm), en éluant par un mélange de dichlorométhane et d'acétate d'éthyle (95/5 puis 9/1 en volumes), on obtient 1.29 g de l'ester *tert-*butyle de l'acide (4,5-dihydro-imidazole)-2-(9-oxo-9H-fluorène-4-yl)-1-carboxylique, sous forme d'une poudre jaune utilisée telle quelle à l'étape suivante, dont la caractéristique est la suivante :
Spectre RMN 1 H (400 MHz, δ en ppm, DMSO-d6) : 0,93 (s, 9H) ; 4,00 (s large, 4H) ; 7,36 (d, J = 7,5 Hz, 1H) ; de 7,39 à 7,46 (m, 2H) ; 7,53 (dd, J = 1,5 et 7,5 Hz, 1 H) ; 7,61 (dt, J = 1,5 et 7,5 Hz, 1 H) ; de 7,66 à 7,70 (m, 2H).

Etape 4 : Dans un ballon de 50 mL sous atmosphère d'argon, on refroidit à 0°C une solution de 1,29 g de l'ester *tert*-butyle de l'acide (4,5-dihydro-imidazole)-2-(9-oxo-9H-fluorène-4-yl)-1-carboxylique, obtenu à l'étape précédente, dans 20 mL de dichlorométhane et l'on ajoute goutte à goutte 8,5 mL d'acide trifluoroacétique. On laisse remonter à température ambiante et on agite pendant 20 heures. On amène à sec sous pression réduite. On ajoute du toluène et on amène à sec sous pression réduite. Le résidu obtenu est empâté dans l'oxyde de diisopropyle, filtré et séché au dessicateur à 35°C. On obtient 1,65 g (100%) du trifluoroacétate de l'acide 4-(4,5-dihydro-1H-imidazol-2-yl)-fluorène-9-one, sous forme d'une poudre jaune utilisée telle quelle à l'étape suivante, dont la caractéristique est la suivante :
Spectre de masse (EI/CI) : m/z = 362 (M+).

Etape 5: Dans un tricol de 100 mL sous atmosphère d'argon, on refroidit à -65°C, une solution de 0,65 mL de chlorure d'oxalyle dans 5 mL de dichlorométhane, puis on ajoute, goutte à goutte, 0,94 mL de diméthylsulfoxyde sur tamis moléculaire 3A. On agite pendant 10 minutes, puis on ajoute, goutte à goutte, une solution de 1.65 g de trifluoroacétate de l'acide 4-(4,5-dihydro-1 H-imidazol-2-yl)-fluorène-9-one dans 15 mL de dichlorométhane puis 5 mL de triéthylamine. On agite à -65°C pendant une heure, puis on laisse remonter à température ambiante et on agite 2 heures. Le milieu réactionnel est versé sur de l'eau, extrait au dichlorométhane. Les phases organiques sont lavées avec une solution aqueuse saturée de chlorure de sodium, séchées sur sulfate de magnésium et amenées à sec sous pression réduite. Après purification par flash-chromatographie sur gel de silice (40-63µm), en éluant par un mélange de dichlorométhane et de méthanol (98/2 en volumes), on obtient 560 mg de 4-(1 H-imidazol-2-yl)-fluorène-9-one, sous forme d'une poudre jaune utilisée telle quelle à l'étape suivante, dont la caractéristique est la suivante :
Spectre RMN 1H (400 MHz, δ en ppm, DMSO-d6) : De 7,13 à 7,33 (m étalé, 2H) ; 7,37 (t, J = 7,5 Hz, 1H) ; de 7,45 à 7,53 (m, 2H) ; 7,64 (d large, J = 7,5 Hz, 1H) ; 7,69 (d, J = 7,5 Hz, 2H) ; 7,83 (d large, J = 7,5 Hz, 1H) ; 12,65 (s large, 1 H).

Etape 6 : On opère comme à l'étape 2 de l'exemple 5, à partir de 560 mg de 4-(1 H-imidazol-2-yl)-fluorène-9-one, obtenue à l'étape précédente, de 474 mg de chlorhydrate d'hydroxylamine et de 933 mg d'acétate de sodium dans 12 mL d'éthanol pendant 20 heures à température ambiante. Après concentration du solvant sous pression réduite, le résidu est repris successivement par de l'eau, puis du toluène et enfin empâté dans de l'oxyde diisopropyle. On obtient ainsi 449 mg de 4-(1H-imidazol-2-yl)-fluorène-9-one oxime (Z,E), en mélange 50-50 des isomères Z et E, sous forme d'une poudre jaune pâle, dont la caractéristique est la suivante :
Spectre de masse (El) : m/z = 261 (M+).

Etape 7 : On opère en autoclave comme dans l'étape 3 de l'exemple 5, à partir de 449 mg du mélange équimoléculaire des isomères Z et E de la 4-(1 H-imidazol-2-yl)-fluorène-9-one oxime, obtenue à l'étape précédente, dans un mélange de 25 mL d'éthanol et 25 mL de tétrahydrofurane, en présence de Nickel activé selon Raney, sous une pression initiale d'hydrogène de 1 bar, à 60°C pendant 3 heures. Après filtration du catalyseur sur Célite, concentration du filtrat sous pression réduite et purification par empâtage à l'oxyde de diisopropyle, on obtient 367 mg de 4-(1 H-imidazol-2-yl)-9H-fluorène-9(R,S)-amine, sous forme d'une poudre blanche, dont les caractéristiques sont les suivantes :
Spectre de masse (El) : m/z = 247 (M+).
Spectre RMN 1 H (400 MHz, δ en ppm, DMSO-d6) : 2,29 (m étalé, 2H) ; 4,76 (s, 1H); 7,14 (s large, 1H) ; 7,16 (t partiellement masqué, J = 7,5 Hz, 1H) ; 7,28 (t, J = 7,5 Hz, 1 H) ; 7,31 (s large, 1 H) ; de 7,36 à 7,42 (m, 3H) ; 7,65 (d, J = 7,5 Hz, 1 H) ; 7,74 (m, 1H) ; 12,4 (s large, 1 H).

Etape 8 : On opère comme dans l'étape 4 de l'exemple 5, à partir de 100 mg de 4-(1H-imidazol-2-yl)-9H-fluorène-9(R,S)-amine, obtenue à l'étape précédente, et 74 mg d'acide 2-amino-5-chloro-pyrimidine-4-carboxylique dans 2 mL de diméthylformamide, en présence de 116 mg de chlorhydrate de 1-(3-diméthylaminopropyl)-3-éthyfcarbodümide (EDCI) et 82 mg de 1-hydroxybenzotriazole (HOBT), pendant 20 heures à température ambiante. Après purification par flash-chromatographie sur gel de silice (40-63µm), en éluant avec un mélange de dichlorométhane et de méthanol (98/2 puis 95/5 en volumes), on obtient 52 mg de [4-(1 H-imidazol-2-yl)-9H-fluorène-9(R,S)-yl]-amide de l'acide 2-amino-5-chloro-pyrimidine-4-carboxylique, sous forme d'une poudre blanc cassé dont les caractéristiques sont les suivantes :
Spectre de masse (EI/CI/LCMS) : m/z = 402 (M+).
Spectre RMN 1 H (400 MHz, δ en ppm, DMSO-d6) : 6,20 (d, J = 8,5 Hz, 1H) ; 7,10 (s large, 2H) ; 7,17 (s large, 1 H) ; 7,27 (t, J = 7,5 Hz, 1 H) ; 7,34 (m, 2H) ; 7,45 (t, J = 7,5 Hz, 1H) ; 7,48 (d, J = 7,5 Hz, 1H) ; 7,54 (d, J = 7,5 Hz, 1H) ; 7,60 (m, 2H) ; 8,38 (s, 1H) ; 9,25 (d, J = 8,5 Hz, 1 H) ; 12,45 (s large, 1H).

### Exemple 2 : Synthèse du [4-(benzofuran-2-yl)-9H-fluorèn-9(R,S)-yl)]-amide de l'acide 1H-pyrrolo[2,3-b]pyridine-4-carboxylique.

*Etape 1* : Dans un réacteur photochimique contenant une lampe de 125 W, on introduit successivement 5 g d'acide 9-fluorènone-4-carboxylique, 22 g de diacétate d'iodobenzène, 17 g d'iode disublimé et 450 mL de tétrachlorure de carbone. Après 20 heures de chauffage à 78°C et sous radiation, on ajoute 300 mL d'une solution aqueuse de thiosulfate de sodium à 10% et agite pendant 15 minutes. Le précipité est éliminé (produit de départ non réagit) et la phase organique du filtrat est purifiée par flash-chromatographie sur gel de silice (20-40 µm) en éluant avec un mélange de cyclohexane et d'acétate d'éthyle (90-10 en volumes). On obtient ainsi, 1,59 g de 4-iodo-fluorèn-9-one sous forme d'un solide jaune dont les caractéristiques sont les suivantes :
Spectre de masse (E/I) : m/z = 306 (M+)
Spectre RMN 1H (400 MHz,δ en ppm, DMSO-d6) : 7,14 (t, J = 8,0 Hz, 1H) ; 7,48 (d large, J = 7,5 Hz, 1 H) ; 7,66 (dd, J = 1,0 et 7,5 Hz, 1 H) ; 7,69 (d large, J = 8,0 Hz, 1H); 7,74 (dt, J = 1,0 et 7,5 Hz, 1 H) ; 8,05 (dd, J = 1,0 et 7,5 Hz, 1H) ; 8,59 (d large, J = 8,0 Hz, 1H).

*Etape 2 :* Dans un tube pour micro-ondes, on dissout 1,2 g de 4-iodo-fluorène-9-one, obtenue à l'étape 1, dans 30 mL d'éthanol, puis on ajoute successivement 0,52 g de chlorure de bis (triphénylphosphine) palladium (II), 0,6 g d'acide benzofurane-2-boronique et 0,5 mL de triéthylamine. Après 6 minutes de réaction à 140°C, on concentre à sec, reprend au dichlorométhane et à l'eau, extrait par deux fois 20 mL de dichlorométhane, sèche sur sulfate de sodium, filtre et concentre à sec. Le solide brut obtenu est purifié par flash-chromatographie sur gel de silice (20-40 µm) en éluant avec un mélange de cyclohexane et d'acétate d'éthyle (98-02 en volumes). On obtient ainsi 0,64 g de 4-(benzofuran-2-yl)-fluorèn-9-one sous forme d'un solide jaune, utilisé tel quel dans l'étape suivante dont la caractéristique est la suivante :
Spectre de masse (E/I) : m/z = 296 (M+)
Spectre RMN 1H (400 MHz,δ en ppm, DMSO-d6) : 7,27 (d, J = 8,0 Hz, 1 H) ; de 7,34 à 7,46 (m, 4H) ; de 7,49 à 7,56 (m, 2H) ; 7,71 (m, 2H) ; 7,78 (m, 2H) ; 7,83 (d, J = 8,0 Hz, 1H).

*Etape 3*: Dans un tricol de 100 mL, on dissout 0,64 g de 4-(benzofuran-2-yl)-fluorèn-9-one, obtenue à l'étape 2, dans 20 mL d'éthanol, puis on ajoute successivement 0,45 g de chlorhydrate d'hydroxylamine et 0,88 g d'acétate de sodium sec. Après une nuit d'agitation à température ambiante, on concentre à sec, ajoute 20 mL d'eau. Le précipité formé est essoré, lavé à l'eau et séché sous hotte. On obtient ainsi 0,58 g du mélange équimoléculaire des oximes Z et E de la 4-(benzofuran-2-yl)-fluorèn-9-one, sous forme d'une poudre jaune, dont les caractéristiques sont les suivantes :
Point de fusion (Kofler) : 188°C
Spectre de masse (E/I) : m/z = 311 (M+)

*Etape 4 :* Dans un autoclave de 100 mL, on dissout 0,58 g de mélange équimoléculaire des isomères Z et E de la 4-(benzofuran-2-yl)-fluorèn-9-one, obtenue à l'étape 3, dans un mélange de 24 mL d'éthanol et 24 mL de tétrahydrofurane, on ajoute une pointe de spatule de Nickel activé selon Raney puis on soumet à une pression initiale d'hydrogène de 1 bar et on chauffe l'autoclave à 60°C pendant 3 heures. Après refroidissement, le filtrat est concentré sous pression réduite. On obtient ainsi 0,4 g de 4-(benzofuran-2-yl)-9H-fluorèn-9(R,S)-yl)-amine, sous forme d'un semi-solide, utilisé tel quel dans l'étape suivante et dont la caractéristique est la suivante :
Spectre de masse (E/I) : m/z = 297 (M+)

*Etape 5:* Dans un tricol de 25 mL, on dissout 400 mg de 4-(benzofuran-2-yl)-9H-fluorén-9(R,S)-yl)-amine, obtenue à l'étape 4, dans 9 mL de diméthylformamide, puis on ajoute successivement 283 mg de chlorhydrate de 1-(3-diméthylaminopropyl)-3-éthylcarbodiimide (EDCI), 90 mg de 1-hydroxybenzotriazole (HOBT) et 218 mg d'acide 1H-pyrrolo[2,3-b]pyridine-4-carboxylique puis on agite 20 heures à température ambiante. On ajoute alors 50 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (20-40 µm) en éluant avec un mélange de dichlorométhane et de méthanol (90-10 en volumes). On obtient ainsi 59 mg de [4-(benzofuran-2-yl)-9H-fluorèn-9(R,S)-yl)]-amide de l'acide 1H-pyrrolo[2,3-b]pyridine-4-carboxylique sous forme d'un solide beige dont les caractéristiques sont les suivantes :
Point de fusion (Kofler) > 260°C
Spectre de masse (E/I) : m/z = 441 (M+)
Spectre RMN 1 H (400 MHz, δ en ppm, DMSO-d6) : 6,41 (d, J = 8,5 Hz, 1 H) ; 6,90 (s large, 1 H) ; 7,19 (d, J = 8,0 Hz, 1 H) ; 7,25 (s, 1H) ; 7,28 (t partiellement masqué, J = 8,0 Hz, 1 H) ; de 7,32 à 7,53 (m, 5H) ; 7,62 (m, 3H) ; 7,69 (d, J = 8,0 Hz, 1H) ; 7,73 (d, J = 8,0 Hz, 1 H) ; 7,79 (d, J = 8,0 Hz, 1 H) ; 8,29 (d, J = 5,0 Hz, 1H) ; 9,25 (d, J = 8,5 Hz, 1 H) ; 11,85 (m large, 1H).

### Exemple 3 : Synthèse du [4-(quinoléin-3-yl)-9H-fluorèn-9(R,S)-yl)]-amide de l'acide 1H-pyrrolo[2,3-b]pyridine-4-carboxylique.

*Etape 1 :* Dans un tube pour micro-ondes, on dissout 1,1 g de 4-iodo-fluorèn-9-one, obtenue comme à l'étape 1 de l'exemple 2, dans 25 mL d'éthanol, puis on ajoute successivement 0,46 g de chlorure de (triphénylphosphine) palladium (II), 0,57 g d'acide quinoléin-3-boronique et 0,9 mL de triéthylamine. Après 8 minutes de réaction à 140°C, on concentre à sec, reprend au dichlorométhane et à l'eau, extrait par deux fois 20 mL de dichlorométhane, sèche sur sulfate de magnésium, filtre et concentre à sec. Le solide brut obtenu est purifié par flash-chromatographie sur gel de silice (20-40 µm) en éluant avec un mélange de cyclohexane et d'acétate d'éthyle (90-10 en volumes). On obtient ainsi, 0,53 g de 4-(quinoléin-3-yl)-fluorèn-9-one sous forme d'un solide jaune, utilisé tel quel dans l'étape suivante dont la caractéristique est la suivante :
Spectre de masse (E/I) : m/z = 307 (M+)

*Etape 2 :* Dans un tricol de 50 mL, on dissout 0,53 g de 4-(quinoléine-3-yl)-fluorèn-9-one, obtenue à l'étape 1, dans 15 mL d'éthanol, puis on ajoute successivement 0,36 g de chlorhydrate d'hydroxylamine et 0,7 g d'acétate de sodium sec. Après une nuit d'agitation à température ambiante, on concentre à sec, ajoute 20 mL d'eau. Le précipité formé est essoré, lavé à l'eau et séché sous hotte. On obtient ainsi 0,51 g (92%) du mélange équimoléculaire des oximes Z et E de la 4-(quinoléin-3-yl)-fluorèn-9-one, sous forme d'une poudre jaune, utilisé tel quel dans l'étape suivante et dont les caractéristiques sont les suivantes :
Point de fusion (Kofler) : 204°C
Spectre de masse (E/I) : m/z = 322 (M+)

*Etape 3 :* Dans un autoclave de 100 mL, on dissout 0,51 g de mélange équimoléculaire des isomères Z et E de la 4-(quinoléin-3-yl)-fluorèn-9-one, obtenue à l'étape 2, dans un mélange de 20 mL d'éthanol et 20 mL de tétrahydrofurane, on ajoute une pointe de spatule de Nickel activé selon Raney puis on soumet à une pression initiale d'hydrogène de 1 bar et on chauffe l'autoclave à 60°C pendant 8 heures. Après refroidissement, on filtre le catalyseur sur Célite. Le filtrat est concentré sous pression réduite. Après purification par flash-chromatographie sur gel de silice (20-40 µm) en éluant avec un mélange de dichlorométhane et de méthanol (95-05 en volumes). On obtient ainsi 0,18 g de [4-(quinoléin-3-y)I-9H-fluorèn-9(R,S)-yl]-amine, sous forme d'une meringue blanche dont la caractéristique est la suivante :
Spectre de masse (E/I) : m/z = 308 (M+)

*Etape 4 :* Dans un tricol de 25 mL, on dissout 180 mg de [4-(quinoléin-3-yl)-9H-fluorèn-9(R,S)-yl]-amine, obtenue à l'étape 3, dans 4 mL de diméthylformamide, puis on ajoute successivement 123 mg de chlorhydrate de 1-(3-diméthylaminopropyl)-3-éthylcarbodiimide (EDCl), 40 mg de 1-hydroxybenzotriazole (HOBT) et 104 mg d'acide 1 H-pyrrolo[2,3-b]pyridine-4-carboxylique puis on agite 20 heures à température ambiante. On ajoute alors 30 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (20-40 µm) en éluant avec un mélange de dichlorométhane et de méthanol (90-10 en volumes). On obtient ainsi 130 mg de [(4-quinoléin-3-yl)-9H-fluorèn-9(R,S)-yl]-amide de l'acide 1H-pyrrolo[2,3-b]pyridine-4-carboxylique sous forme d'un solide blanc dont les caractéristiques sont les suivantes :
Point de fusion (Kofler) > 260°C
Spectre de masse (E/I) : m/z = 452 (M+)
Spectre RMN 1 H (400 MHz, δ en ppm, DMSO-d6) : 6,43 (d, J = 8,5 Hz, 1H) ; 6,74 (dt, J = 8,0 Hz, 1 H) ; 6,92 (dd, J = 2,0 et 3,5 Hz, 1H) ; 7,12 (t, J = 7,5 Hz, 1 H) ; 7,29 (t, J = 7,5 Hz, 1 H) ; 7,43 (d, J = 7,5 Hz, 1H) ; 7,47 (d, J = 5,0 Hz, 1H) ; 7,49 (t, J = 7,5 Hz, 1H) ; de 7,60 à 7,67 (m, 2H) ; de 7,70 à 7,75 (m, 2H) ; 7,88 (t large, J = 7,5 Hz, 1H) ; 8,12 (d, J = 8,5 Hz, 1H) ; 8,17 (d, J = 8,5 Hz, 1H) ; 8,30 (d, J = 5,0 Hz, 1 H) ; 8,53 (m étalé, 1 H) ; 9,01 (m étalé, 1H) ; 9,26 (d, J = 8,5 Hz, 1H) ; 11,85 (m large, 1H).

### Exemple 4 : Synthèse du [4-(6-fluoro-pyridin-3-yl)-9H-fluorèn-9-(R,S)-yl]-amide de l'acide 1H-pyrrolo[2,3-b]pyridine-4-carboxylique.

*Etape 1 :* Dans un tube pour micro-ondes, on dissout 0,6 g de 4-Iodo-fluorèn-9-one, obtenue comme à l'étape 1 de l'exemple 2, dans 12 mL d'éthanol, puis on ajoute successivement 0,28 g de chlorure de bis (triphénylphosphine) palladium (II), 0,29 g d'acide 2-fluoropyridin-5-boronique et 0,55 mL de triéthylamine. Après 6 minutes de réaction à 140°C, on concentre à sec, reprend au dichlorométhane et à l'eau, extrait par deux fois 20 mL de dichlorométhane, sèche sur sulfate de magnésium, filtre et concentre à sec. Le solide brut obtenu est purifié par flash-chromatographie sur gel de silice (20-40 µm) en éluant avec un mélange de cyclohexane et d'acétate d'éthyle (95-05 en volumes). On obtient ainsi 0,28 g de 4-(6-fluoro-pyridin-3-yl)-fluorèn-9-one sous forme d'un solide jaune dont les caractéristiques sont les suivantes :
Point de fusion (Kofler) : 138°C
Spectre de masse (E/I) : m/z = 275 (M+)

*Etape 2:* Dans un tricol de 50 mL, on dissout 0,28 g de 4-(6-fluoro-pyridin-3-yl)-fluorèn-9-one obtenue à l'étape 1, dans 10 mL d'éthanol, puis on ajoute successivement 0,21 g de chlorhydrate d'hydroxylamine et 0,41 g d'acétate de sodium sec. Après 4 heures d'agitation à température ambiante, on concentre à sec, ajoute 100 mL d'eau. Le précipité formé est essoré, lavé à l'eau et séché sous hotte. On obtient ainsi 0,27 g du mélange équimoléculaire des oximes Z et E de la 4-(6-fluoro-pyridin-3-yl)-fluorèn-9-one, sous forme d'un solide jaune pâle, utilisé tel quel dans l'étape suivante et dont la caractéristique est la suivante :
Spectre de masse (E/I) : m/z = 290 (M+)

*Etape 3:* Dans un autoclave de 50 mL, on dissout 0,27 g de mélange équimoléculaire des isomères Z et E de la 4-(6-fluoro-pyridin-3-yl)-fluorèn-9-one, obtenue à l'étape 2, dans un mélange de 10 mL d'éthanol et 10 mL de tétrahydrofurane, on ajoute une pointe de spatule de Nickel activé selon Raney puis on soumet à une pression initiale d'hydrogène de 1 bar et on chauffe l'autoclave à 60°C pendant 3 heures. Après refroidissement, le concentré sous pression réduite. On obtient ainsi, 0,23 g de 4-(6-fluoro-pyridin-3-yl-9H-)-fluorèn-9(R,S)-yl]-amine, sous forme d'une laque jaune, utilisée telle quelle et dont la caractéristique est la suivante :
Spectre de masse (E/I) : m/z = 276 (M+)

*Etape 4:* Dans un tricol de 25 mL, on dissout 230 mg de 4-(6-fluoro-pyridin-3-yl-9H-)-fluorèn-9(R,S)-yl]-amine, obtenue à l'étape 3, dans 7 mL de diméthylformamide, puis on ajoute successivement 175 mg de chlorhydrate de 1-(3-diméthylaminopropyl)-3-éthylcarbodiimide (EDCI), 57 mg de 1-hydroxybenzotriazole (HOBT) et 149 mg d'acide 1H-pyrrolo[2,3-b]pyridine-4-carboxylique puis on agite 20 heures à température ambiante. On ajoute alors 30 mL d'eau, et on essore le précipité formé qui est ensuite lavé à l'eau puis avec une solution saturée d'hydrogénocarbonate de sodium. Le solide brut obtenu est purifié par flash-chromatographie sur gel de silice (20-40 µm) en éluant avec un mélange de dichlorométhane et d'éthanol (95-05 en volumes). On obtient ainsi 184 mg de [4-(6-fluoro-pyridin-3-yl)-9H-fluorèn-9-(R,S)-yl]-amide de l'acide 1H-pyrrolo[2,3-b]pyridine-4-carboxylique, sous forme d'un solide rosé dont les caractéristiques sont les suivantes :
Point de fusion (Kofler) > 260°C
Spectre de masse (E/I) : m/z = 420 (M+)
Spectre RMN 1 H (400 MHz, δ en ppm, DMSO-d6) : 6,40 (d, J = 8,5 Hz, 1 H) ; 6,80 (d, J = 7,5 Hz, 1 H) ; 6,91 (dd, J = 2,0 et 3,5 Hz, 1 H) ; 7,21 (t, J = 7,5 Hz, 1 H) ; de 7,29 à 7,34 (m, 2H) ; 7,41 (dd, J = 2,5 et 8,5 Hz, 1 H) ; 7,55 (d, J = 5,0 Hz, 1 H) ; 7,56 (t, J = 7,5 Hz, 1 H) ; 7,61 (m, 2H) ; 7,65 (d, J = 7,5 Hz, 1H) ; 8,11 (m étalé, 1 H) ; 8,28 (d, J = 5,0 Hz, 1 H) ; 8,35 (m étalé, 1H) ; 9,23 (d, J = 8,5 Hz, 1 H) ; 11,85 (m large, 1H).

Exemple 5 : [4-(1H-indo)-2-yl)-9H-fluorèn-9-(R,S)-yl]-amide de l'acide 1 H-pyrrolo[2,3-b]pyridine-4-carboxylique

*Etape 1:* Dans un tricol de 100 mL, sous atmosphère d'Argon, on introduit successivement 0,59 g de 4-iodo-fluorèn-9-one, obtenue comme à l'étape 1 de l'exemple 2, 0,22 g de palladium (0) tétrakis (triphénylphosphine), 0,6 g de carbonate de sodium, 0,5 g d'acide 1-(tert-butoxycarbonyl)-indole-2 boronique, 2,8 mL d'eau et 25 mL dioxanne. Après 1h30 de chauffage à 100°C, on refroidit puis on ajoute du dichlorométhane et de l'eau, lave par trois fois 30 mL d'eau, sèche sur sulfate de magnésium, filtre et concentre à sec. Le solide brut obtenu est purifié par flash-chromatographie sur gel de silice (20-40 µm) en éluant avec un mélange de cyclohexane et d'acétate d'éthyle (98-02 en volumes). On obtient ainsi 0,44 g d'ester tertbutylique de l'acide 2-(9-oxo-9H-fluorèn-4-yl)-indole-1-carboxylique sous forme d'un solide jaune dont les caractéristiques sont les suivantes :
Point de fusion (Kofler) : 170°C
Spectre de masse (E/I) : m/z = 395 (M+)

*Etape 2:* Dans un monocol de 50 mL, on dissout 0,44 g d' ester tertbutylique de l'acide 2-(9-oxo-9H-fluorèn-4-yl)-indole-1-carboxylique obtenue à l'étape 1, dans 10 mL d'éthanol, puis on ajoute successivement 0,23 g de chlorhydrate d'hydroxylamine et 0,45 g d'acétate de sodium sec. Après 20 heures d'agitation à température ambiante, on concentre à sec, ajoute 100 mL d'eau. Le précipité formé est essoré, lavé à l'éther de pétrole et séché sous hotte. On obtient ainsi 0,4 g du mélange équimoléculaire des oximes Z et E de l'ester *tert*.butylique de l'acide 2-(9-oxo-9H-fluorèn-4-yl)-indole-1-carboxylique, sous forme d'un solide jaune pâle, utilisé tel quel dans l'étape suivante et dont les caractéristiques sont les suivantes : »
Point de fusion (Kofler) : 130°C
Spectre de masse (E/I) : m/z = 410 (M+)

*Etape 3:* Dans un autoclave de 100 mL, on dissout 0,4 g de mélange équimoléculaire des isomères Z et E d'ester *tert*.butylique de l'acide 2-(9-oxo-9H-fluorèn-4-yl)-indole-1-carboxylique, obtenue à l'étape 2, dans un mélange de 12 mL d'éthanol et 12 mL de tétrahydrofurane, on ajoute une pointe de spatule de Nickel activé selon Raney puis on soumet à une pression initiale d'hydrogène de 1 bar et on chauffe l'autoclave à 60°C pendant 8 heures. Après refroidissement, on filtre le catalyseur sur Célite. Le filtrat est concentré sous pression réduite. On obtient ainsi 0,38 g d'ester *tert*.butylique de l'acide 2-[9(R,S)-amino-9H-fluorèn-4-yl-]-indole-1-carboxylique, sous forme d'une laque blanche, utilisée telle quelle et dont la caractéristique est la suivante :
Spectre de masse (E/I) : m/z = 396 (M+)

*Etape 4:* Dans un tricol de 25 mL, on dissout 375 mg d'ester *tert*.butylique de l'acide 2-[9(R,S)-amino-9H-fluorèn-4-yl-]-indole-1-carboxylique, obtenue à l'étape 3, dans 8 mL de diméthylformamide, puis on ajoute successivement 200 mg de chlorhydrate de 1-(3-diméthylaminopropyl)-3-éthylcarbodiimide (EDCI), 65 mg de 1-hydroxyberizotriazole (HOBT) et 169 mg d'acide 1 H-pyrrolo[2,3-b]pyridine-4-carboxylique puis on agite 20 heures à température ambiante. On ajoute alors 200 mL d'eau, et on essore le précipité formé qui est ensuite lavé à l'eau, avec une solution saturée d'hydrogénocarbonate de sodium puis avec de l'éther de pétrole. On obtient ainsi 400 mg d'ester *tert*.butylique de l'acide 2-{(R,S)-9-[(1H-pyrrolo[2,3-b]pyridine-4-carbonyl)-amino]-9H-fluorèn-4-yl}-indole-1-carboxylique sous forme d'un solide jaune pâle, utilisé tel quel et dont la caractéristique est la suivante :
Spectre de masse (E/I) : m/z = 540 (M+)

*Etape 5:* On dissout 85 mg d'ester *tert*.butyle de l'acide 2-{-9-(R,S)-[(1H-pyrrolo[2,3-b]pyridine-4-carbonyl)-amino]-9H-fluorèn-4-yl}-indole-1-carboxylique, obtenu à l'étape 4, dans 2 mL de dichlorométhane et on ajoute 0,2 mL d'acide trifluoroacétique. Après 20 heures d'agitation à température ambiante, on concentre à sec, puis lave par une solution d'hydrogénocarbonate de sodium, le brut est purifié par flash-chromatographie sur gel de silice (20-40 µm) en éluant avec un mélange de dichlorométhane et de méthanol (95-05 en volumes). On obtient ainsi 35 mg de [4-(1H-indol-2-yl)-9H-fluorène-9(R,S)-yl]-amide de l'acide 1 H-pyrrolo[2,3-b]pyridine-4-carboxylique sous forme d'une poudre blanche, dont les caractéristiques sont les suivantes :
Spectre de masse (LC/MS) : m/z = 440 (M+).
Spectre RMN 1 H (400 MHz,□ en ppm, DMSO-d6) : 6,41 (d, J = 8,5 Hz, 1 H) ; 6,59 (s large, 1 H) ; 6,91 (d large, J = 3,5 Hz, 1H) ; de 7,02 à 7,11 (m, 2H) ; de 7,13 à 7,22 (m, 2H) ; 7,31 (t, J = 7,5 Hz, 1H) ; de 7,41 à 7,51 (m, 4H) ; de 7,57 à 7,69 (m, 4H) ; 8,29 (d, J = 5,0 Hz, 1 H) ; 9,24 (d, J = 8,5 Hz, 1 H) ; 11,55 (m large, 1 H) ; 11,85 (m large, 1H).

### Exemple 6: [4-(benzoxazol-2-yl)-9H-fluorèn-9-(R,S)-yl]-amide de l'acide 1H-pyrrolo[2,3-b]pyridine-4-carboxylique

*Etape 1*: Dans un tricol de 500 mL, on dissout 2,5 g de 2-amino-phénol dans 120 mL de dichlorométhane, puis on ajoute successivement à température ambiante, 2,8 mL de triéthylamine et 2,5 g de chlorure de l'acide fluorèn-4-one-9-carboxylique. Après 20 heures d'agitation à température ambiante, on verse sur 150 mL d'eau, extrait avec 20 mL de dichlorométhane, lave avec une solution d'hydrogénocarbonate de sodium, à l'eau puis après séchage sur sulfate de magnésium et purification par flash-chromatographie sur gel de silice (40-63 µm), en éluant avec un mélange de dichlorométhane et de méthanol (98-2 en volumes), on obtient 1,8 g de (2-hydroxy-phényl)-amide de l'acide 9-oxo-9H-fluorèn-4-carboxylique utilisé tel quel à l'étape suivante.
Spectre de masse (E/I) : m/z = 315(M+).

Etape 2: Dans un réacteur à micro-ondes, on chauffe, à 200°C pendant 30 minutes, une solution de 1,5 g de (2-hydroxy-phényl)-amide de l'acide 9-oxo-9H-fluorèn-4-carboxylique obtenu à l'étape précédente, dans 80 mL d'acide acétique. Après refroidissement, on concentre à l'évaporateur rotatif et purifie le résidu par flash-chromatographie sur gel de silice (40-63 µm), en éluant avec du dichlorométhane, on obtient ainsi, 0,2 g de (4-benzoxazol-2-yl)-9H-fluorèn-9-one, sous forme d'un solide jaune, et dont les caractéristiques sont les suivantes :
Spectre de masse (E/I) : m/z = 297(M+).

*Etape 3* : On opère comme à l'étape 3 de l'exemple 2, à partir de 280 mg de (4-benzoxazol-2-yl)-9H-fluorèn-9-one, obtenue à l'étape précédente, de 196 mg de chlorhydrate d'hydroxylamine et de 386 mg d'acétate de sodium agités à température ambiante pendant 48 heures dans 12 mL d'éthanol. Après lavage à l'eau et à l'éther de pétrole du précipité formé, on obtient après filtration et séchage 220 mg de (4-benzoxazol-2-yl)-9H-fluorèn-9-one oxime, en mélange 50-50 des isomères Z et E, sous forme d'un solide blanc cassé, dont les caractéristiques sont les suivantes :
Point de fusion (Kofler) : 206°C
Spectre de masse (E/I) : m/z = 312(M+).

*Etape 4 :* Dans un autoclave de 50 mL, on dissout 0,22 g de mélange équimoléculaire des isomères Z et E de 4-(benzoxazol-2-yl)-fluorèn-9-one, obtenue à l'étape 3, dans un mélange de 12 mL d'éthanol et 12 mL de tétrahydrofurane, on ajoute une pointe de spatule de Nickel activé selon Raney puis on soumet à une pression initiale d'hydrogène de 1 bar et on chauffe l'autoclave à 60°C pendant 2 heures. Après refroidissement, on filtre le catalyseur sur Célite. Le filtrat est concentré sous pression réduite. On obtient ainsi, 0,21 g de [4-(benzoxazol-2-yl)-9H-fluorèn-9-(R,S)-yl]-amine sous forme d'une laque brune, utilisée telle quelle et dont la caractéristique est la suivante :
Spectre de masse (E/I) : m/z = 298 (M+)

*Etape 5:* On opère comme dans l'étape 3 de l'exemple 5 à partir de 205 mg de [4-(benzoxazol-2-yl)-9H-fluorèn-9-(R,S)-yl]-amine, obtenue à l'étape 4, et de 123 mg d'acide 1H-pyrrolo[2,3-b]pyidine-4-carboxylique , en présence de 145 mg d'EDCl et de 47 mg d'HOBT, dans 6 mL de DMF pendant 6 heures. Le milieu réactionnel est versé sur 50 mL d'eau et le précipité formé est essoré, lavé à l'eau, puis avec une solution saturée d'hydrogénocarbonate de sodium et de nouveau à l'eau. Le solide obtenu est purifié par chromatographie sur gel de silice (40-63 µm) en éluant avec un mélange de dichlorométhane et de méthanol (95/05 en volumes). Après évaporation à sec sous vide, on triture le solide obtenu dans de l'éther de pétrole, filtre et sèche sous vide à 40°C. On obtient ainsi 129 mg de [4-(benzoxazol-2-yl)-9H-fluorèn-9-(R,S)-yl]-amide de l'acide 1H-pyrrolo[2,3-b]pyridine-4-carboxylique, sous forme d'un solide beige dont les caractéristiques sont les suivantes :
Point de fusion (Kofler) >260°C
Spectre de RMN 1 H (400 MHz - DMSO-d6) δ en ppm : 6,42 (d, J = 8,5 Hz, 1 H) ; 6,91 (d large, J = 3,5 Hz, 1 H) ; de 7,35 à 7,45 (m, 2H) ; 7,47 (d, J = 5,0 Hz, 1 H) ; de 7,48 à 7,56 (m, 2H) ; 7,59 (t, J = 7,5 Hz, 1H) ; 7,63 (t, J = 3,5 Hz, 1H) ; 7,66 (d, J = 7,5 Hz, 1H) ; 7,86 (d, J = 7,5 Hz, 1H) ; 7,88 (d, J = 7,5 Hz, 1H) ; 7,97 (d, J = 7,5 Hz, 1H) ; 8,01 (d, J = 7,5 Hz, 1H) ; de 8,25 à 8,35 (m, 2H) ; 9,29 (d, J = 8,5 Hz, 1 H) ; 11,85 (m large, 1 H)

### Exemple 7: [4-(pyrimidin-5-yl)-9H-fluorèn-9-(R,S)yl]-amide de l'acide 1 H-pyrrolo[2,3-b]pyridine-4-carboxylique

*Etape 1*: Dans un tube pour micro-ondes, on dissout 0,5 g de 4-bromo-fluorèn-9-one, qui peut être obtenu selon J. Amer. Chem. Soc, 57, 2443-6, 1935, dans 12 mL d'éthanol, puis on ajoute successivement 0,27 g de chlorure de bis (triphénylphosphine) palladium (II), 0,25 g d'acide pyrimidino-5-boronique et 0,54 mL de triéthylamine. Après 18 minutes de réaction à 140°C, on concentre à sec, reprend au dichlorométhane et à l'eau, sèche sur sulfate de magnésium, filtre et concentre à sec. Le solide brut obtenu est purifié par flash-chromatographie sur gel de silice (20-40 µm) en éludant avec un mélange de cyclohexane et d'acétate d'éthyle (80-20 en volumes). On obtient ainsi 0,3 g de 4-(pyrimidin-5-yl)-fluorèn-9-one sous forme d'un solide jaune, utilisé tel quel dans l'étape suivante dont la caractéristique est la suivante :
Point de fusion (Kofler) : 188°C

Etape 2 : On opère comme à l'étape 2 de l'exemple 5, à partir de 300 mg de 4-(pyrimidin-5-yl)-fluorèn-9-one, obtenue à l'étape précédente, de 242 mg de chlorhydrate d'hydroxylamine et de 477 mg d'acétate de sodium agités à température ambiante pendant 24 heures dans 10 mL d'éthanol. Après concentration du solvant sous pression réduite, le résidu est repris par de l'eau et le précipité formé est essoré puis rincé au pentane. On obtient ainsi, 310 mg de 4-(pyrimidin-5-yl)-fluorèn-9-one oxime, en mélange 50-50 des isomères Z et E, sous forme d'un solide jaune, dont les caractéristiques sont les suivantes :
Point de fusion (Kofler) : 222°C
Spectre de masse (E/I) : m/z = 273 (M+).

Etape 3 : Dans un autoclave de 50 mL, on dissout 0,31 g de mélange équimoléculaire des isomères Z et E de 4(-pyrimidin-5-yl)-fluorèn-9-one, obtenue à l'étape 2, dans un mélange de 15 mL d'éthanol et 15 mL de tétrahydrofurane, on ajoute une pointe de spatule de Nickel activé selon Raney puis on soumet à une pression initiale d'hydrogène de 1 bar et on chauffe l'autoclave à 60°C pendant 2 heures. Après refroidissement, on filtre le catalyseur sur Célite. Le filtrat est concentré sous pression réduite. On obtient ainsi, 0,3 g de 4-(pyrimidin-5-yl)-9H-fluorèn-9-(R,S)-yl-amine sous forme d'une laque brune, utilisée telle quelle et dont la caractéristique est la suivante :
Spectre de masse (E/I) : m/z = 259 (M+)

*Etape 4 :* On opère comme dans l'étape 4 de l'exemple 5 à partir de 290 mg de 4-(pyrimidin-5-yl)-9H-fluorèn-9-(R,S)-yl-amine, obtenue à l'étape 4, et de 200 mg d'acide 1H-pyrrolo[2,3-b]pyridine-4-carboxylique , en présence de 236 mg d'EDCI et de 77 mg d'HOBT, dans 15 mL de DMF pendant 48 heures. Le milieu réactionnel est versé sur 50 mL d'eau et le précipité formé est essoré, lavé à l'eau, puis avec une solution saturée d'hydrogénocarbonate de sodium et de nouveau à l'eau. Le solide obtenu est purifié par chromatographie sur gel de silice (40-63 µm) en éluant avec un mélange de dichlorométhane et de méthanol (95/05 en volumes). On obtient ainsi 195 mg de [4-(pyrimidin-5-yl)-9H-fluorèn-9-(R,S)-yl]-amide de l'acide 1H-pyrrolo[2,3-b]pyridlne-4-carboxylique, sous forme d'un solide blanc dont les caractéristiques sont les suivantes :
Point de fusion (Kofler) >260°C
Spectre de masse (E/I) : m/z = 403 (M+)
Spectre de RMN 1 H (400 MHz - DMSO-d6) δ en ppm : 6,40 (d, J = 8,5 Hz, 1 H) ; 6,78 (d, J = 7,5 Hz, 1H) ; 6,91 (d large, J = 3,5 Hz, 1 H) ; 7,21 (t, J = 7,5 Hz, 1H) ; 7,32 (t, J = 7,5 Hz, 1 H) ; 7,38 (d, J = 7,5 Hz, 1 H) ; 7,47 (d, J = 5,0 Hz, 1H) ; 7,50 (t, J = 7,5 Hz, 1H) ; 7,62 (m, 2H) ; 7,72 (d, J = 7,5 Hz, 1 H) ; 8,29 (d, J = 5,0 Hz, 1H) ; 8,98 (s, 2H) ; 9,23 (d, J = 8,5 Hz, 1H) ; 9,39 (s, 1 H) ; 11,85 (m large, 1H).

### Exemple 8: [4-(quinoléin-2-yl)-9H-fluorèn-9-(R,S)-yl]-amide de l'acide 1H-pyrrolo[2,3-b]pyridine-4-carboxylique

*Etape 1 :* Dans un tricol de 250 mL, on introduit successivement 2 g de 4-bromo-fluorèn-9-one, qui peut être obtenue selon J. Amer. Chem. Soc. 57, 2443-6 (1935), 175 mg d'acétate de palladium (II), 3,8 g d'acétate de potassium, 4,8 g de 4,4,5,5,4',4',5',5'-octaméthyl-2,2'-bi[1,3,2-dioxaborolanyl] et 150 mL de diméthylformamide. Un courant d'argon est passé dans la solution obtenue pendant 1 heure puis on chauffe vers 70°C pendant 1 heure. Après refroidissement, le mélange est filtré sur célite, on ajoute 200 mL d'eau au filtrat, extrait par trois fois 50 mL d'acétate d'éthyle, sèche sur sulfate de magnésium et concentre sous pression réduite. Le solide obtenu est purifié par chromatographie sur gel de silice (40-63 µm) en éluant avec un mélange de cyclohexane et d'acétate d'éthyle (95/05 en volumes). On obtient ainsi, 2 g de 4-(4,4,5,5-tetraméthyl-1,3,2-dioxaborolan-2-yl)-fluorèn-9-one, sous forme de solide jaune et qui est utilisé tel quel à l'étape suivante et dont les caractéristiques sont les suivantes :
Spectre de masse (E/I) : m/z = 306 (M+)
Spectre de 1 H (400 MHz - DMSO-d6) δ en ppm :1,40 (s, 12H) ; 7,39 (t large, J = 7,5 Hz, 2H) ; de 7,59 à 7,66 (m, 2H) ; 7,71 (d large, J = 7,5 Hz, 1 H) ; 7,87 (dd, J = 1,0 et 7,5 Hz, 1 H) ; 8,47 (d large, J = 7,5 Hz, 1H).

*Etape 2*: Dans un tricol de 250 mL, sous atmosphère d'Argon, on introduit successivement 2 g de 4-(4,4,5,5-tetraméthyl-1,3,2-dioxaborolan-2-yl)-fluorèn-9-one, obtenue à l'étape précédente, 1 g de palladium (0) tétrakis (triphénylphosphine), 2,2 g de carbonate de césium, 0,96 g de 2-bromoquinoléine obtenue selon Tetrahedron Letters, 40, (1999), 7477-78, et 60 mL de diméthylformamide anhydre. Après 5h30 de chauffage à 80°C, on refroidit puis on verse sur 200 mL d'eau, extrait par trois fois 50 mL d'acétate d'éthyle, sèche sur sulfate de magnésium, filtre et concentre sous pression réduite. Le solide brut obtenu est purifié par flash-chromatographie sur gel de silice (20-40 µm) en éluant avec un mélange de cyclohexane et d'acétate d'éthyle (90-10 en volumes). On obtient ainsi, 0,95 g de 4-(quinoléin-2-yl)-fluorèn-9-one sous forme d'un solide jaune dont les caractéristiques sont les suivantes :
Point de fusion (Kofler) : 155°C
Spectre de masse (E/I) : m/z = 307 (M+)

*Etape 3:* On opère comme à l'étape 2 de l'exemple 5 à partir de 940 mg de 4-quinoléin-2yl-fluorèn-9-one, obtenue à l'étape précédente, de 640 mg de chlorhydrate d'hydroxylamine et de 1,25 g d'acétate de sodium agités à température ambiante pendant 24 heures dans 26 mL d'éthanol. Après concentration du solvant sous pression réduite, le résidu est repris par de l'eau et le précipité formé est filtré puis rincé au pentane. On obtient ainsi, 910 mg de 4-(quinoléin-2-yl)-fluorèn-9-one oxime, en mélange 50-50 des isomères Z et E, sous forme d'un solide blanc, dont les caractéristiques sont les suivantes :
Point de fusion (Kofler) : 260°C
Spectre de masse (E/I) : m/z = 322 (M+).

*Etape 4* : Dans un autoclave de 100 mL, on dissout 0,9 g de mélange équimoléculaire des isomères Z et E de 4-(quinoléin-2-yl)-fluorèn-9-one oxime, obtenue à l'étape 3, dans un mélange de 40 mL d'éthanol et 40 mL de tétrahydrofurane, on ajoute une pointe de spatule de Nickel activé selon Raney puis on soumet à une pression initiale d'hydrogène de 1 bar et on chauffe l'autoclave à 60°C pendant 2 heures. Après refroidissement, on filtre le catalyseur sur Célite et le filtrat est concentré sous pression réduite. Après purification par flash-chromatographie sur gel de silice (20-40 µm) en éluant avec un mélange de dichlorométhane et de méthanol (95-05 en volumes). On obtient ainsi, 0,72 g de 4-(quinoléin-2-yl)-9H-fluorèn-9-(R,S)-yl-amine sous forme d'une meringue blanche dont la caractéristique est la suivante :
Spectre de masse (E/I) : m/z = 308 (M+)

*Etape 5*: On opère comme dans l'étape 4 de l'exemple 5 à partir de 200 mg de 4-(quinoléin-2-yl)-9H-fluorèn-9-(R,S)-yl-amine, obtenue à l'étape 4, et de 116 mg d'acide 1H-pyrrolo[2,3-b]pyridine-4-carboxylique , en présence de 137 mg d'EDCI et de 45 mg d'HOBT, dans 6 mL de DMF pendant 20 heures. Le milieu réactionnel est versé sur 60 mL d'eau et le précipité formé est essoré, lavé avec 6 fois 20 mL d'eau, puis avec une solution saturée d'hydrogénocarbonate de sodium, de nouveau à l'eau puis à l'éther isopropylique. On obtient ainsi, 185 mg de [4-(quinoléin-2-yl)-9H-fluorèn-9-(R,S)-yl]-amide de l'acide 1H-pyrrolo[2,3-b]pyridine-4-carboxylique, sous forme d'un solide blanc rosé dont les caractéristiques sont les suivantes :
Point de fusion (Kofler) = 260°C
Spectre de masse (E/I) : m/z = 452 (M+)
Spectre de RMN 1 H (400 MHz - DMSO-d6) δ en ppm : 6,43 (d, J = 8,5 Hz, 1H) ; 6,82 (d, J = 7,5 Hz, 1H) ; 6,92 (s, 1H) ; 7,08 (t, J = 7,5 Hz, 1 H) ; 7,28 (t, J = 7,5 Hz, 1 H) ; de 7,44 à 7,54 (m, 3H) ; de 7,57 à 7,65 (m, 2H) ; de 7,62 à 7,75 (m, 2H) ; 7,78 (t, J = 8,0 Hz, 1H) ; 7,86 (t, J = 8,0 Hz, 1H) ; 8,06 (d, J = 8,0 Hz, 1H) ; 8,13 (d, J = 8,0 Hz, 1H) ; 8,30 (d, J = 5,0 Hz, 1H) ; 8,58 (d, J = 8,0 Hz, 1H) ; 9,26 (d, J = 8,5 Hz, 1 H) ; 11,9 (s, 1H).

### Exemple 9: (4-[1,2,4]triazolo[1,5-a]pyridin-2-yl-9H-fluoren-9(R,S)-yl)-amide de l'acide 1H-pyrrolo[2,3-b]pyridine-4-carboxylique

*Etape 1* : Dans un réacteur micro-onde, on introduit successivement 165 mg de 4-(4,4,5,5-tetraméthyl-1,3,2-dioxaborolan-2-yl)-fluorèn-9-one, obtenue à l'étape 1 de l'exemple 8, 109 mg de palladium (0) tétrakis (triphénylphosphine), 230 mg de carbonate de césium, 93 mg de 2-bromo-[1,2,4]triazolo[1,5-a]pyridine dans 2 mL de diméthylformamide anhydre. Après 18 minutes de chauffage à 140°C, on verse sur 50 mL d'eau, et on filtre le précipité formé qui est ensuite dissout dans 20 mL d'un mélange de dichlorométhane et de méthanol, séché sur sulfate de magnésium et concentré sous pression réduite. Le solide brut obtenu est purifié par flash-chromatographie sur 25 g de gel de silice (20-40 µm), en éluant avec un mélange de cyclohexane et d'acétate d'éthyle (70-30 en volumes). On obtient ainsi 70 mg de 4-{[1,2,4]triazolo[1,5-a]pyridin-2-yl}-fluorèn-9-one, sous forme d'un solide jaune dont la caractéristique est la suivante :
Spectre de masse (E/I) : m/z = 297 (M+)

La 2-bromo-[1,2,4]triazolo[1,5-a]pyridine peut être obtenue par une réaction de diazo-bromation de Sandmeyer-Gattermann à partir de la [1,2,4]triazolo[1,5-a]pyridine-2-amine, elle même préparée selon Monatsch Chem. 1983, *114*, 789.

*Etape 2:* On opère comme à l'étape 2 de l'exemple 5, à partir de 65 mg de 4-{[1,2,4]triazolo[1,5-a]pyridin-2-yl}-fluorèn-9-one, obtenue à l'étape précédente, de 45,6 mg de chlorhydrate d'hydroxylamine et de 89,7 mg d'acétate de sodium agités à température ambiante pendant 2 heures, puis au reflux pendant 9 heures et 30 minutes dans 26 mL d'éthanol. Après concentration du solvant sous pression réduite, le résidu est repris par de l'eau et le précipité formé est filtré puis séché sous vide à 50°. On obtient ainsi 67 mg de 4-{[1,2,4]triazolo[1,5-a]pyridin-2-yl}-fluorèn-9-one oxime, en mélange 50-50 des isomères Z et E, sous forme d'une poudre blanche utilisée telle quelle à l'étape suivante, dont la caractéristique est la suivante :
Spectre de masse (E/I) : m/z = 312 (M+).

*Etape 3*: Dans un tricol de 25 mL, on dissout 67 mg de mélange équimoléculaire des isomères Z et E de 4-{[1,2,4]triazolo[1,5-a]pyridin-2-yl}-fluorèn-9-one oxime, obtenue à l'étape 2, dans un mélange de 2 mL d'éthanol, 1 mL d'acide acétique et 1 mL d'eau, puis on ajoute 56 mg de zinc et on agite à température ambiante pendant 1 heure. Après filtration de l'excès de zinc sur Célite, rinçage à l'éthanol et concentration à sec sous pression réduite, le résidu est repris avec 50 mL d'eau. Le pH est amené à 12 par addition d'une solution aqueuse 1 N d'hydroxyde de sodium et le précipité formé est essoré, puis repris par 50 mL d'un mélange de dichlorométhane et de méthanol, agité pendant 15 minutes et enfin filtré. On obtient ainsi 54 mg de 4-{[1,2,4]triazolo[1,5-a]pyridin-2-yl}-9H-fluorèn-9-(R,S)-yl-amine, sous forme d'une poudre blanc-cassé utilisée telle quelle à l'étape suivante, dont la caractéristique est la suivante :
Spectre de masse (E/I) : m/z = 298 (M+)

*Etape 4 :* On opère comme dans l'étape 4 de l'exemple 5 à partir de 48 mg de 4-{[1,2,4]triazolo[1,5-a]pyridin-2-yl}-9H-fluorèn-9-(R,S)-yl-amine, obtenue à l'étape 3, et de 28,7 mg d'acide 1H-pyrrolo[2,3-b]pyridine-4-carboxylique, en présence de 33,9 mg d'EDCI et de 12,3 mg d'HOBT, dans 1 mL de DMF pendant 2 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après séchage sous vide à 50°, on obtient ainsi 65 mg de 4-{[1,2,4]triazolo[1,5-a]pyridin-2-yl}-9H-fluorèn-9-(R,S)-yl-amide de l'acide 1 H-pyrrolo[2,3-b]pyridine-4-carboxylique, sous forme d'un solide beige clair dont la caractéristique est la suivante :
Spectre de masse (E/I) : m/z = 452 (M+)

### Exemple 10 : [4-(1,4-benzoxazin-2H-3-yl)-9H-fluorèn-9(R,S)-yl]-amide de l'acide 1H-pyrrolo[2,3-b]pyridine-4-carboxylique

*Etape 1* : Dans un réacteur micro-onde, on introduit successivement 410 mg de 4-(4,4,5,5-tetraméthyl-1,3,2-dioxaborolan-2-yl)-fluorèn-9-one, obtenue à l'étape 1 de l'exemple 8, 535 mg de palladium (0) tétrakis (triphénylphosphine), 535 mg de carbonate de césium et 555 mg de N-*tert-*butyloxycarbonyl-3-iodo-1,4-benzoxazine dans 10 mL de diméthylformamide anhydre. Après 12 minutes de chauffage à 140°C, on verse sur 60 mL d'eau, et on extrait 2 fois avec 30 mL d'acétate d'éthyle. Après séchage sur sulfate de magnésium et concentration sous pression réduite, le solide brut obtenu est purifié par flash-chromatographie sur 25 g de gel de silice (40-60 µm), en éluant avec du cyclohexane puis avec un mélange de cyclohexane et d'acétate d'éthyle (90-10 en volumes). On obtient ainsi 421 mg de 4-(N-*tert-*butyloxycarbonyl-1,4-benzoxazin-3-yl)-fluorèn-9-one, sous forme d'un solide beige dont la caractéristique est la suivante :
Spectre de masse (E/I) : m/z = 411 (M+)

La N-*tert*-butyloxycarbonyl-3-iodo-1,4-benzoxazine peut être obtenue en opérant selon Tetrahedron Lett. (1998), 39(32), 5763-4.

*Etape 2:* On opère comme à l'étape 2 de l'exemple 5, à partir de 374 mg de 4-( N-*tert*-butyloxycarbonyl-1,4-benzoxazin-3-yl)-fluorèn-9-one, obtenue à l'étape précédente, de 189,5 mg de chlorhydrate d'hydroxylamine et de 373 mg d'acétate de sodium agités à température ambiante pendant 3 jours dans 15 mL d'éthanol. Après concentration du solvant sous pression réduite, le résidu est repris par de l'eau ; le précipité formé est essoré, lavé avec de l'éther de pétrole puis séché à l'air. On obtient ainsi 340 mg de 4-(N-*tert*-butyloxycarbonyl-1,4-benzoxazin-3-yl)-fluorèn-9-one oxime, en mélange 50-50 des isomères Z et E, sous forme d'une poudre jaune utilisée telle quelle à l'étape suivante, dont les caractéristiques sont les suivantes :
Spectre de masse (E/I) : m/z = 323 (M+).
Point de fusion (Kofler) = 223°C.

*Etape 3:* Dans un tricol de 25 mL, on dissout 190 mg de mélange équimoléculaire des isomères Z et E de 4-(N-*tert*-butyloxycarbonyl-1,4-benzoxazin-3-yl)-fluorèn-9-one oxime, obtenue à l'étape 2, dans un mélange de 4 mL d'éthanol, 2 mL d'acide acétique et 2 mL d'eau, puis on ajoute 116,5 mg de zinc et on agite à température ambiante pendant 2 heures. Après filtration de l'excès de zinc sur Célite, on rince au dichlorométhane puis à l'éthanol, on concentre à sec sous pression réduite. Le solide brut obtenu est purifié par flash-chromatographie sur 25 g de gel de silice (40-60 µm), en éluant avec du dichlorométhane puis avec un mélange de dichlorométhane et de méthanol ammoniacal 0,7N (98-2 en volumes). On obtient ainsi 134 mg de 4-(N-tert-butyloxycarbonyl-1,4-benzoxazin-3-yl)-9H-fluorèn-9-(R,S)-yl-amine, sous forme d'une huile visqueuse jaune utilisée telle quelle à l'étape suivante, dont la caractéristique est la suivante :
Spectre de masse (E/l) : m/z = 412 (M+)

*Etape 4 :* On opère comme dans l'étape 4 de l'exemple 5 à partir de 210 mg de 4-(N-*tert*-butyloxycarbonyl-1,4-benzoxazin-3-yl)-9H-fluorèn-9-(R,S)-yl-amine, obtenue à l'étape 3, et de 90,89 mg d'acide 1 H-pyrrolo[2,3-b]pyridine-4-carboxylique, en présence de 107,3 4 mg d'EDCI et de 34,96 mg d'HOBT, dans 4 mL de DMF pendant 20 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 25 g de gel de silice (40-60 µm), en éluant avec du dichlorométhane puis avec un mélange de dichlorométhane et de méthanol (98-2 en volumes, on obtient 195 mg de [4-(N-tert-butyloxycarbonyl-1,4-benzoxazin-3-yl)-9H-fluorèn-9-(R,S)-yl]-amide de l'acide 1H-pyrrolo[2,3-b]pyridine-4-carboxylique, sous forme d'un solide jaune, dont la caractéristique est la suivante :
Spectre de masse (E/I) : m/z = 556 (M+)

*Etape 5:* Dans un ballon de 25 mL, on dissout 180 mg de 4-[(N-*tert-*butyloxycarbonyl-1,4-benzoxazin-3-yl)-9H-fluorèn-9-(R,S)-yl]-amide de l'acide 1H-pyrrolo[2,3-b]pyridine-4-carboxylique, obtenu à l'étape précédente, dans 10 mL de dichlorométhane, puis on introduit lentement 1 mL de solution 4N d'acide chlorhydrique dans le dioxane et on agite une nuit à température ambiante. Après concentration à sec, le résidu est repris dans 20 mL d'une solution 7N de méthanol ammoniacal et de nouveau concentré à sec. Après purification par flash-chromatographie sur 25 g de gel de silice (20-40 µm), en éluant avec du dichlorométhane puis avec un mélange de dichlorométhane et de méthanol (98-2 en volumes, on obtient 80 mg de [4-(1,4-benzoxazin-2H-3-yl)-9H-fluorèn-9-(R,S)-yl]-amide de l'acide 1H-pyrrolo[2,3-b]pyridine-4-carboxylique, sous forme d'un solide jaune dont les caractéristiques sont les suivantes :
Spectre de masse (E/I) : m/z = 456 (M+)
Spectre de RMN 1 H (400 MHz - DMSO d6) δ en ppm : 5,00 (d, J = 16,0 Hz, 1H) ; 5,14 (d, J = 16,0 Hz, 1H) ; 6,39 (d, J = 8,0 Hz, 1H) ; 6,90 (d, J = 3,5 Hz, 1 H) ; 7,05 (d, J = 8,0 Hz, 1 H) ; 7,09 (t, J = 8,0 Hz, 1 H) ; 7,28 (t, J = 8,0 Hz, 1 H) ; de 7,33 à 7,42 (m, 3H) ; 7,44 (d, J = 5,0 Hz, 1H) ; 7,48 (t, J = 8,0 Hz, 1H) ; 7,54 (d, J = 8,0 Hz, 1 H) ; 7,62 (m, 2H) ; 7,71 (d, J = 8,0 Hz, 1 H) ; 7,87 (m, 1 H) ; 8,29 (d, J = 5,0 Hz, 1H) ; 9,21 (d, J = 8,0 Hz, 1 H) ; 11,85 (s large, 1H) .

### Exemple 11 : [4-(quinoléin-2-yl)-9H-fluorèn-9(R,S)-yl]-amide de l'acide 7H-pyrrolo[2,3-c]pyrimidine-4-carboxylique

Dans un tricol de 50 mL sous atmosphère d'argon, on agite à température ambiante pendant 10 minutes 308,4 mg de 4-(quinoléin-3-yl)-9H-fluorèn-9-(R,S)-yl-amine, obtenue comme à l'étape 3 de l'exemple 3, et 239 mg d'ester éthylique de l'acide 7H-pyrrolo[2,3-c]pyrimidine-4-carboxylique dans 32 mL de tetrahydrofurane. A la suspension brune ainsi obtenue, on ajoute 1 mL d'une solution 2M de triméthylaluluminium dans le tetrahydrofurane et on agite 1 heure à température ambiante. Cette opération - addition de 1 mL d'une solution 2M de triméthylaluluminium dans le tetrahydrofurane puis agitation 1 heure à température ambiante - est répétée deux fois supplémentaires. On ajoute alors 100 mL d'une solution aqueuse 0,1 N d'acide chlorhydrique et 50 mL d'acétate d'éthyle. La phase organique est décantée, puis la phase aqueuse est réextraite 2 fois avec 50 mL d'acétate d'éthyle. Les phases organiques jointes sont lavées avec une solution aqueuse saturée d'hydrogénocarbonate de sodium, séchées sur sulfate de magnésium et concentrées à sec sous pression réduite. Après purification par flash-chromatographie sur 50 g de gel de silice (20-40 µm), en éluant avec un mélange de dichlorométhane et de méthanol (97,5-2,5 en volumes), on obtient 90 mg de [4-(quinoléin-3-yl)-9H-fluorèn-9(R,S)-yl]-amide de l'acide 7H-pyrrolo[2,3-c]pyrimidine-4-carboxylique, sous forme d'un solide beige clair dont les caractéristiques sont les suivantes :
Spectre de masse (E/I) : m/z = 453 (M+)
Spectre de RMN 1 H (400 MHz - DMSO-d6) δ en ppm : 6,31 (d, J = 8,5 Hz, 1 H) ; 6,73 (d, J = 8,0 Hz, 1 H) ; 7,11 (t, J = 8,0 Hz, 1 H) ; 7,13 (d, J = 3,5 Hz, 1 H) ; 7,26 (t, J = 8,0 Hz, 1 H) ; 7,41 (d, J = 8,0 Hz, 1 H) ; 7,48 (d, J = 8,0 Hz, 1 H) ; 7,57 (d, J = 8,0 Hz, 1 H) ; 7,67 (d, J = 8,0 Hz, 1H) ; 7,71 (t, J = 8,0 Hz, 1H) ; 7,79 (d, J = 3,5 Hz, 1H) ; 7,88 (t large, J = 8,0 Hz, 1H) ; 8,11 (d, J = 8,0 Hz, 1H) ; 8,19 (d, J = 8,0 Hz, 1H) ; 8,53 (s large, 1 H) ; 8,82 (s, 1 H) ; 9,05 (d, J = 2,0 Hz, 1H) ; 9,41 (d, J = 8,5 Hz, 1 H) ; 12,4 (s large, 1 H).

### Exemple 12 : [4-(quinoxalin-2-yl)-9H-fluorèn-9(R,S)-yl]-amide de l'acide 1H-pyrrolo[2,3-b]pyridine-4-carboxylique

*Etape 1:* Dans un réacteur micro-onde, on introduit successivement 284 mg de 4-(4,4,5,5-tetraméthyl-1,3,2-dioxaborolan-2-yl)-fluorèn-9-one, obtenue à l'étape 1 de l'exemple 8, 280 mg de palladium (0) tétrakis (triphénylphosphine), 443,5 mg de carbonate de césium et 150 mg de 2-chloroquinoxaline dans 5 mL de diméthylformamide anhydre. Après 30 minutes de chauffage à 140°C, on verse sur 60 mL d'eau, et on extrait 2 fois avec 30 mL d'acétate d'éthyle. Après séchage sur sulfate de magnésium et concentration sous pression réduite, le solide brut obtenu est purifié par flash-chromatographie sur 25 g de gel de silice (40-60 µm), en éluant avec du cyclohexane puis avec un mélange de cyclohexane et d'acétate d'éthyle (80-20 en volumes). On obtient ainsi 175 mg de 4-(quinoxalin-2-yl)-fluorèn-9-one, sous forme d'un solide jaune dont la caractéristique est la suivante :
Spectre de masse (E/I) : m/z = 308 (M+)

*Etape 2 :* On opère comme à l'étape 3 de l'exemple 2, à partir de 170 mg de 4-(quinoxalin-2-yl)-fluorèn-9-one, obtenue à l'étape précédente, de 115 mg de chlorhydrate d'hydroxylamine et de 226 mg d'acétate de sodium, agités à température ambiante pendant 20 heures, puis au reflux pendant 3 heures dans 8 mL d'éthanol. Après concentration du solvant sous pression réduite, le résidu est repris par de l'eau ; le précipité formé est filtré, lavé avec de l'éther de pétrole puis séché à l'air. On obtient ainsi 175 mg de 4-(quinoxalin-2-yl)fluorèn-9-one oxime, en mélange 50-50 des isomères Z et E, sous forme d'une poudre grise, utilisée telle quelle à l'étape suivante, dont la caractéristique est la suivante :
Spectre de masse (E/I) : m/z = 323 (M+).

*Etape 3:* Dans un tricol de 25 mL, on dissout 175 mg de mélange équimoléculaire des isomères Z et E de 4-(quinoxalin-2-yl)fluorèn-9-one oxime, obtenue à l'étape 2, dans un mélange de 2,5 mL d'éthanol, 2,5 mL d'acide acétique et 5 mL d'eau, puis on ajoute 141,5 mg de zinc et on agite à température ambiante pendant 5 heures. Après filtration de l'excès de zinc sur Célite, rinçage à l'éthanol, on ajoute 20 mL d'une solution 7N d'ammoniac dans le méthanol et on concentre à sec sous pression réduite. Le solide brut obtenu est purifié par flash-chromatographie sur 25 g de gel de silice (40-60 µm), en éluant avec du dichlorométhane puis avec un mélange de dichlorométhane et de méthanol (98-2 en volumes). On obtient ainsi 30 mg de 4-(quinoxalin-2-yl)-9H-fluorèn-9-(R,S)-yl-amine, sous forme d'une meringue jaune utilisée telle quelle à l'étape suivante, dont la caractéristique est la suivante :
Spectre de masse (E/I) : m/z = 309 (M+)

*Etape 4* : On opère comme dans l'étape 4 de l'exemple 5 à partir de 30 mg de 4-(quinoxalin-2-yl)-9H-fluorèn-9-(R,S)-yl-amine, obtenue à l'étape 3, et de 17,3 mg d'acide 1H-pyrrolo[2,3-b]pyridine-4-carboxylique, en présence de 20,4 mg d'EDCI et de 6,6 mg d'HOBT, dans 1 mL de DMF pendant 7 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 25 g de gel de silice (40-60 µm), en éluant avec du dichlorométhane puis avec un mélange de dichlorométhane et de méthanol (98-2 en volumes, on obtient 15 mg de [4-(quinoxalin-2-yl)-9H-fluorèn-9(R,S)-yl]-amide de l'acide 1H-pyrrolo[2,3-b]pyridine-4-carboxylique, sous forme d'un solide blanc dont les caractéristiques sont les suivantes :
Spectre de masse (E/I) : m/z = 453 (M+)
Spectre de RMN 1 H (400 MHz - DMSO-d6) δ en ppm : 6,43 (d, J = 8,5 Hz, 1 H) ; 6,85 (d, J = 7,5 Hz, 1 H) ; 6,92 (dd, J = 2,0 et 3,5 Hz, 1 H) ; 7,13 (t, J = 7,5 Hz, 1 H) ; 7,31 (t, J = 7,5 Hz, 1H) ; 7,48 (d, J = 5,0 Hz, 1H) ; 7,55 (t, J = 7,5 Hz, 1 H) ; de 7,60 à 7,68 (m, 3H) ; 7,79 (d, J = 7,5 Hz, 1 H) ; 7,98 (m, 2H) ; 8,17 (m, 1H) ; 8,26 (m, 1 H) ; 8,30 (d, J = 5,0 Hz, 1 H) ; 9,22 (s, 1 H) ; 9,28 (d, J = 8,5 Hz, 1H) ; 11,85 (s large, 1H).

### Exemple 13 : [4-(quinoléin-3-yl)-9H-fluorèn-9(R,S)-yl]-amide de l'acide 6-bromo-3H-imidazo[4,5-b]pyridine-7-carboxylique

On opère comme dans l'étape 4 de l'exemple 5 à partir de 400 mg de 4-(quinoléin-3-yl)-9H-fluorèn-9(R,S)-yl-amine, obtenue comme à l'étape 4 de l'exemple 8, et de 282,4 mg d'acide 6-bromo-3H-imidazo[4,5-b]pyridine-7-carboxylique, en présence de 246 mg d'EDCI et de 173,5 mg d'HOBT, dans 12 mL de DMF pendant 20 heures. Le milieu réactionnel est versé sur 60 mL d'eau et le précipité formé est essoré, lavé avec 6 fois 20 mL d'eau, puis avec une solution saturée d'hydrogénocarbonate de sodium, de nouveau à l'eau puis à l'éther isopropylique. Après purification par flash-chromatographie sur 50 g de gel de silice (20-40 µm), en éluant avec un mélange de dichlorométhane et de méthanol (97,5-2,5 en volumes), on obtient 245 mg de [4-(quinoléin-3-yl)-9H-fluorèn-9(R,S)-yl]-amide de l'acide 6-bromo-3H-imidazo[4,5-b]pyridine-7-carboxyfique, sous forme d'un solide beige dont les caractéristiques sont les suivantes :
Spectre de masse (E/I) : m/z = 532 (M+)
Spectre de RMN 1 H (500 MHz - DMSO-d6) δ en ppm : 6,43 (d, J = 8,5 Hz, 1 H) ; 6,71 (d, J = 7,5 Hz, 1 H) ; 7,13 (t, J = 7,5 Hz, 1 H) ; 7,33 (t, J = 7,5 Hz, 1H) ; 7,43 (d, J = 7,5 Hz, 1 H) ; 7,55 (t, J = 7,5 Hz, 1H) ; 7,71 (t, J = 7,5 Hz, 1 H) ; 7,80 (m large, 1H) ; 7,88 (t, J = 7,5 Hz, 1H) ; 7,91 (m large, 1 H) ; 8,10 (d, J = 7,5 Hz, 1H) ; 8,17 (d, J = 7,5 Hz, 1H) ; 8,49 (m large, 1 H) ; 8,53 (s, 1 H) ; 8,60 (s, 1 H) ; 9,00 (m étalé, 1H) ; 9,47 (d large, J = 8,5 Hz, 1 H) ; 13,4 (m étalé, 1H).

### Exemple 14 : [4-(2-morpholino-pyridin-5-yl)-9H-fluorèn-9(R,S)-yl]-amide de l'acide 1H-pyrrolo[2,3-b]pyridine-4-carboxylique

*Etape 1* : Dans un tricol de 50 mL, on introduit successivement 864 mg de 4-bromo-fluorèn-9-one, qui peut être obtenue selon J. Amer. Chem. Soc. 57, 2443-6 (1935), 1 g de 5-(4,4,5,5-tetraméthyl-1,3,2-dioxaborolan-2-yl)-2-morpholino-pyridine, 773 mg de palladium (0) tétrakis (triphénylphosphine) et 1,635 g de carbonate de césium dans 21 mL de diméthylformamide anhydre. Après 20 heures de chauffage à 120°C, on verse sur 25 mL d'eau, et on extrait 2 fois avec 30 mL d'acétate d'éthyle. Après séchage sur sulfate de magnésium et concentration sous pression réduite, le solide brut obtenu est purifié par flash-chromatographie sur 100 g de gel de silice (40-60 µm), en éluant avec du cyclohexane puis avec un mélange de cyclohexane et d'acétate d'éthyle (80-20 en volumes). On obtient ainsi 1 g de 4-(2-morpholino-pyridin-5-yl)-fluorèn-9-one, sous forme d'un solide jaune dont la caractéristique est la suivante :
Spectre de masse (E/I) : m/z = 342 (M+)

*Etape 2 :* On opère comme à l'étape 3 de l'exemple 2, à partir de 1 g 4-(2-morpholino-pyridin-5-yl)-fluorèn-9-one, obtenue à l'étape précédente, de 609 mg de chlorhydrate d'hydroxylamine et de 1,198 g d'acétate de sodium, agités à température ambiante pendant 20 heures dans 27 mL d'éthanol. Après concentration du solvant sous pression réduite, le résidu est repris par de l'eau ; le précipité formé est filtré, lavé avec du pentane puis séché à l'air. On obtient ainsi 1 g de 4-(2-morpholino-pyridin-5-yl)-fluorèn-9-one oxime, en mélange 50-50 des isomères Z et E, sous forme d'une poudre jaune, utilisée telle quelle à l'étape suivante, dont la caractéristique est la suivante :
Spectre de masse (E/I) : m/z = 357 (M+).

*Etape 3:* Dans un autoclave de 100 mL, on dissout 1 g de mélange équimoléculaire des isomères Z et E de 4-(2-morpholino-pyridin-5-yl)-fluorèn-9-one oxime, obtenue à l'étape 2, dans un mélange de 25 mL d'éthanol et 25 mL de tetrahydrofyrane, puis on ajoute 175 mg de Nickel de Raney et on agite sous une pression de 1 bar d'hydrogène pendant 10 heures à 60°C. Après filtration et lavage à l'éthanol du catalyseur, on concentre à sec sous pression réduite. Le solide brut obtenu est purifié par empâtage dans l'oxyde de diisopropyle. On obtient ainsi 960 mg de 4-(2-morpholino-pyridin-5-yl)fluorèn-9(R,S)-yl-amine, sous forme d'un solide vert clair utilisé tel quel à l'étape suivante, dont la caractéristique est la suivante :
Spectre de masse (E/I) : m/z = 343 (M+)

*Etape 4 :* On opère comme dans l'étape 4 de l'exemple 5 à partir de 343 mg de 4-(2-morpholino-pyridin-5-yl)fluorèn-9(R,S)-yl-amine, obtenue à l'étape 3, et de 162 mg d'acide 1H-pyrrolo[2,3-b]pyridine-4-carboxylique, en présence de 211 mg d'EDCI et de 148,6 mg d'HOBT, dans 10 mL de DMF pendant 20 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 50 g de gel de silice (40-60 µm), en éluant avec un mélange de dichlorométhane et de méthanol (97,5-2,5 en volumes), on obtient 240 mg de [4-(2-morpholino-pyridin-5-yl)-9H-fluorèn-9(R,S)-yl]-amide de l'acide 1H-pyrrolo[2,3-b]pyridine-4-carboxylique, sous forme d'un solide beige dont les caractéristiques sont les suivantes :
Spectre de masse (E/I) : m/z = 487 (M+)
Spectre de RMN 1 H (500 MHz - DMSO-d6) δ en ppm : 3,55 (m, 4H) ; 3,87 (m, 4H) ; 6,39 (d, J = 8,5 Hz, 1 H) ; 6,90 (dd, J = 2,0 et 3,0 Hz, 1 H) ; 7,02 (d, J = 7,5 Hz, 1H) ; 7,08 (d, J = 7,5 Hz, 1 H) ; 7,23 (m, 2H) ; 7,30 (t, J = 7,5 Hz, 1H) ; 7,40 (t, J = 7,5 Hz, 1H) ; 7,46 (d, J = 5,0 Hz, 1 H) ; 7,60 (m, 2H) ; 7,62 (t, J = 3,0 Hz, 1H) ; 7,70 (m étalé, 1 H) ; 8,22 (s large, 1 H) ; 8,29 (d, J = 5,0 Hz, 1H) ; 9,22 (d, J = 8,5 Hz, 1H) ; 11,9 (s large, 1H) .

### Exemple 15: [4-(quinoléin-3-yl)-9H-fluorèn-9(R,S)-yl]-amide de l'acide 2-amino-5-chloro-pyrimidine-4-carboxylique

On opère comme dans l'étape 4 de l'exemple 5 à partir de 308 mg de 4-(quinoléin-3-yl)-9H-fluorèn-9(R,S)-yl-amine, obtenue comme à l'étape 4 de l'exemple 8, et de 173,6 mg d'acide 2-amino-5-chloro-pyrimidine-4-carboxylique, en présence de 211 mg d'EDCI et de 148,6 mg d'HOBT, dans 10 mL de DMF pendant 3 jours. Le milieu réactionnel est versé sur 60 mL d'eau et le précipité formé est essoré, lavé avec 6 fois 20 mL d'eau, puis avec une solution saturée d'hydrogénocarbonate de sodium, de nouveau à l'eau puis à l'éther isopropylique. Le produit brut est purifié successivement par flash-chromatographie sur 25 g de gel de silice (20-40 µm), en éluant avec un mélange de dichlorométhane et de méthanol (97,5-2,5 en volumes), puis CLHP sur une colonne Kromasil C18 10 µm (longueur 35 cm, diamètre 8 cm) en élauant avec un mélange d'eau, contenant 0,1% d'acide trifluoroacétique, et d'acétonitrile (65/35 en volumes). On obtient ainsi 106 mg de [4-(quinoléin-3-yl)-9H-fluorèn-9(R,S)-yl]-amide de l'acide 2-amino-5-chloro-pyrimidine-4-carboxylique, sous forme de cristaux blancs dont les caractéristiques sont les suivantes :
Spectre de masse (E/I) : m/z = 463 (M+)
Spectre de RMN 1 H (400 MHz - DMSO-d6) δ en ppm : 6,28 (d, J = 8,5 Hz, 1 H) ; 6,71 (d, J = 7,5 Hz, 1H) ; 7,09 (s, 2H) ; 7,11 (t, J = 7,5 Hz, 1H) ; 7,30 (t, J = 7,5 Hz, 1H) ; 7,42 (d, J = 7,5 Hz, 1H) ; 7,52 (t, J = 7,5 Hz, 1H) ; 7,58 (d, J = 7,5 Hz, 1H) ; 7,68 (d, J = 7,5 Hz, 1H) ; 7,72 (t, J = 7,5 Hz, 1H) ; 7,88 (t large, J = 7,5 Hz, 1H) ; 8,10 (d large, J = 7,5 Hz, 1H) ; 8,17 (d, J = 7,5 Hz, 1 H) ; 8,39 (s, 1 H) ; 8,51 (s large, 1H) ; 8,99 (s large, 1 H) ; 9,28 (d, J = 8,5 Hz, 1H).

### Exemple 16 : [4-(indazole-1-carbonyl)-9H-fiuorèn-9(R,S)-yl]-amide de l'acide 1H-pyrrolo[2,3-b]pyridine-4-carboxylique

*Etape 1 :* Dans un tricol de 50 mL sous argon, on dissout 243,4 mg d'indazole dans 10 mL de diméthylformamide anhydre, puis on ajoute, par fraction, 99 mg d'hydrure de sodium et on agite, pendant 30 minutes, jusqu'à dissolution complète. On ajoute alors une solution de 500 mg du chlorure de l'acide fluorèn-9-one-4-carboxylique dans 7 mL de diméthylformamide. Après 1 heure et 30 minutes d'agitation à température ambiante, on chauffe pendant 1 heure à 70°C, puis on maintient l'agitation pendant 1 nuit. On verse sur 100 mL d'eau, le précipité formé est essoré puis lavé avec de l'éther de pétrole et séché à l'air. On obtient ainsi 385 mg de 4-(indazole-1-carbonyl)-fluorèn-9-one, sous forme d'un solide jaune dont la caractéristique est la suivante :
Spectre de masse (E/I) : m/z = 324 (M+)

*Etape 2 :* On opère comme à l'étape 3 de l'exemple 2, à partir de 385 mg de 4-(indazole-1-carbonyl)-flurorèn-9-one, obtenue à l'étape précédente, de 318 mg de chlorhydrate d'hydroxylamine et de 318 mg d'acétate de sodium, agités à température ambiante pendant 3 heures, puis au reflux pendant 1 heures dans 20 mL d'éthanol. Après concentration du solvant sous pression réduite, le résidu est repris par de l'eau ; le précipité formé est filtré, lavé avec de l'éther de pétrole puis séché à l'air. On obtient ainsi 364 mg de 4-(indazole-1-carbonyl)-fluorèn-9-one oxime, en mélange 50-50 des isomères Z et E, sous forme d'une poudre jaune, utilisée telle quelle à l'étape suivante, dont la caractéristique est la suivante :
Spectre de masse (E/I) : m/z = 339 (M+).

*Etape 3:* Dans un tricol de 25 mL, on dissout 360 mg de mélange équimoléculaire des isomères Z et E de 4-(indazole-1-carbonyl)-fluorèn-9-one oxime, obtenue à l'étape 2, dans un mélange de 9,5 mL d'éthanol, 4,5 mL d'acide acétique et 4,5 mL d'eau, puis on ajoute 277,5 mg de zinc et on agite à température ambiante pendant 1 heure et 30 minutes. Après filtration de l'excès de zinc sur Célite, rinçage à l'éthanol, on ajoute 20 mL d'une solution 7N d'ammoniac dans le méthanol et on concentre à sec sous pression réduite. Le solide brut obtenu est purifié par flash-chromatographie sur 25 g de gel de silice (15-40 µm), en éluant avec un mélange de dichlorométhane et de méthanol (95-5 en volumes). On obtient ainsi 239 mg de 4-(indazole-1-carbonyl)-fluorèn-9(R,S)-yl-amine, sous forme d'un solide blanc utilisé tel quel à l'étape suivante, dont la caractéristique est la suivante :
Spectre de masse (E/I) : m/z = 325 (M+)

*Etape 4 :* On opère comme dans l'étape 4 de l'exemple 5 à partir de 235 mg de 4-(indazole-1-carbonyl)-fluorèn-9(R,S)-yl-amine, obtenue à l'étape 3, et de 128,8 mg d'acide 1H-pyrrolo[2,3-b]pyridine-4-carboxylique, en présence de 152,2 mg d'EDCI et de 49,8 mg d'HOBT, dans 5 mL de DMF pendant 1 nuit. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 25 g de gel de silice (20-40 µm), en éluant avec du dichlorométhane puis avec des mélanges de dichlorométhane et de méthanol (98-2 puis 95-5 en volumes), on obtient 122 mg de [4-(indazole-1-carbonyl)-9H-fluorèn-9(R,S)-yl]-amide de l'acide 1H-pyrrolo[2,3-b]pyridine-4-carboxylique, sous forme d'un solide blanc dont les caractéristiques sont les suivantes :
Point de fusion (Kofler) = 220°C
Spectre de masse (E/I) : m/z = 453 (M+)
Spectre de RMN 1 H (400 MHz - DMSO-d6) δ en ppm : 6,41 (d, J = 8,5 Hz, 1 H) ; 6,92 (dd, J = 2,0 et 3,0 Hz, 1 H) ; 7,09 (d, J = 7,5 Hz, 1 H) ; 7,20 (t, J = 7,5 Hz, 1 H) ; 7,31 (t, J = 7,5 Hz, 1 H) ; 7,48 (d, J = 5,0 Hz, 1H) ; 7,50 (t, J = 7,5 Hz, 1 H) ; 7,58 (t, J = 7,5 Hz, 1 H) ; de 7,60 à 7,69 (m, 3H) ; 7,80 (m, 2H) ; 7,99 (d, J = 7,5 Hz, 1 H) ; 8,29 (d, J = 5,0 Hz, 1H) ; 8,43 (s, 1 H) ; 8,60 (d, J = 7,5 Hz, 1 H) ; 9,30 (d, J = 8,5 Hz, 1 H) ; 11,85 (s large, 1 H) .

### Exemple 17 : [4-(1H-pyrrolo[2,3-b]pyridin-5-yl)-9H-fluorèn-9(R,S)-yl]-amide de l'acide 1 H-pyrrolo[2,3-b]pyridine-4-carboxylique

*Etape 1:* Dans un tricol de 50 mL, on introduit successivement 1,062 g de 4-bromo-fluorèn-9-one, qui peut être obtenue selon J. Amer. Chem. Soc. 57, 2443-6 (1935), 1 g de 5-(4,4,5,5-tetraméthyl-1,3,2-dioxaborolan-2-yl)-1H-pyrrolo[2,3-b]pyridine, 947 mg de palladium (0) tétrakis (triphénylphosphine) et 2,003 g de carbonate de césium dans 32 mL de diméthylformamide anhydre. Après 20 heures de chauffage à 120°C, on concentre le diméthylformamide sous pression réduite puis on verse sur 25 mL d'eau, et on extrait 2 fois avec 30 mL d'acétate d'éthyle. Après séchage sur sulfate de magnésium et concentration sous pression réduite, le solide brut obtenu est purifié par flash-chromatographie sur 100 g de gel de silice (40-60 µm), en éluant avec du cyclohexane puis avec un mélange de cyclohexane et d'acétate d'éthyle (70-30 en volumes). On obtient ainsi 455 mg de 4-(1H-pyrrolo[2,3-b]pyridin-5-yl)-fluorèn-9-one, sous forme d'un solide brun clair dont la caractéristique est la suivante :
Spectre de masse (E/I) : m/z = 296 (M+)

*Etape 2:* On opère comme à l'étape 3 de l'exemple 2, à partir de 450 g 4-(1H-pyrrolo[2,3-b]pyridin-5-yl)-fluorèn-9-one, obtenue à l'étape précédente, de 317 mg de chlorhydrate d'hydroxylamine et de 623 mg d'acétate de sodium, agités à température ambiante pendant 20 heures dans 14 mL d'éthanol. Après concentration du solvant sous pression réduite, le résidu est repris par de l'eau ; le précipité formé est filtré, lavé avec une solution saturée d'hydrogénocarbonate de sodium puis avec du pentane puis séché à l'air. On obtient ainsi 450 mg de 4-(1H-pyrrolo[2,3-b]pyridin-5-yl)-fluorèn-9-one oxime, en mélange 50-50 des isomères Z et E, sous forme d'une poudre jaune, utilisée telle quelle à l'étape suivante, dont la caractéristique est la suivante :
Spectre de masse (E/I) : m/z = 311 (M+).

*Etape 3:* Dans un autoclave de 100 mL, on dissout 450 mg de mélange équimoléculaire des isomères Z et E de 4-(1H-pyrrolo[2,3-b]pyridin-5-yl)-fluorèn-9-one oxime, obtenue à l'étape 2, dans un mélange de 12 mL d'éthanol et 12 mL de tetrahydrofyrane, puis on ajoute 90 mg de Nickel de Raney et on agite sous une pression de 1 bar d'hydrogène pendant 10 heures à 60°C. Après filtration et lavage à l'éthanol du catalyseur, on concentre à sec sous pression réduite. Le solide brut obtenu est purifié par empâtage dans l'oxyde de diisopropyle. On obtient ainsi 400 mg de 4-(1 H-pyrrolo[2,3-b]pyridin-5-yl)-fluorèn-9(R,S)-yl-amine, sous forme d'un solide verdâtre utilisé tel quel à l'étape suivante, dont la caractéristique est la suivante :
Spectre de masse (E/I) : m/z = 297 (M+)

*Etape 4 :* On opère comme dans l'étape 4 de l'exemple 5 à partir de 400 mg de 4-(1H-pyrrolo[2,3-b]pyridin-5-yl)-fluorèn-9(R,S)-yl-amine; obtenue à l'étape 3, et de 218 mg d'acide 1H-pyrrolo[2,3-b]pyridine-4-carboxylique, en présence de 284 mg d'EDCI et de 200 mg d'HOBT, dans 13,5 mL de DMF pendant 20 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 25 g de gel de silice (40-60 µm), en éluant avec un mélange de dichlorométhane et de méthanol (97,5-2,5 en volumes), on obtient 175 mg de [4-(1H-pyrrolo[2,3-b]pyridin-5-yl)-9H-fluorèn-9(R,S)-yl]-amide de l'acide 1H-pyrrolo[2,3-b]pyridine-4-carboxylique, sous forme d'un solide beige dont les caractéristiques sont les suivantes :
Spectre de masse (E/I) : m/z = 441 (M+) :
Spectre de RMN 1 H (400 MHz - DMSO-d6) δ en ppm : 6,41 (d, J = 8,5 Hz, 1H) ; 6,55 (dd, J = 2,0 et 3,0 Hz, 1 H) ; 6,73 (d, J = 7,5 Hz, 1H) ; 6,92 (dd, J = 2,0 et 3,0 Hz, 1 H) ; 7,10 (t, J = 7,5 Hz, 1 H) ; 7,26 (t, J = 7,5 Hz, 1H) ; 7,31 (d, J = 7,5 Hz, 1 H) ; 7,44 (t, J = 7,5 Hz, 1 H) ; 7,48 (d, J = 5,0 Hz, 1 H) ; de 7,55 à 7,68 (m, 4H) ; 8,02 (m étalé, 1H) ; 8,27 (m étalé, 1 H) ; 8,29 (d, J = 5,0 Hz, 1 H) ; 9,21 (d, J = 8,5 Hz, 1 H) ; 11,8 (s large, 1 H) ; 11,85 (s large, 1H) .

### Exemple 18 : [4-(5,6,7,8-tetrahydro-1,8-naphtyridin-3-yl)-9H-fluorèn-9(R,S)-yl]-amide de l'acide 1H-pyrrolo[2,3-b]pyridine-4-carboxylique

*Etape 1:* Dans un réacteur micro-onde, on introduit successivement 916 mg de 4-(4,4,5,5-tetraméthyl-1,3,2-dioxaborolan-2-yl)-fluorèn-9-one, obtenue comme à l'étape 1 de l'exemple 8, 671 mg de palladium (0) tétrakis (triphénylphosphine), 1,42 g de carbonate de césium et 1 g de N8-pivaloyl-3-iodo-5,6,7,8-tetrahydro-1,8-naphtyridine dans 20 mL de diméthylformamide anhydre. Après 20 heures de chauffage à 120°C, on verse sur 60 mL d'eau, et on extrait 2 fois avec 30 mL d'acétate d'éthyle. Après séchage sur sulfate de magnésium et concentration sous pression réduite, le solide brut obtenu est purifié par flash-chromatographie sur 100 g dé gel de silice (40-60 µm), en éluant avec du cyclohexane puis avec un mélange de cyclohexane et d'acétate d'éthyle (70-30 en volumes). On obtient ainsi 1,1 g de 4-(N8-pivaloyl-5,6,7,8-tetrahydro-1,8-naphtyridin-3-yl)-fluorèn-9-one, sous forme d'une meringue jaune dont la caractéristique est la suivante :
Spectre de masse (E/I) : m/z = 396 (M+)

*Etape 2 :* On opère comme à l'étape 3 de l'exemple 2, à partir de 950 mg de 4-(N8-pivaloyl-5,6,7,8-tetrahydro-1,8-naphtyridin-3-yl)-fluorën-9-one, obtenue à l'étape précédente, de 500 mg de chlorhydrate d'hydroxylamine et de 983 mg d'acétate de sodium, agités à température ambiante pendant 20 heures, puis au reflux pendant 3 heures dans 22 mL d'éthanol. Après concentration du solvant sous pression réduite, le résidu est repris par de l'eau ; le précipité formé est filtré, lavé avec du pentane puis séché à l'air. On obtient ainsi 750 mg de 4-(5,6,7,8-tetrahydro-1,8-naphtyridin-3-yl)-fluorèn-9-one oxime, en mélange 50-50 des isomères Z et E, sous forme d'une poudre grise, utilisée telle quelle à l'étape suivante, dont la caractéristique est la suivante :
Spectre de masse (E/I) : m/z = 327 (M+).

*Etape 3*: Dans un autoclave de 250 mL, on dissout 750 mg de mélange équimoléculaire des isomères Z et E de 4-(5,6,7,8-tetrahydro-1,8-naphtyridin-3-yl)-fluorèn-9-one oxime, obtenue à l'étape 2, dans un mélange de 23 mL d'éthanol et 23 mL de tetrahydrofyrane, puis on ajoute 143 mg de Nickel de Raney et on agite sous une pression de 1 bar d'hydrogène pendant 8 heures à 60°C. Après filtration et lavage à l'éthanol du catalyseur, on concentre à sec sous pression réduite. Le solide brut obtenu est purifié par empâtage dans l'oxyde de diisopropyle. On obtient ainsi 700 mg de 4-(5,6,7,8-tetrahydro-1,8-naphtyridin-3-yl)-fluorèn-9(R,S)-yl-amine, sous forme d'une gomme verdâtre utilisée telle quelle à l'étape suivante, dont la caractéristique est la suivante :
Spectre de masse (E/I) : m/z = 313 (M+)

*Etape 4 :* On opère comme dans l'étape 4 de l'exemple 5 à partir de 700 mg de 4-(5,6,7,8-tetrahydro-1,8-naphtyridin-3-yl)-fluorèn-9(R,S)-yl-amine, obtenue à l'étape 3, et de 362 mg d'acide 1H-pyrrolo[2,3-b]pyridine-4-carboxylique, en présence de 471 mg d'EDCI et de 332 mg d'HOBT, dans 23 mL de DMF pendant 20 heures. Le milieu réactionnel est versé sur 7 mL d'eau et le précipité formé est essoré, lavé avec 10 mL d'eau, puis 3 fois avec une solution saturée d'hydrogénocarbonate de sodium puis à l'eau. Après purification par flash-chromatographie sur 100 g de gel de silice (40-60 µm), en éluant avec un mélange de dichlorométhane et de méthanol (97,5-2,5 en volumes), on obtient 510 mg de [4-(5,6,7,8-tetrahydro-1,8-naphtyridin-3-yl)-9H-fluorèn-9(R,S)-yl]-amide de l'acide 1H-pyrrolo[2,3-b]pyridine-4-carboxylique, sous forme d'un solide blanc dont les caractéristiques sont les suivantes :
Spectre de masse (E/I) : m/z = 457 (M+)
Spectre de RMN 1 H (400 MHz - DMSO-d6) δ en ppm : 1,85 (m, 2H) ; 2,77 (m, 2H) ; 3,48 (m, 2H) ; 6,37 (d, J = 8,5 Hz, 1H) ; 6,61 (s large, 1 H) ; 6,91 (dd, J = 2,0 et 3,0 Hz, 1H) ; de 7,15 à 7,33 (m, 5H) ; 7,38 (t, J = 7,5 Hz, 1 H) ; 7,47 (d, J = 5,0 Hz, 1 H) ; de 7,51 à 7,67 (m, 3H) ; 7,84 (s large, 1 H) ; 8,29 (d, J = 5,0 Hz, 1 H) ; 9,19 (d, J = 8,5 Hz, 1 H) ; 11,85 (s large, 1H) .

### Exemple 19 : 4-{imidazo[1,2-a]pyridin-2-yl}-9H-fluorèn-9-(R,S)-yl-amide de l'acide 1H-pyrrolo[2,3-b]pyridine-4-carboxylique

*Etape 1:* Dans un ballon de 1 L contenant 30 g d'acide monochloracétique dissous dans 150 mL d'eau distillée, on additionne par petites portions 24,1 g de carbonate de potassium. Après agitation pendant 15 minutes après la fin de cette addition (fin de l'effervescence), on ajoute 30 g de 2-aminopyridine et porte le milieu réactionnel au reflux pendant 6 heures. On laisse revenir à température ambiante durant la nuit. La suspension rosâtre est filtrée, le solide est lavé avec de l'eau distillée puis est séché sous vide à 40°C. On obtient 15 g d'un solide beige de 2-hydroxy-imidazo[1,2-a]pyridine.

*Etape 2:* Dans un ballon de 500 mL contenant 1 g de 2-hydroxy-imidazo[1,2-a]pyridine obtenue à l'étape précédente et 60 mL de toluène on ajoute 2.6 g de N-phenyl -bis(trifluorométhanesulfonimide) et 1 mL de triéthylamine puis chauffe au reflux. Après 2 heures et 4 heures de reflux on rajoute respectivement 2,5 mL et 5 mL de triéthylamine puis continue encore le reflux pendant 8 heures. Après refroidissement on ajoute 50 mL d'eau distillée, décante et ré-extrait la phase acqueuse avec 3 fois 50 mL d'acétate d'éthyle. Les phases organiques réunies sont lavées avec 3 fois 50 mL d'eau distillée, 1 fois 50 mL de solution de chlorure de sodium saturée puis sèchées sur sulfate de magnésium. Après évaporation à sec sous vide, on chromatographie le résidu huileux sur gel de silice (40-63 µm) en éluant avec du dichlorométhane. On obtient 0,3 g de 2-trifluorométhanesulfonyloxy-imidazo[1,2-a]pyridine sous forme de cristaux blancs dont la caractéristique est la suivante :
Spectre de masse (LC/MS) : m/z = 266 (M+).

*Etape 3:* Dans un ballon de 100 mL on chauffe sous argon à 120°C un mélange de 178 mg de 4-(4,4,5,5-tetraméthyl-1,3,2-dioxaborolan-2-yl)-fluorèn-9-one, obtenue à l'étape 1 de l'exemple 8, 150 mg de 2-trifluorométhanesulfonyloxy-imidazo[1,2-a]pyridine obtenue à l'étape précédente, de 130 mg de palladium (0) tétrakis (triphénylphosphine), 276 mg de carbonate de césium dans 20mL de diméthylformamide anhydre pendant 3 heures. Après évaporation à sec sous vide du milieu réactionnel, on reprend le résidu par 20 mL d'eau distillée et extrait avec 8 fois 15 mL d'acétate d'éthyle. Les phases organiques réunies sont lavées avec 2 fois 15 mL d'eau distillée, 15 mL de solution de chlorure de sodium saturée puis séchées sur sulfate de sodium. Après évaporation à sec sous vide, on chromatographie le résidu huileux sur gel de silice (40-63µm) en éluant avec un mélange d'acétate d'éthyle et de cyclohexane (4 :6). On obtient 120 mg de 4-{imidazo[1,2-a]pyridin-2-yl}-fluorèn-9-one sous forme de cristaux jaunes dont la caractéristique est la suivante :
Spectre de masse (LC/MS) : m/z = 296(M+).

Etape 4: Dans un ballon de 100 mL, on agite sous argon à température ambiante 120 mg de 4-{imidazo[1,2-a]pyridin-2-yl}-fluorèn-9-one obtenue à l'étape précédente, 84 mg de chlorhydrate d'hydroxylamine et 160 mg d'acétate de sodium dans 5 mL d'éthanol. Le milieu réactionnel est évaporé à sec sous vide. Le résidu est repris dans 10 mL d'eau distillée et le précipité est filtré, lavé avec 3 fois 1,5 ml d'eau distillée, 2 fois 1,5 mL d'une solution saturée de bicarbonate de sodium, 3 fois 1,5 mL d'eau distillée puis sèché sous vide. On obtient 48 mg de 4-{imidazo[1,2-a]pyridin-2-yl}-fluorèn-9-one oxime, en mélange équimoléculaire des isomères Z et E, sous forme d'un solide beige dont la caractéristique est la suivante :
Spectre de masse (LC/MS) : m/z = 311 (M+).

*Etape 5:* Dans un ballon de 25 mL on agite à température ambiante durant 1 nuit 102 mg de 4-{imidazo[1,2-a]pyridin-2-yl}-fluorèn-9-one oxime obtenue à l'étape précédente et de 86 mg de zinc en poudre dans un mélange de 0,6 mL d'acide acétique, 0,6 mL d'eau distillée et 0,6 mL d'éthanol. Après addition de Celite, on filtre le milieu réactionnel et lave le précipité avec 3 fois 1,5 mL d'éthanol. Le filtrat est évaporé à sec sous vide et le résidu est chromatographié sur gel de silice (40-63 µm) en éluant avec un mélange de dichlorométhane- éthanol-ammoniac 7N dans méthanol (90/10/0,5 en volumes). On obtient 95 mg de 4-{imidazo[1,2-a]pyridin-2-yl}-9H-fluorèn-9(R,S)-yl-amine sous forme d'une meringue beige dont la caractéristique est la suivante :
Spectre de masse (LC/MS) : m/z = 297 (M+).

*Etape 6 :* Dans un ballon de 50 mL on agite sous argon durant une nuit à température ambiante, 94 mg de 4-{imidazo[1,2-a]pyridin-2-yl}-9H-fluorèn-9(R,S)-yl-amine obtenue à l'étape précédente, 51 mg d'acide 1H-pyrrolo[2,3-b]pyridine-4-carboxylique, 61 mg d'EDCI et 21 mg de HOBt dans 10 mL de DMF. Après évaporation à sec sous vide du milieu réactionnel, on reprend le résidu par 15 mL d'eau distillée et extrait avec 3 fois 15 mL d'acétate d'éthyle. Les phases organiques regroupées sont lavées avec 10 mL d'une solution saturée de bicarbonate de sodium, 2 fois 10 mL d'eau distillée, 10 mL de solution saturée de chlorure de sodium et sèchée sur sulfate de magnésium. Après évaporation à sec sous vide, le résidu est chromatographié sur gel de silice (40-63 µm) en éluant avec un mélange de dichlorométhane-éthanol (92,5: 7,5). On obtient ainsi 38 mg de 4-{imidazo[1,2-a]pyridin-2-yl}-9H-fluorèn-9(R,S)-yl-amide de l'acide 1H-pyrrolo[2,3-b]pyridine-4-carboxylique sous forme de cristaux blancs dont les caractéristiques sont les suivantes :
Point de fusion (Kofler) : 270°C
Spectre de masse (LC/SM) : m/z 441 (M+)
Spectre de RMN 1 H (400 MHz - DMSO-d6) δ en ppm : 6,39 (d, J = 8,5 Hz, 1 H) ; 6,91 (dd,, J = 2,0 et 3,5 Hz, 1H) ; 7,00 (dt, J = 1,5 et 7,5 Hz, 1H) ; 7,21 (dt, J = 1,5 et 7,5 Hz, 1 H) ; de 7,28 à 7,39 (m, 2H) ; 7,42 (t, J = 7,5 Hz, 1 H) ; 7,47 (d, J = 5,5 Hz, 1H) ; 7,50 (d, J = 7,5 Hz, 1 H) ; 7,59 (d, J = 7,5 Hz, 1 H) ; 7,62 (m, 3H) ; 7,69 (d, J = 7,5 Hz, 1 H) ; 8,20 (s, 1 H) ; 8,29 (d, J = 5,5 Hz, 1 H) ; 8,63 (d, J = 7,5 Hz, 1H) ; 9,26 (d, J = 8,5 Hz, 1 H) ; 11,9 (s large, 1H) .

### Exemple 20 : composition pharmaceutique:

On a préparé des comprimés répondant à la formule suivante :

| | |
|---|---|
| Produit de l'exemple 1 | 0,2 g |
| Excipient pour un comprimé terminé à | 1 g |

| | |
|---|---|
| (détail de l'excipient : lactose, talc, amidon, stéarate de magnésium). | |

### Exemple 21 : composition pharmaceutique

On a préparé des comprimés répondant à la formule suivante :

| | |
|---|---|
| Produit de l'exemple 16 | 0,2 g |
| Excipient pour un comprimé terminé à | 1 g |

| | |
|---|---|
| (détail de l'excipient : lactose, talc, amidon, stéarate de magnésium). | |

La présente invention comprend également toutes les compositions pharmaceuriques préparées avec tout produit de formule (I) selon la présente invention

### Tests biologiques permettant de caractériser biologiquement les produits de l'invention :

Le phosphate inorganique libéré au cours de l'hydrolyse de l'ATP par l'activité ATPasique de Hsp82 est quantifié par la méthode du green Malachite. En présence de ce réactif, il y a formation du complexe phosphate inorganique-molybdate-vert de malachite qui absorbe à une longueur d'onde de 620 nm. Les produits à évaluer sont incubés dans un volume réactionnel de 30 µl, en présence de 1µM Hsp82 et de 250 µM de substrat (ATP) dans un tampon composé de 50 mM Hepes-NaOH (pH 7.5), 1 mM DTT, 5 mM MgCl2 et 50 mM KCI à 37 °C pendant 60 min. Parallèlement, une gamme de phosphate inorganique comprise entre 1 et 40 µM est constituée dans le même tampon. L'activité ATPasique est ensuite révélée par l'addition de 60 µl du réactif biomol green (Tebu). Après 20 min d'incubation à température ambiante, l'absorbance des différents puits est mesurée à l'aide d'un lecteur de microplaque à 620 nm. La concentration en phosphate inorganique de chaque échantillon est alors calculée à partir de la courbe d'étalonnage. L'activité ATPasique d' Hsp82 est exprimée en concentration de phosphate inorganique produit en 60 min.L'effet des divers produits testés est exprimé en pourcentage d'inhibition de l'activité ATPasique.

Dans le test ci-dessus, le composé A000187458 a une concentration inhibitrice 50% (Cl50) égale à 2.5 µM

La formation d'ADP due à l'activité ATPasique de Hsp82 a été utilisée pour mettre au point une autre méthode d'évaluation de l'activité enzymatique de cette enzyme par application d'un système de couplage enzymatique faisant intervenir la pyruvate kinase (PK) et la lactate deshydrogensase (LDH). Dans cette méthode spectrophotométrique de type cinétique, la PK catalyse la formation d'ATP et de pyruvate à partir de phosphoenol-pyruvate (PEP) et de l'ADP produit par HSP82. Le pyruvate formé, substrat de la LDH, est ensuite transformé en lactate en présence de NADH. Dans ce cas, la diminution de la concentration en NADH, mesurée par la diminution de l'absorbance à la longueur d'onde de 340 nm est proportionnelle à la concentration en ADP produit par HSP82.

Les produits testés sont incubés dans un volume réactionnel de 100 µl de tampon composé de 100 mM Hepes-NaOH ( pH 7.5), 5 mM MgCl2, 1mM DTT, 150 mM KCI, 0.3 mM NADH, 2.5 mM PEP et 250 µM ATP. Ce mélange est preincubé à 37°C pendant 30 min avant addition de 3.77 unités de LDH et 3.77 unités de PK. La réaction est initiée par addition du produit à évaluer, en concentrations variables, et de Hsp82, à la concentration de 1 µM. La mesure de l'activité enzymatique de Hsp82 est alors réalisée, en continu, dans un lecteur de microplaque, à 37°C, à la longueur d'onde de 340nm. La vitesse initiale de la réaction est obtenue par la mesure de la pente de la tangente à l'origine de la courbe enregistrée. L'activité enzymatique est exprimée en µM d'ADP formé par minute. L'effet des divers produits testés est exprimé en pourcentage d'inhibition de l'activité ATPasique selon la codification ci-dessous :
A: IC50<µM
B : 1 µM < IC50 < 10µM
C : 10µM < IC₅₀ < 100 µM

**Tableau de résultats**

| Ex | Structure | Hsp82 ATPase IC50 µM | Ex | Structure | Hsp82 ATPase IC50 µM |
|---|---|---|---|---|---|
| 1 | 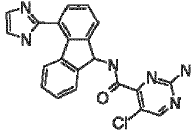 | B | 2 | 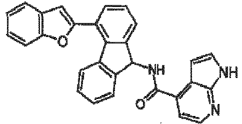 | B |
| 3 | 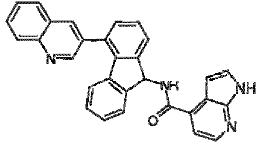 | B | 4 | 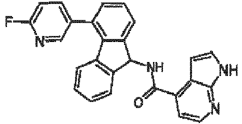 | B |
| 5 | 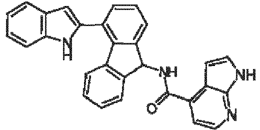 | A | 6 | 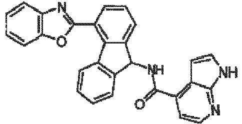 | A |
| 7 | 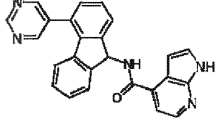 | A | 8 | 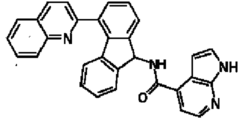 | B |
| 9 | 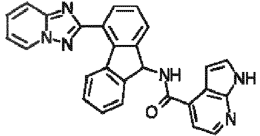 | B | 10 | 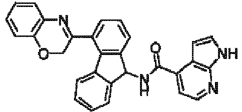 | B |
| 11 | 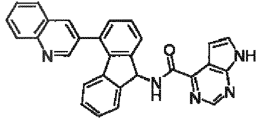 | A | 12 | 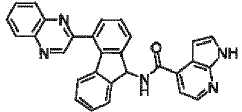 | B |
| 13 | 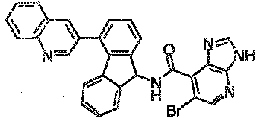 | A | 14 | 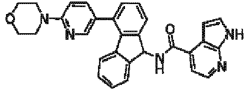 | B |
| 15 | 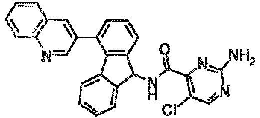 | A | 16 | 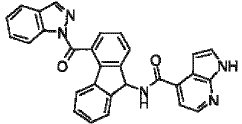 | B |
| 17 | 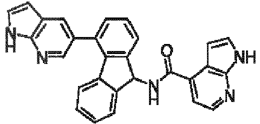 | A | 18 | 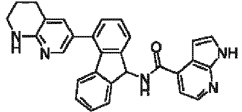 | B |
| 19 | 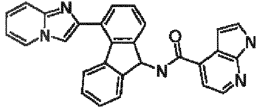 | A | | | |
| | | | | | |

## Revendications

1. Produits de formule (I) : 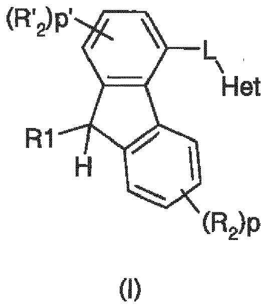 dans lesquels :
Het est choisi parmi les radicaux imidazolyle, benzofuranyle, quinoléinyle, pyridinyle, indolyle, benzoxazolyle, pyrimidinyle, triazolopyridinyle, benzoxazinyle, quinoxalinyle, indazolyle, pyrrolopyridinyle, tetrahydro-1,8-naphtyridinyle; ces radicaux étant éventuellement substitués par un ou plusieurs radicaux R identiques ou différents choisis parmi les atomes d'halogène et les radicaux cyano et morpholino;
R1 représente le radical -NH-C(O)-hétéroaryle, avec hétéroaryle choisi parmi les radicaux quinolyle, pyridyle, purines, quinoxaline, pyrazole, pyrimidinyle, pyrrolo[2,3-b]pyridine, pyrrolo[2,3-c]pyrimidine, imidazo[4,5-b]pyridine, ces radicaux hétéroaryle étant éventuellement substitués par un ou plusieurs radicaux identiques ou différents choisis parmi les atomes d'halogène, les radicaux méthyle, éthyle, NH2, Nhalk et NH-Me,
R2 et R'2, représentent H,
L représente simple liaison ou C(O),
lesdits produits de formule (I) étant sous toutes les formes tautomères et isomères possibles racémiques, énantiomères et diastéréoisomères, ainsi que les sels d'addition avec les acides minéraux et organiques ou avec les bases minérales et organiques desdits produits de formule (Ib) .

2. Produits de formule (I) tels que définis à la revendication 1 dans lesquels Het, R2, R'2, et L ont les significations dans cette revendication et R1 est choisi parmi les radicaux suivants : 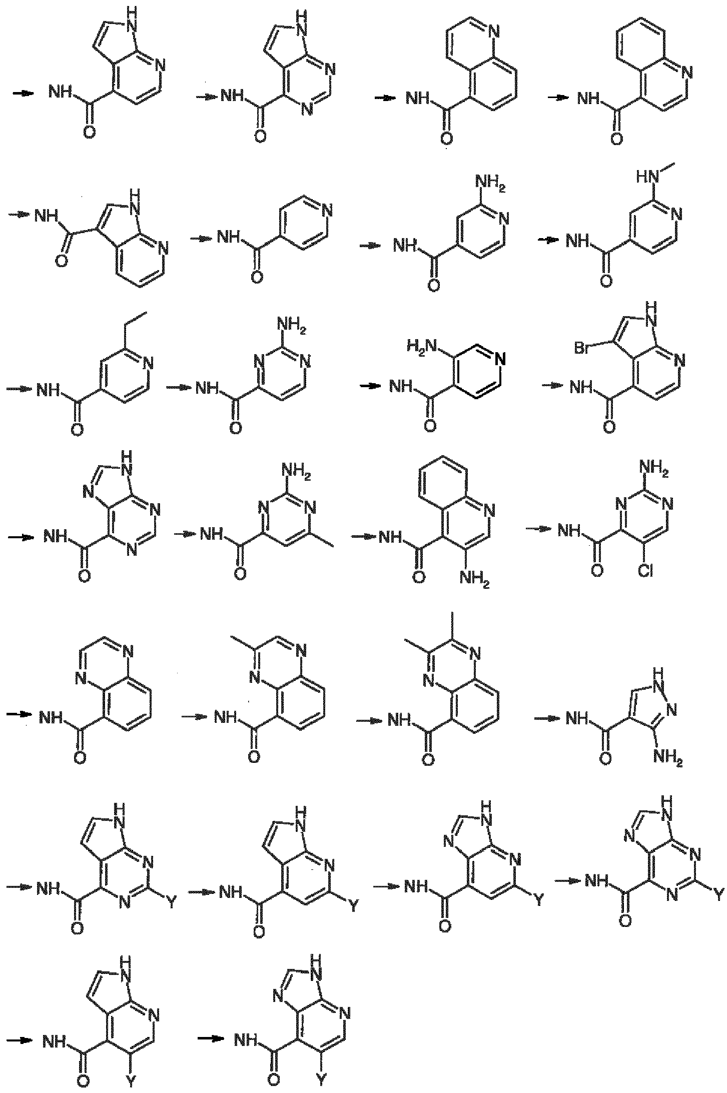 avec Y représente un atome d'halogène ou un radical méthyle ou éthyle,
lesdits produits de formule (I) étant sous toutes les formes tautomères et isomères possibles racémiques, énantiomères et diastéréoisomères, ainsi que les sels d'addition avec les acides minéraux et organiques ou avec les bases minérales et organiques desdits produits de formule (I).

3. Produits de formule (I) tels que définis à l'une quelconque des revendications précédentes dont les noms suivent :
- [4-(1H-imidazol-2-yl)-9H-fluorène-9(R,S)-yl]-amide de l'acide 2-amino-5-chloro-pyrimidine-4-carboxylique
- [4-(benzofuran-2-yl)-9H-fluorèn-9(R,S)-yl)]-amide de l'acide 1 H-pyrrolo[2,3-b]pyridine-4-carboxylique.
- [4-(quinoléin-3-yl)-9H-fluorèn-9(R,S)-yl)]-amide de l'acide 1H-pyrrolo[2,3-b]pyridine-4-carboxylique
- [4-{6-fluoro-pyridin-3-yl)-9H-fluorèn-9-(R,S)-yl]-amide de l'acide 1H-pyrrolo[2,3-b]pyridine-4-carboxylique.
- [4-(1H-indol-2-yl)-9H-fluorèn-9-(R,S)-yl]-amide de l'acide 1H-pyrrolo[2,3-b]pyridine-4-carboxylique
- [4-(benzoxazol-2-yl)-9H-fluorèn-9-(R,S)-yl]-amide de l'acide 1H-Pyrrolo[2,3-b]pyridine-4-carboxylique
- [4-(Pyrimidin-5-yl)-9H-fluorèn-9-(R,S)yl]-amide de l'acide 1 H-Pyrrolo[2,3-b]pyridine-4-carboxylique
- [4-(quinoléin-2-yl)-9H-fluorèn-9-(R,S)-yl]-amide de l'acide 1H-pyrrolo[2,3-b]pyridine-4-carboxylique
- (4-[1,2,4]triazolo[1,5-a]pyridin-2-yl-9H-fluoren-9(R,S)-yl)-amide de l'acide 1H-pyrrolo[2,3-b]pyridine-4-carboxylique
- [4-(1,4-benzoxazin-2H-3-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 7H-pyrrolo[2,3-c]pyrimidine-4-carboxylique
- [4-(quinoxalin-2-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 6-bromo-3H-imidazo[4,5-b]pyridine-7-carboxylique
- [4-(2-morpholino-pyridin-5-yl)-9H-fluorèn-9(R,S)-yl]-amide de l'acide 1H-pyrrolo[2,3-b]pyridine-4-carboxylique
- [4-(quinoléin-3-yl)-9H-fluorèn-9(R,S)-yl]-amide de l'acide 2-amino-5-chloro-pyrimidine-4-carboxylique
- [4-(indazole-1-carbonyl)-9H-fluorèn-9(R,S)-yl]-amide de l'acide 1H-pyrrolo[2,3-b]pyridine-4-carboxylique
- [4-(1H-pyrrolo[2,3-b]pyridin-5-yl)-9H-fluorén-9(R,S)-yl]-amide de l'acide 1 H-pyrrolo[2,3-b]pyridine,4-arboxylique
- 4-(5,6,7,8-tetrahydro-1,8-naphtyridin-3-yl)-9H-fluorèn-9(R,S)-yl]-amide de l'acide 1H-pyrrolo[2,3-b]pyridine-4-carboxylique
- 4-{imidazo[1,2-a]pyridin-2-yl}-9H-fluorèn-9-(R,S)-yl-amide de l'acide 1H-pyrrolo[2,3-b]pyridine-4-carboxylique
lesdits produits de formule (I) étant sous toutes les formes isomères possibles racémiques, énantiomères et diastéréo-isomères, ainsi que les sels d'addition avec les acides minéraux et organiques ou avec les bases minérales et organiques.

4. A titre de médicaments, les produits de formule (I) telle que définie aux revendications 1 à 3, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

5. A titre de médicaments, les produits de formule (I) telle que définie à la revendication 3, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

6. Les compositions pharmaceutiques contenant à titre de principe actif, l'un au moins des médicaments tels que définis aux revendications 4 ou 5.

7. Compositions pharmaceutiques telles que définies à la revendication précédente contenant en plus, des principes actifs d'autres médicaments de chimiothérapie contre le cancer.

8. Compositions pharmaceutiques selon l'une quelconque des revendications 6 ou 7 **caractérisées en ce qu'**elles sont utilisées comme médicaments, en particulier pour la chimiothérapie de cancers.

9. Utilisation de produits de formule (I) tels que définis à l'une quelconque des revendications 1 à 3 ou de sels pharmaceutiquement acceptables desdits produits de formule (I) pour la préparation de médicaments destinés à inhiber l'activité de protéines chaperones et notamment d'Hsp90.

10. Utilisation de produits de formule (I) telle que définie à l'une quelconque des revendications 1 à 3 ou de sels pharmaceutiquement acceptables desdits produits de formule (I) pour la préparation d'un médicament destiné à prévenir ou traiter une maladie **caractérisée par** le dérèglement de l'activité d'une protéine chaperone de type Hsp90 .

11. Utilisation de produits de formule (I) telle que définie à l'une quelconque des revendications 1 à 3 ou de sels pharmaceutiquement acceptables desdits produits de formule (I) pour la préparation d'un médicament destiné à prévenir ou traiter une maladie appartenant au groupe suivant: les maladies neurodégénératives telles que la maladie de Huntington, la maladie de Parkinson, l'ischémie cérébrale focale, la maladie d'Alzheimer, la sclérose en plaques et la sclérose latérale amyotrophique, la malaria, les filarioses de Brugia et de Bancroft, la toxoplasmose, les mycoses résistantes aux traitements, l'hépatite B, l'hépatite C, le virus de l'Herpes, la dengue (ou grippe tropicale), l'atrophie musculaire spinale et bulbaire, désordres de la prolifération de cellules mésangiales, thromboses, rétinopathies, psoriasis, dégénération musculaire, maladies en oncologie, cancers.

12. Utilisation de produits de formule (I) telle que définie à l'une quelconque des revendications 1 à 3 ou de sels pharmaceutiquement acceptables desdits produits de formule (I) pour la préparation d'un médicament destiné à traiter des cancers.

13. Utilisation de produits de formule (I) selon la revendication précédente dans laquelle la maladie à traiter est un cancer de tumeurs solides ou liquides.

14. Utilisation de produits de formule (I) selon la revendication précédente dans laquelle la maladie à traiter est un cancer résistant aux agents cytotoxiques.

15. Utilisation de produits de formule (I) telle que définie à l'une quelconque des revendications 1 à 3 ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloîdes chroniques, les leucémies lymphoblastiques aigûes, les cancers de la prostate, du pancréas et du colon, les mélanomes métastatiques, les tumeurs de la thyroïde et les carcinomes rénaux..

16. Utilisation de produits de formule (I) telle que définie à l'une quelconque des revendications 1 à 3 ou de sels pharmaceutiquement acceptables desdits produits de formule (I) pour la préparation d'un médicament destiné à la chimiothérapie de cancers.

17. Utilisation de produits de formule (I) telle que définie à l'une quelconque des revendications 1 à 3 ou de sels pharmaceutiquement acceptables desdits produits de formule (I) pour la préparation de médicaments destinés à la chimiothérapie de cancers utilisés seuls ou en association.

18. Utilisation de produits de formule (I) telle que définie à l'une quelconque des revendications 1 à 3 ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

19. Utilisation de produits de formule (I) selon la revendication précédente dans laquelle les agents thérapeutiques peuvent être des agents anti-tumoraux utilisés communément.

20. Produits de formule (I) tels que définis à l'une quelconque des revendications 1 à 3 comme inhibiteurs de HSP90, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

## Patentansprüche

1. Produkte der Formel (I): 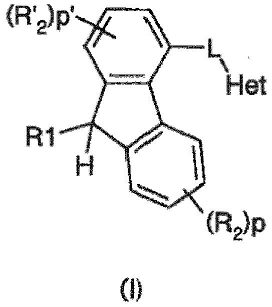 worin:
Het aus Imidazolyl-, Benzofuranyl-, Chinolinyl-, Pyridinyl-, Indolyl-, Benzoxazolyl-, Pyrimidinyl-, Triazolopyridinyl-, Benzoxazinyl-, Chinoxalinyl-, Indazolyl-, Pyrrolopyridinyl- und Tetrahydro-1,8-naphthyridinylresten ausgewählt ist; wobei diese Reste gegebenenfalls durch einen oder mehrere gleiche oder verschiedene Reste R, die aus Halogenatomen und Cyano- und Morpholinoresten ausgewählt sind, substituiert sind;
R1 für den Rest -NH-C(0)-Heteroaryl steht, wobei das Heteroaryl aus Chinolyl-, Pyridyl-, Purin-, Chinoxalin-, Pyrazol-, Pyrimidinyl-, Pyrrolo[2,3-b]pyridin-, Pyrrolo[2,3-c]pyrimidin- und Imidazo-[4,5-b]pyridinresten ausgewählt ist, wobei diese Heteroarylreste gegebenenfalls durch einen oder mehrere gleiche oder verschiedene Reste, die aus Halogenatomen und Methyl-, Ethyl-, NH2-, Nhalk- und NH-Me-Resten ausgewählt sind, substituiert sind,
R2 und R'2 für H stehen,
L für eine Einfachbindung oder C(0) steht,
wobei die Produkte der Formel (I) in allen möglichen tautomeren und racemischen, enantiomeren und diastereoisomeren isomeren Formen vorliegen, sowie Additionssalze der Produkte der Formel (I) mit anorganischen und organischen Säuren oder mit anorganischen und organischen Basen.

2. Produkte der Formel (I) nach Anspruch 1, worin Het, R2, R'2 und L die in diesem Anspruch angegebenen Bedeutungen besitzen und R1 aus den folgenden Resten ausgewählt ist: 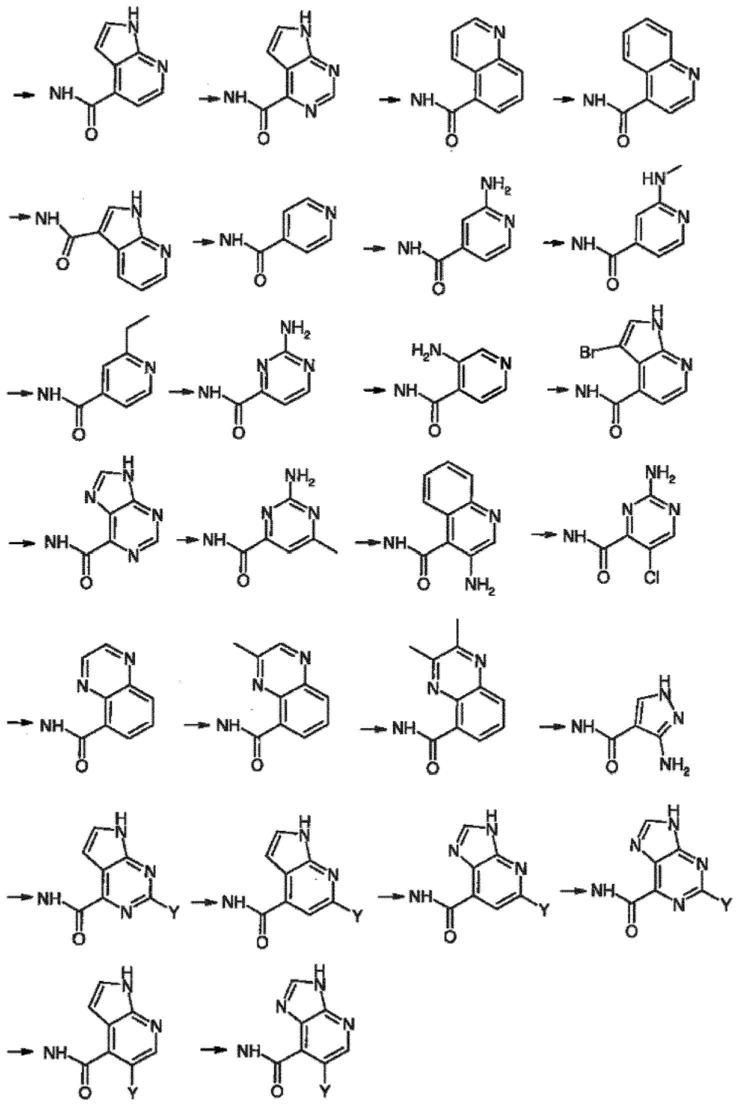 wobei Y für ein Halogenatom oder einen Methyl- oder Ethylrest steht,
wobei die Produkte der Formel (I) in allen möglichen tautomeren und racemischen, enantiomeren und diastereoisomeren isomeren Formen vorliegen, sowie Additionssalze der Produkte der Formel (I) mit anorganischen und organischen Säuren oder mit anorganischen und organischen Basen.

3. Produkte der Formel (I) nach einem der vorhergehenden Ansprüche mit den folgenden Namen:
- 2-Amino-5-chlorpyrimidin-4-carbonsäure[4-(1H-imidazol-2-yl)-9H-fluoren-9(R,S)-yl]amid
- 1H-Pyrrolo[2,3-b]pyridin-4-carbonsäure[4-(benzofuran-2-yl)-9H-fluoren-9(R,S)-yl)]amid
- 1H-Pyrrolo[2,3-b]pyridin-4-carbonsäure[4-(chinolin-3-yl)-9H-fluoren-9(R,S)-yl)]amid
- 1H-Pyrrolo[2,3-b]pyridin-4-carbonsäure[4-(6-fluorpyridin-3-yl)-9H-fluoren-9-(R,S)-yl]-amid
- 1H-Pyrrolo[2,3-b]pyridin-4-carbonsäure[4-(1H-indol-2-yl)-9H-fluoren-9-(R,S)-yl]amid
- 1H-Pyrrolo[2,3-b]pyridin-4-carbonsäure[4-(benzoxazol-2-yl)-9H-fluoren-9-(R,S)-yl]amid
- 1H-Pyrrolo[2,3-b]pyridin-4-carbonsäure[4-(pyrimidin-5-yl)-9H-fluoren-9-(R,S)-yl]amid
- 1H-Pyrrolo[2,3-b]pyridin-4-carbonsäure[4-(chinolin-2-yl)-9H-fluoren-9-(R,S)-yl]amid
- 1H-Pyrrolo[2,3-b]pyridin-4-carbonsäure(4-[1,2,4]triazolo[1,5-a]pyridin-2-yl-9H-fluoren-9(R,S)-yl)amid
- 1H-pyrrolo[2,3-b]pyridin-4-carbonsäure[4-(1,4-benzoxazin-2H-3-yl)-9H-fluoren-9(R,S)-yl]amid
- 7H-Pyrrolo[2,3-c]pyrimidin-4-carbonsäure[4-(chinolin-3-yl)-9H-fluoren-9(R,S)-yl]amid
- 1H-Pyrrolo[2,3-b]pyridin-4-carbonsäure[4-(chinoxalin-2-yl)-9H-fluoren-9(R,S)-yl]amid
- 6-Brom-3H-imidazo[4,5-b]pyridin-7-carbonsäure[4-(chinolin-3-yl)-9H-fluoren-9(R,S)-yl]amid
- 1H-Pyrrolo[2,3-b]pyridin-4-carbonsäure[4-(2-morpholinopyridin-5-yl)-9H-fluoren-9(R,S)-yl]amid
- 2-Amino-5-chlorpyrimidin-4-carbonsäure[4-(chinolin-3-yl)-9H-fluoren-9(R,S)-yl]amid
- 1H-Pyrrolo[2,3-b]pyridin-4-carbonsäure[4-(indazol-1-carbonyl)-9H-fluoren-9(R,S)-yl]amid
- 1H-Pyrrolo[2,3-b]pyridin-4-carbonsäure[4-(1H-pyrrolo[2,3-b]pyridin-5-yl)-9H-fluoren-9(R,S)-yl]amid
- 1H-Pyrrolo[2,3-b]pyridin-4-carbonsäure-[4-(5,6,7,8-tetrahydro-1,8-naphthyridin-3-yl)-9H-fluoren-9(R,S)-yl]amid
- 1H-Pyrrolo[2,3-b]pyridin-4-carbonsäure-4-{imidazo[1,2-a]pyridin-2-yl}-9H-fluoren-9-(R,S)-ylamid,
wobei die Produkte der Formel (I) in allen möglichen racemischen, enantiomeren und diastereoisomeren isomeren Formen vorliegen, sowie Additionssalze mit anorganischen und organischen Säuren oder mit anorganischen und organischen Basen.

4. Produkte der Formel (I) nach den Ansprüchen 1 bis 3, wobei die Produkte der Formel (I) in allen möglichen racemischen, enantiomeren und diastereoisomeren isomeren Formen vorliegen, sowie Additionssalze der Produkte der Formel (I) mit anorganischen und organischen Säuren oder mit anorganischen und organischen Basen, die pharmazeutisch unbedenklich sind, als Medikamente.

5. Produkte der Formel (I) nach Anspruch 3, wobei die Produkte der Formel (I) in allen möglichen racemischen, enantiomeren und diastereoisomeren isomeren Formen vorliegen, sowie Additionssalze der Produkte der Formel (I) mit anorganischen und organischen Säuren oder mit anorganischen und organischen Basen, die pharmazeutisch unbedenklich sind, als Medikamente.

6. Pharmazeutische Zusammensetzungen, die als Wirkstoff mindestens eines der Medikamente nach den Ansprüchen 4 oder 5 enthalten.

7. Pharmazeutische Zusammensetzungen nach dem vorhergehenden Anspruch, die außerdem Wirkstoffe anderer Medikamente für die Krebschemotherapie enthalten.

8. Pharmazeutische Zusammensetzungen nach einem der Ansprüche 6 oder 7, **dadurch gekennzeichnet, dass** sie als Medikamente verwendet werden, insbesondere für die Krebschemotherapie.

9. Verwendung von Produkten der Formel (I) nach einem der Ansprüche 1 bis 3 oder von pharmazeutisch unbedenklichen Salzen der Produkte der Formel (I) zur Herstellung von Medikamenten zur Inhibierung der Aktivität von Chaperon-Proteinen und insbesondere Hsp90.

10. Verwendung von Produkten der Formel (I) nach einem der Ansprüche 1 bis 3 oder von pharmazeutisch unbedenklichen Salzen der Produkte der Formel (I) zur Herstellung eines Medikaments zur Prävention oder Behandlung einer Erkrankung, die durch die Störung der Aktivität eines Chaperon-Proteins vom Hsp90-Typ gekennzeichnet ist.

11. Verwendung von Produkten der Formel (I) nach einem der Ansprüche 1 bis 3 oder von pharmazeutisch unbedenklichen Salzen der Produkte der Formel (I) zur Herstellung eines Medikaments zur Prävention oder Behandlung einer Erkrankung aus der folgenden Gruppe: neurodegenerative Erkrankungen wie Chorea Huntington, Parkinson-Krankheit, fokale Hirnischämie, Alzheimer-Krankheit, multiple Sklerose und amyotrophe Lateralsklerose, Malaria, Brugia- und Bancroft-Filarie, Toxoplasmose, behandlungsresistente Mykosen, Hepatitis B, Hepatitis C, Herpesvirus, Dengue (oder Tropengrippe), spinale und bulbäre Muskelatrophie, Störungen der Mesangialzellenproliferation, Thrombosen, Retinopathien, Psoriasis, Muskeldegeneration, onkologische Erkrankungen, Krebserkrankungen.

12. Verwendung von Produkten der Formel (I) nach einem der Ansprüche 1 bis 3 oder von pharmazeutisch unbedenklichen Salzen der Produkte der Formel (I) zur Herstellung eines Medikaments zur Behandlung von Krebserkrankungen.

13. Verwendung von Produkten der Formel (I) nach dem vorhergehenden Anspruch, wobei es sich bei der zu behandelnden Erkrankung um einen Krebs mit soliden oder flüssigen Tumoren handelt.

14. Verwendung von Produkten der Formel (I) nach dem vorhergehenden Anspruch, wobei es sich bei der zu behandelnden Erkrankung um einen Krebs handelt, der gegenüber Cytotoxika resistent ist.

15. Verwendung von Produkten der Formel (I) nach einem der Ansprüche 1 bis 3 oder von pharmazeutisch unbedenklichen Salzen der Produkte der Formel (I) zur Herstellung eines Medikaments zur Behandlung von Krebserkrankungen, u.a. Lungen-, Brust- und Eierstockkrebs, Glioblastomen, chronischen myeloischen Leukämien, akuten lymphoblastischen Leukämien, Prostata-, Bauchspeicheldrüsen- und Kolonkrebs, metastatischen Melanomen, Schilddrüsentumoren und Nierenkarzinomen.

16. Verwendung von Produkten der Formel (I) nach einem der Ansprüche 1 bis 3 oder von pharmazeutisch unbedenklichen Salzen der Produkte der Formel (I) zur Herstellung eines Medikaments für die Krebschemotherapie.

17. Verwendung von Produkten der Formel (I) nach einem der Ansprüche 1 bis 3 oder von pharmazeutisch unbedenklichen Salzen der Produkte der Formel (I) zur Herstellung von Medikamenten für die Krebschemotherapie, die allein oder in Kombination verwendet werden.

18. Verwendung von Produkten der Formel (I) nach einem der Ansprüche 1 bis 3 oder von pharmazeutisch unbedenklichen Salzen der Produkte der Formel (I) zur Herstellung von Medikamenten zur alleinigen Verwendung oder zur Verwendung in Kombination mit Chemotherapie oder Strahlentherapie oder alternativ dazu in Kombination mit anderen Therapeutika.

19. Verwendung von Produkten der Formel (I) nach dem vorhergehenden Anspruch, wobei es sich bei den Therapeutika um gängige Antitumormittel handeln kann.

20. Produkte der Formel (I) nach einem der Ansprüche 1 bis 3 als HSP90-Inhibitoren, wobei die Produkte der Formel (I) in allen möglichen racemischen, enantiomeren und diastereoisomeren isomeren Formen vorliegen, sowie Additionssalze der Produkte der Formel (I) mit anorganischen und organischen Säuren oder mit anorganischen und organischen Basen, die pharmazeutisch unbedenklich sind.

## Claims

1. Products of formula (I): 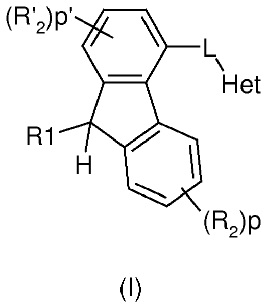 in which:
Het is selected from the imidazolyl, benzofuranyl, quinolinyl, pyridinyl, indolyl, benzoxazolyl, pyrimidinyl, triazolopyridinyl, benzoxazinyl, quinoxalinyl, indazolyl, pyrrolopyridinyl, tetrahydro-1,8-naphthyridinyl radicals; said radicals being optionally substituted with one or more radicals R, which may be identical or different, selected from halogen atoms and the cyano and morpholino radicals;
R1 represents the radical -NH-C(O)-heteroaryl, the heteroaryl being selected from the quinolyl, pyridyl, purines, quinoxaline, pyrazole, pyrimidinyl, pyrrolo[2,3-b]pyridine, pyrrolo[2,3-c]pyrimidine, imidazo[4,5-b]pyridine radicals, these heteroaryl radicals being optionally substituted with one or more radicals, which may be identical or different, selected from halogen atoms, the methyl, ethyl, NH2, Nhalk and NH-Me radicals,
R2 and R'2 represent H,
L represents a single bond or C(O),
said products of formula (I) being in all possible tautomeric and isomeric forms: racemates, enantiomers and diastereoisomers, as well as salts of addition of said products of formula (Ib) with inorganic and organic acids or with inorganic and organic bases.

2. Products of formula (I) as defined in Claim 1 in which Het, R2, R'2 and L have the meanings in this claim and R1 is selected from the following radicals: 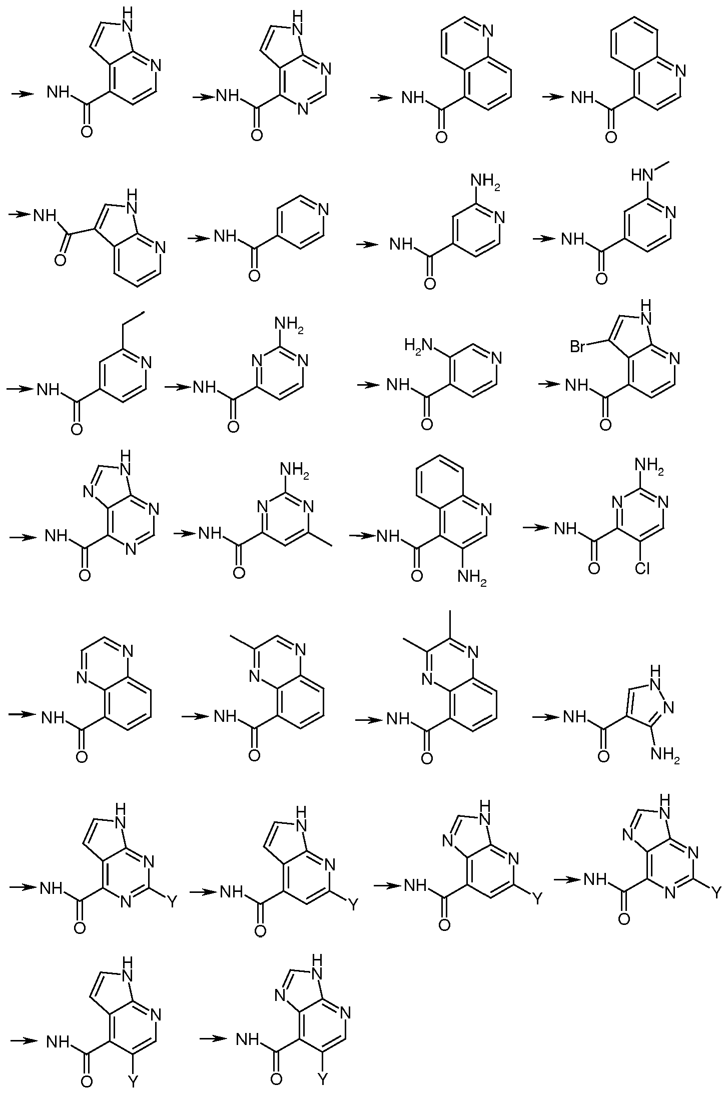 with Y representing a halogen atom or a methyl or ethyl radical,
said products of formula (I) being in all possible tautomeric and isomeric forms: racemates, enantiomers and diastereoisomers, as well as salts of addition of said products of formula (I) with inorganic and organic acids or with inorganic and organic bases.

3. Products of formula (I) as defined in either one of the preceding claims, with the following names:
- [4-(1H-imidazol-2-yl)-9H-fluoren-9(R,S)-yl]-amide of 2-amino-5-chloro-pyrimidine-4-carboxylic acid
- [4-(benzofuran-2-yl)-9H-fluoren-9(R,S)-yl)]-amide of 1H-pyrrolo[2,3-b]pyridine-4-carboxylic acid
- [4-(quinolin-3-yl)-9H-fluoren-9(R,S)-yl)]-amide of 1H-pyrrolo[2,3-b]pyridine-4-carboxylic acid
- [4-(6-fluoro-pyridin-3-yl)-9H-fluoren-9-(R,S)-yl]-amide of 1H-pyrrolo[2,3-b]pyridine-4-carboxylic acid
- [4-(1H-indol-2-yl)-9H-fluoren-9-(R,S)-yl]-amide of 1H-pyrrolo[2,3-b]pyridine-4-carboxylic acid
- [4-(benzoxazol-2-yl)-9H-fluoren-9-(R,S)-yl]-amide of 1H-Pyrrolo[2,3-b]pyridine-4-carboxylic acid
- [4-(pyrimidin-5-yl)-9H-fluoren-9-(R,S)yl]-amide of 1H-Pyrrolo[2,3-b]pyridine-4-carboxylic acid
- [4-(quinolin-2-yl)-9H-fluoren-9-(R,S)-yl]-amide of 1H-pyrrolo[2,3-b]pyridine-4-carboxylic acid
- (4-[1.2.4]triazolo[1,5-a]pyridin-2-yl-9H-fluoren-9(R,S)-yl)-amide of 1H-pyrrolo[2,3-b]pyridine-4-carboxylic acid
- [4- (1,4-benzoxazin-2H-3-yl)-9H-fluoren-9(R,S)-yl] - amide of 1H-pyrrolo[2,3-b]pyridine-4-carboxylic acid
- [4-(quinolin-3-yl)-9H-fluoren-9(R,S)-yl]-amide of 7H-pyrrolo[2,3-c]pyrimidine-4-carboxylic acid
- [4-(quinoxalin-2-yl)-9H-fluoren-9(R,S)-yl]-amide of 1H-pyrrolo[2,3-b]pyridine-4-carboxylic acid
- [4-(quinolin-3-yl)-9H-fluoren-9(R,S)-yl]-amide of 6-bromo-3H-imidazo[4,5-b]pyridine-7-carboxylic acid
- [4-(2-morpholino-pyridin-5-yl)-9H-fluoren-9(R,S)-yl]-amide of 1H-pyrrolo[2,3-b]pyridine-4-carboxylic acid
- [4-(quinolin-3-yl)-9H-fluoren-9(R,S)-yl]-amide of 2-amino-5-chloro-pyrimidine-4-carboxylic acid
- [4-(indazole-1-carbonyl)-9H-fluoren-9(R,S)-yl]-amide of 1H-pyrrolo[2,3-b]pyridine-4-carboxylic acid
- [4-(1H-pyrrolo[2,3-b]pyridin-5-yl)-9H-fluoren-9(R,S)-yl]-amide of 1H-pyrrolo[2,3-b]pyridine-4-carboxylic acid
- 4-(5,6,7,8-tetrahydro-1,8-naphthyridin-3-yl)-9H-fluoren-9(R,S)-yl]-amide of 1H-pyrrolo[2,3-b]pyridine-4-carboxylic acid
- 4-{imidazo[1,2-a]pyridin-2-yl}-9H-fluoren-9-(R,S)-yl-amide of 1H-pyrrolo[2,3-b]pyridine-4-carboxylic acid
said products of formula (I) being in all possible isomeric forms: racemates, enantiomers and diastereoisomers, as well as salts of addition with inorganic and organic acids or with inorganic and organic bases.

4. As medicinal products, the products of formula (I) as defined in Claims 1 to 3, said products of formula (I) being in all possible isomeric forms: racemates, enantiomers and diastereoisomers, as well as salts of addition of said products of formula (I) with pharmaceutically acceptable inorganic and organic acids or inorganic and organic bases.

5. As medicinal products, the products of formula (I) as defined in Claim 3, said products of formula (I) being in all possible isomeric forms: racemates, enantiomers and diastereoisomers, as well as salts of addition of said products of formula (I) with pharmaceutically acceptable inorganic and organic acids or inorganic and organic bases.

6. Pharmaceutical compositions containing, as active principle, at least one of the medicinal products as defined in Claims 4 or 5.

7. Pharmaceutical compositions as defined in the preceding claim, additionally containing active principles of other medicinal products for cancer chemotherapy.

8. Pharmaceutical compositions according to either one of Claims 6 and 7, **characterized in that** they are used as medicinal products, in particular for cancer chemotherapy.

9. Use of products of formula (I) as defined in any one of Claims 1 to 3 or of pharmaceutically acceptable salts of said products of formula (I) for the preparation of medicinal products intended to inhibit the activity of chaperone proteins and notably of Hsp90.

10. Use of products of formula (I) as defined in any one of Claims 1 to 3 or of pharmaceutically acceptable salts of said products of formula (I) for the preparation of a medicinal product intended for preventing or treating a disease **characterized by** disturbance of the activity of a chaperone protein of the Hsp90 type.

11. Use of products of formula (I) as defined in any one of Claims 1 to 3 or of pharmaceutically acceptable salts of said products of formula (I) for the preparation of a medicinal product intended for preventing or treating a disease belonging to the following group: neurodegenerative diseases such as Huntington's disease, Parkinson's disease, focal cerebral ischaemia, Alzheimer's disease, multiple sclerosis and amyotrophic lateral sclerosis, malaria, brugian and bancroftian filarioses, toxoplasmosis, mycoses resistant to treatments, hepatitis B, hepatitis C, herpes virus, dengue (or breakbone fever), spinal and bulbar muscular atrophy, disorders of proliferation of mesangial cells, thromboses, retinopathies, psoriasis, muscular degeneration, oncologic diseases, cancers.

12. Use of products of formula (I) as defined in any one of Claims 1 to 3 or of pharmaceutically acceptable salts of said products of formula (I) for the preparation of a medicinal product intended for treating cancers.

13. Use of products of formula (I) according to the preceding claim in which the disease to be treated is a cancer of solid or liquid tumours.

14. Use of products of formula (I) according to the preceding claim in which the disease to be treated is a cancer that is resistant to cytotoxic agents.

15. Use of products of formula (I) as defined in any one of Claims 1 to 3 or of pharmaceutically acceptable salts of said products of formula (I) for the preparation of a medicinal product intended for the treatment of cancers including lung cancer, breast cancer and ovarian cancer, glioblastomas, chronic myeloid leukaemias, acute lymphoblastic leukaemias, cancers of the prostate, pancreas and colon, metastatic melanomas, tumours of the thyroid and renal carcinomas.

16. Use of products of formula (I) as defined in any one of Claims 1 to 3 or of pharmaceutically acceptable salts of said products of formula (I) for the preparation of a medicinal product intended for cancer chemotherapy.

17. Use of products of formula (I) as defined in any one of Claims 1 to 3 or of pharmaceutically acceptable salts of said products of formula (I) for the preparation of medicinal products intended for cancer chemotherapy, used alone or in combination.

18. Use of products of formula (I) as defined in any one of Claims 1 to 3 or of pharmaceutically acceptable salts of said products of formula (I) for the preparation of medicinal products intended to be used alone or in combination with chemotherapy or radiotherapy or alternatively in combination with other therapeutic agents.

19. Use of products of formula (I) according to the preceding claim in which the therapeutic agents can be commonly used antitumour agents.

20. Products of formula (I) as defined in any one of Claims 1 to 3 as Hsp90 inhibitors, said products of formula (I) being in all possible isomeric forms: racemates, enantiomers and diastereoisomers, as well as salts of addition of said products of formula (I) with pharmaceutically acceptable inorganic and organic acids or inorganic and organic bases.
